# EUROPEAN PATENT APPLICATION

(11) **EP 3 498 848 A2**
(43) Date of publication of application: **19.06.2019**
(21) Application number: 18200215.4
(22) Date of filing: 02.03.2012
(51) Int. Cl.: C12N 15/82, C12N 9/00, A01H 5/10

(54) **MUTANTS**

(30) Priority: 02.03.2011 GB 201103569
(62) Divisional of application: 12708383.0
(71) Applicant: Plant Bioscience Limited, Norwich, Norfolk NR4 7UH (GB)
(72) Inventor: BANCROFT, Ian, Norwich Norfolk NR4 7UH (GB); WELLS, Rachel, Norwich Norfolk NR4 7UH (GB)
(74) Representative: D Young & Co LLP

(57) **Abstract**

Identification of new *FAD2* mutants which result in plants with a more desirable oleic acid composition than in known plants. For the first time, this patent disclosure provides a complete characterization of the genome of a given germplasm of *Brassica napus,* and reports that there are, in fact, four *FAD2* genes in any given genotype, and that in any given germplasm, one or more of the genes are active, thereby reducing the total percentage of oleic acid achievable in the total fatty acids produced in that germplasm. Armed with this knowledge, the inventors herein have produced a novel series of modifications in the genome of various *Brassica napus* germplasms and provide a germplasm with a compromised and/or totally inactive set of *FAD2* genes.

## Description

### FIELD OF THE INVENTION

The present invention relates to new *FAD2* mutants which result in plants with a desirable fatty acid composition, and particularly a desirable oleic acid content; although alternatively the present invention also allows a desirable polyunsaturated fatty acid content to be achieved. The present invention also relates to corresponding markers and their use in methods for the provision of new materials (plants, their progeny and seeds, and (bio)lubricants) comprising the desirable fatty acid composition.

### BACKGROUND OF THE INVENTION

The quality of edible and industrial oil derived from a plant is in part determined by its fatty acid composition. Both the type and amount of fatty acid unsaturation have implications for both dietary and industrials applications. Oils exhibiting reduced levels of polyunsaturated fatty acids (PUFAs) are associated with higher oxidative stability; the susceptibility of a fatty acid to oxidation being dependent on its degree of unsaturation. For example the rate of oxidation of linolenic acid (C18:3) is 100 times that of oleic acid (C18:1). The rate of oxidation of linoleic acid (C18:2) is also greater than that of oleic acid. Moreover, oleic acid has been associated with beneficial health effects, such as reduced cholesterol.

For many years, those skilled in this particular field have been exploring the production of high oleic acid plants. Many reports and patent documents exist in the art reporting various methods, means, compositions and plants in which, in particular, efforts have been made to silence, mutate, delete, inactivate or otherwise compromise the activity of the delta-12 oleate desaturase enzyme, known generally as FAD2.

For example, a non-exhaustive list of previously published patent documents in this field include the following: WO99/53050, disclosing, for example, at page 31-32, methods for using hairpin RNA molecules encoding portions of *FAD2* (e.g. Genbank AF123460, AF12042841, L26296 or A65102) of oilseed rape (*Brassica juncea, napus, rapa, oleracea, campestris, carinata*) as well as corn, cotton, groundnut, sunflower, castor beans, flax, coconut, linseed, or soybean, to increase the oleic acid percentage of total fatty acid, with concomitant decrease in the percentage of the linolenic and linoleic acid percentage in total fatty acid in plants expressing such hairpin RNAs. See also, for example, the following patent disclosures by a wide and diverse series of patent applicants, relating to various efforts to achieve increased oleic acid content in a variety of different plants: US 5,840,946 (Pioneer, "Vegetable Oil Extracted From Rapeseeds Having a Genetically Controlled Unusually High Oleic Acid Content"); WO2004/072259 (Dow Agrosciences, "Altered Fad2 and Fad3 Genes in Brassica and the Molecular Marker-Assisted Detection Thereof"); WO2007/107590 (Monsanto, "FAD-2 Mutants and High Oleic Plants"); WO2007/138444 (INRA, "Genetic Markers for High Oleic Acid Content in Plants"); US 7,423,198 and US 7,605,301 (Viterra, "High Oleic Acid *Brassica juncea"* and "Plant Fad2 Coding Sequence Balancing Fatty Acid Profiling in Edible Oils", respectively).

The plethora of work and reports in this field notwithstanding, in *Brassica napus,* (oilseed rape) for example, there has been considerable confusion about exactly how many copies (or, as we shall term them, homologues) of the FAD2 desaturase are present and active in any given germplasm. In general, those skilled in the art have made reference to *FAD2-1* and *FAD2-2,* which would lead those skilled in the art to conclude that there are but two homologues of the delta-12 oleate desaturase enzyme encoded by the *B. napus* genome.

Residual oleate desaturase activity has been attributed to other, compensating pathways, and those skilled in the art have not appreciated that there may be additional functional homologues of *FAD2* in leading lines used for oleic acid production. Since any remaining FAD2 activity in a given plant will result in reduction in the oleic acid fraction and a corresponding increase of polyunsaturated fatty acids of the total fatty acids produced by that plant, there remains a need in the art to fully understand the genotype of oleic acid producing plants and seeds, to be able to optimally achieve a phenotype with maximal production of the this valuable commodity. The present invention meets that need.

On the other hand there is a growing body of evidence that the polyunsaturated fatty acids may be beneficial to health, for example in the areas of cardio-protection and brain development and tissue repair. There is also therefore a need in the art to fully understand the genotype of PUFA producing plants and seeds, to be able to optimally achieve a phenotype with maximal production of the this valuable commodity. The present invention meets that need.

Lubricants are a key element for industrial and transport applications. More than 95% of the market is currently satisfied by low-cost mineral oils. As 30% of lubricants inevitably end up in the ecosystem, natural based (bio)lubricants offer a potentially attractive alternative. However, there is a need to develop more economically sustainable means of producing such (bio)lubricants. Moreover degradation problems are associated with the use of a natural oil which is high in PUFAs. The present invention seeks to address these issues.

### SUMMARY OF THE INVENTION

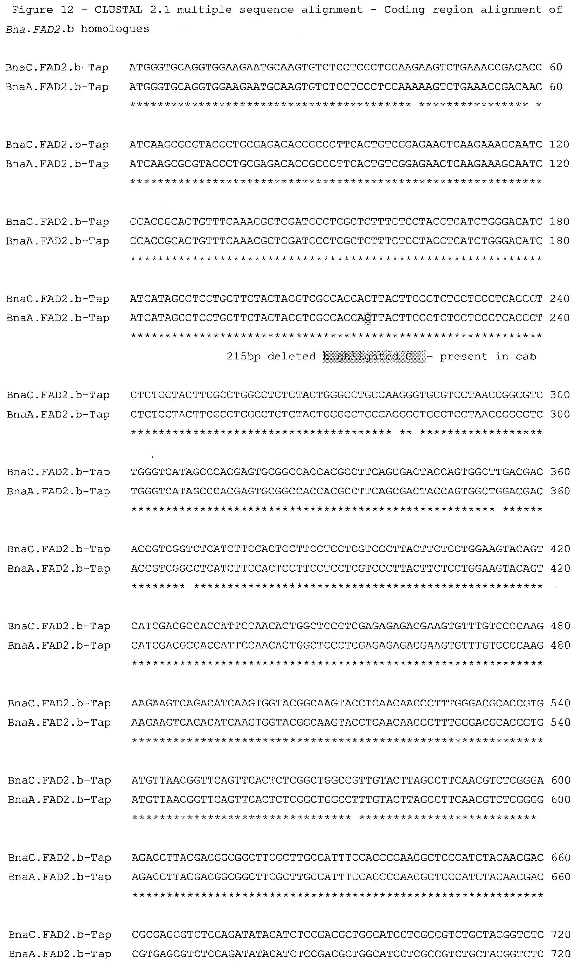

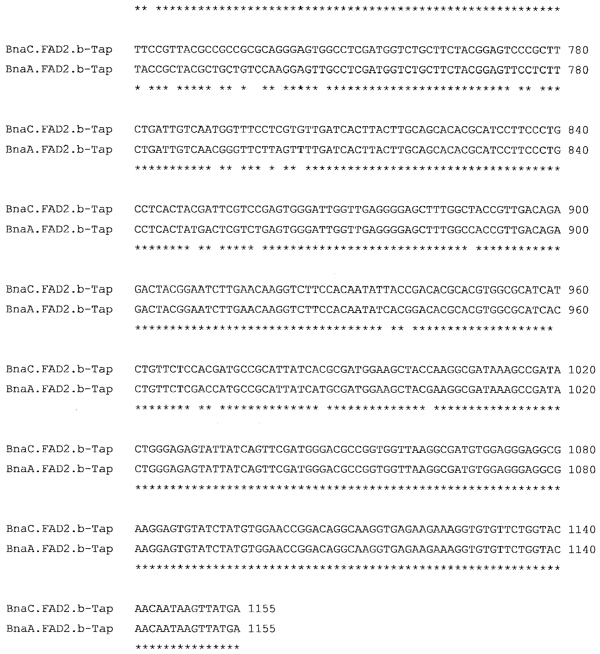

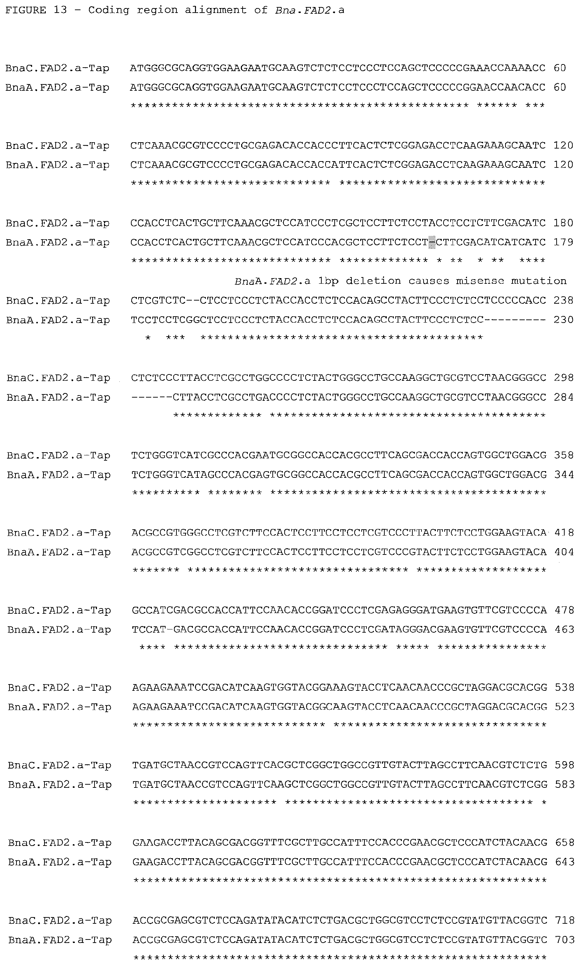

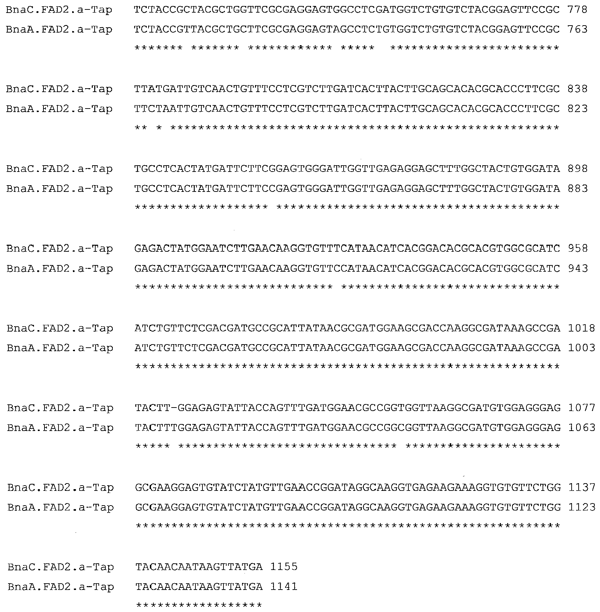

For the first time, the present invention provides a complete survey of the genome for homologues of *FAD2* and their characterization in a given germplasm of *Brassica napus,* and reports that there are, in fact, four *FAD2* genes in any given genotype, present as homologous pairs of genes on sister chromosomes from the A and C genomes, namely *BnaA.FAD2.a* (chromosome A1), *BnaC.FAD2.a* (chromosome C1), *BnaA.FAD2.b* (chromosome A5), *BnaC.FAD2.b* (chromosome C5), and that in any given germplasm, one or more of the genes are active, thereby reducing the total percentage of oleic acid achievable in the total fatty acids produced in that germplasm. Examples of sequences of these four genes can be seen in Figures 12 and 13 and these also form part of the present invention. Figure 12 shows ∼97% sequence similarity. The sequences are difficult to separate via homologue-specific PCR due to this high level of sequence conservation, hence providing details of each of these sequences is an important contribution to the art. Such sequences form part of the invention.

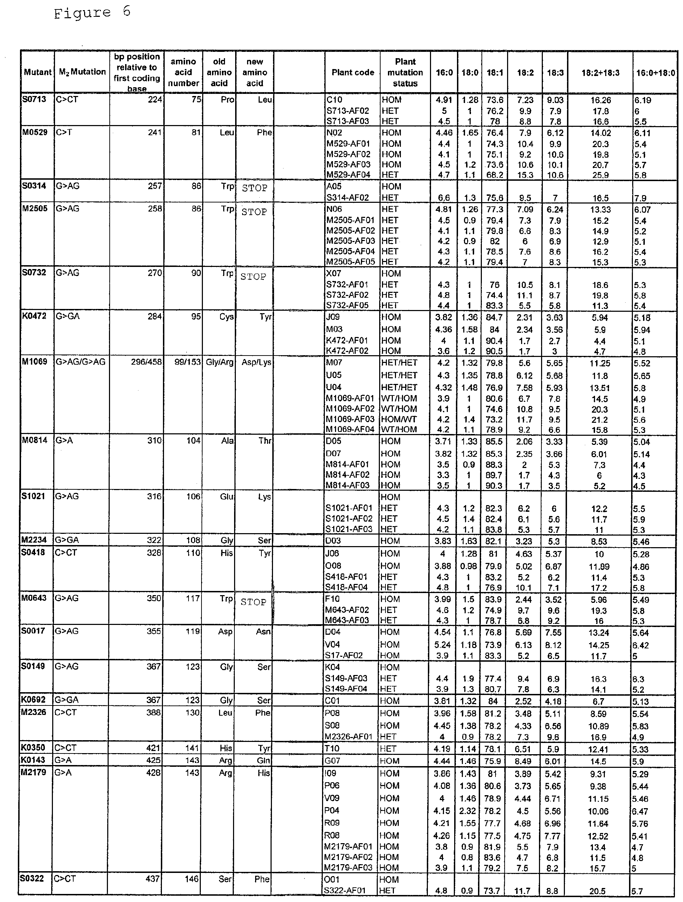

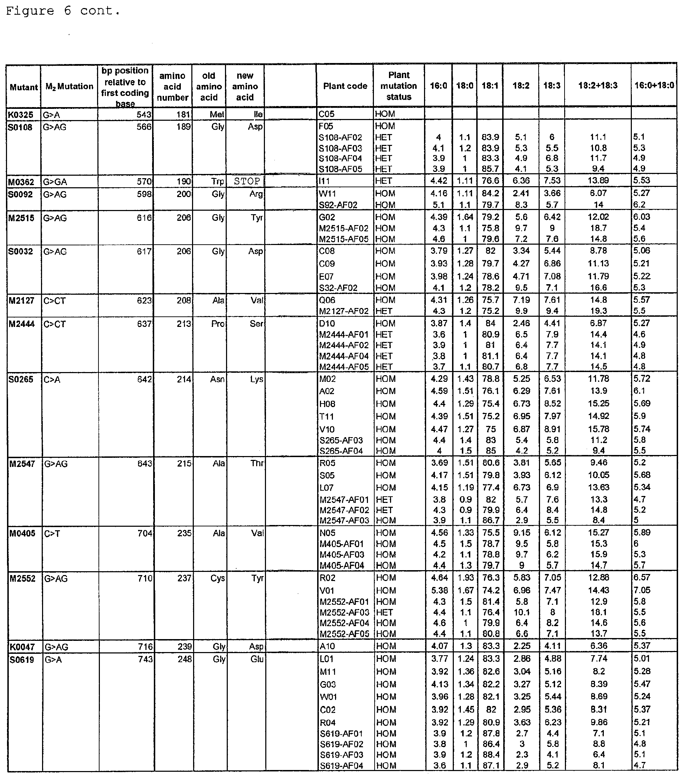

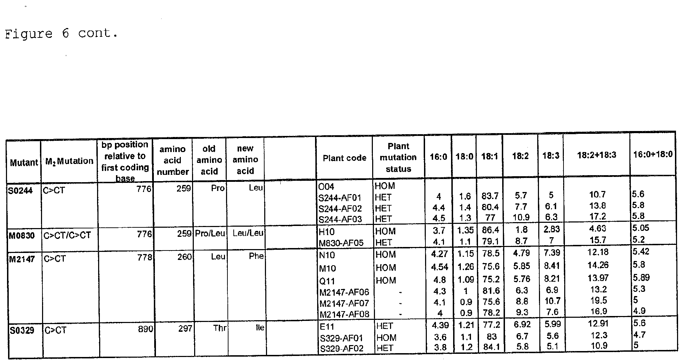

The inventors have produced a novel series of modifications in the genome of various *Brassica napus* germplasms and provide a germplasm with a compromised and/or totally inactive set of *FAD2* genes. A wide variety of new mutations in the *FAD2* homologue *BnaC.FAD2.b* are provided which compromise or abolish activity of the enzyme encoded by that gene. These mutations include, but are not limited to: conversion of a codon encoding an amino acid to a stop codon; conversion of a codon to encode an alternative amino acid; and the like. As exemplified in Figure 6, a wide number of single nucleotide modifications, deletions, insertions or the like are available to achieve these objectives, including, but not limited to, the specific nucleotide changes (and the resultant amino acid changes) and combinations thereof. By producing germplasm comprising any one or a combination of these mutations in *BnaC.FAD2.b* of *B. napus,* it is possible, depending on the starting genotype of the *B. napus* variety used, to produce a *B. napus* germplasm in which there is no active FAD2 enzyme activity at all. Thus, for example, starting with variety Cabriolet, which we have found has one active homologue of *FAD2,* namely *BnaC.FAD2.b,* while the other three homologues are not active, we have been able to produce a new variety of *B. napus* which has no active FAD2 whatsoever. Those skilled in the art will appreciate, therefore, that any given germplasm may be utilized as starting material and the work disclosed herein reproduced to compromise the activity of the homologue equivalent in that variety to *BnaC.FAD2.b* of *B. napus,* and this will produce a new variety in which that homologue is compromised in activity. Used in combination with known mutations or varieties in which any of the other active homologues are likewise inactivated or compromised, new varieties are producible which have some, little or no delta-12 oleate desaturase activity.

Accordingly, it is a first object of this invention to provide a novel germplasm for the efficient production of a desirable fatty acid composition in plants cultivated for this purpose. The desirable fatty acid composition is one which has a relatively high level of monounsaturated fatty acid, in more detail a relatively high level of oleic acid. Preferably the desirable fatty acid composition has a relatively low level of PUFA, in more detail a relatively low level of linoleic and/or linolenic fatty acid.

In a second aspect of the present invention, however, the desirable fatty acid composition is one which has a relatively high level of polyunsaturated fatty acid, in more detail a relatively high level of linoleic and/or linoleic acid. Preferably the desirable fatty acid composition has a relatively low level of monounsaturated fatty acid, in more detail a relatively low level of oleic.

It is a further object of this invention to provide plants and particularly a *Brassica napus* with a totally inactivated FAD2.

It is a further object of this invention to provide methods for producing the desirable fatty acid content in a plant and to provide tools, such as primers and genetic markers, to enable a skilled worker to select such a plant.

### STATEMENTS OF THE INVENTION

According to the first aspect of the present invention, there is provided a mutated nucleic acid sequence of a *Brassica* FAD2-encoding nucleic acid sequence, wherein said nucleic acid sequence has one or more of the mutations shown in Figure 6 in relation to the *BnaC.FAD2.b* gene, or a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or a complementary sequence of one of said mutated, homologous or hybridizing nucleic acid sequences.

The mutation may be one of the following mutations:
224C to T (75 Pro to Leu), 241C to T (81 Leu to Phe),
257G to A (86 Typ to **Stop**)**,** 258G to A (86 Trp to **Stop**),
270G to A (90 Trp to **Stop**), 284G to A (95 Cys to Tyr),
296G to A (99 Gly to Asp), 310G to A (104 Ala to Thr),
316G to A (106 Glu to Lys), 322G to A (108 Gly to Ser),
328C to T (110 His to Tyr), 350G to A (117 Trp to **Stop**),
355G to A (119 Asp to Asn), 367G to A (123 Gly to Ser),
388C to T (130 Leu to Phe), 421C to T (141 His to Tyr),
425G to A (143 Arg to Gln), 428G to A (143 Arg to His),
437C to T (146 Ser to Phe), 458G to AG (153 Arg to Lys),
543G to A (181 Met to Ile), 566G to A (189 Gly to Asp),
570G to A (190 Trp to **Stop**), 598G to A (200 Gly to Arg),
616G to A (206 Gly to Tyr), 617G to A (206 Gly to Asp),
623C to T (208 Ala to Val), 637C to T (213 Pro to Ser),
642C to A (214 Asn to Lys), 643G to A (215 Ala to Thr),
704C to T (235 Ala to Val), 710G to A (237 Cys to Tyr),
716G to A (239 Gly to Asp), 743G to A (248 Gly to Glu),
776C to T (259 Pro to Leu), 778C to T (260 Leu to Phe),
890C to T (297 Thr to Ile).

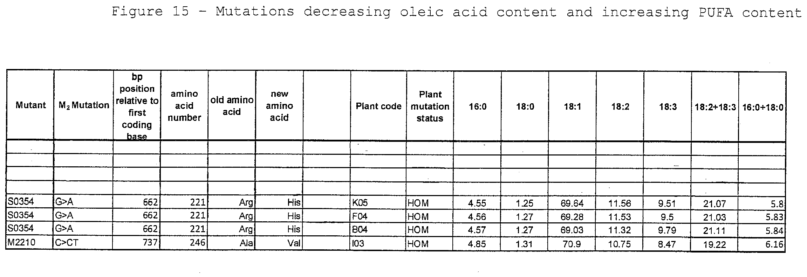

In one embodiment, in relation to the *BnaA.FAD2.b* gene, the mutation is not one of the mutations shown in Figure 15, i.e. is not 662G to A (221 Arg to His) or 737C to T (246 Ala to Val).

According to the first aspect of the present invention there is also provided a mutated nucleic acid sequence of a *Brassica* FAD2-encoding nucleic acid sequence, wherein said nucleic acid sequence encodes a FAD2 enzyme in which amino acid position 241 and/or 246 are changed in relation to the FAD2 enzyme encoded by *BnaC.FAD2.b* gene, or
a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or
a complementary sequence of one of said mutated, homologous or hybridizing nucleic acid sequences.

Also according to the first aspect of the present invention there is provided a mutated nucleic acid sequence of a *Brassica* FAD2-encoding nucleic acid sequence, wherein said nucleic acid sequence has a 1bp deletion at coding base position 215 in relation to the *BnaA.FAD2.b* gene, or
a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or
a complementary sequence of one of said mutated, homologous or hybridizing nucleic acid sequences.

The mutations of the first aspect of the present invention are associated with the first desirable fatty acid composition in which the level of monounsaturated fatty acid, and preferably oleic acid, is increased.

On the other hand according to a second aspect of the present invention, there is provided a mutated nucleic acid sequence of a *Brassica* FAD2-encoding nucleic acid sequence, wherein said nucleic acid sequence has one or more of the mutations shown in Figure 15, i.e. preferably 662G to A (221 Arg to His) and/or 737C to T (246 Ala to Val) in relation to the *BnaC.FAD2.b* gene, or
a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence encodes an active FAD2 enzyme, or a hybridizing nucleic acid sequence which hybridizes under stringent conditions to said mutated nucleic acid sequence, provided that the complementary sequence of said hybridizing nucleic acid sequence encodes an active FAD2 enzyme, or
a complementary sequence of one of said mutated, homologous or hybridizing nucleic acid sequences.

The homology level may be at or at least 95%, 97%, 98% or 99% identity.

The mutations of the second aspect of the present invention are associated with the second desirable fatty acid composition in which the level of polyunsaturated fatty acid, and preferably linoleic and/or linolenic acid, is increased.

In one embodiment, the mutations are not those of the first aspect of the invention.

FAD2 as referred to herein means delta-12 oleate desaturase enzyme.

As used herein, the phrase "nucleic acid" refers to any physical string of monomer units that can be corresponded to a string of nucleotides, including a polymer of nucleotides (e.g., a typical DNA, cDNA or RNA polymer), modified oligonucleotides (e.g., oligonucleotides comprising bases that are not typical to biological RNA or DNA, such as 2'-O-methylated oligonucleotides), and the like. In some embodiments, a nucleic acid can be single-stranded, double-stranded, multi-stranded, or combinations thereof. Unless otherwise indicated, a particular nucleic acid sequence of the presently disclosed subject matter optionally comprises or encodes complementary sequences, in addition to any sequence explicitly indicated.

Mutations as referred to herein include addition, deletion, substitution, modification, replacement and/or variation of at least one nucleotide. The mutated nucleic acid sequences may be truncated. Preferably the mutation will cause addition, deletion, substitution modification replacement and/or variation of at least one amino acid residue present in the FAD2 encoded by the mutated nucleic acid sequence. For example, mutations in coding sequences may be made so as to introduce substitutions within functional motifs in FAD2. Mutations in the nucleic acid sequences may cause the production of a truncated FAD2 amino acid sequence.

A mutation may cause inactivation of the mutated nucleic acid sequence or inactivation of the FAD2 enzyme encoded by said mutated nucleic acid sequence.

Mutations may be introduced using synthetic oligonucleotides. These oligonucleotides contain nucleotide sequences flanking the desired mutation sites. A suitable method is disclosed in Morinaga et al., (Biotechnology (1984) 2, p646-649). Another method of introducing mutations into enzyme-encoding nucleotide sequences is described in Nelson and Long (Analytical Biochemistry (1989), 180, p 147-151).

The present invention also encompasses sequences that are complementary to the nucleic acid sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

In one embodiment, the present invention encompasses sequences that are complementary to sequences that are capable of hybridising under stringent conditions (e.g. 50°C and 0.2xSSC {1xSSC = 0.15 M NaCl, 0.015 M Na₃citrate pH 7.0}) to the nucleotide sequences presented herein.

More preferably, the the present invention encompasses sequences that are complementary to sequences that are capable of hybridising under high stringent conditions (e.g. 65°C and 0.1xSSC {1xSSC = 0.15 M NaCl, 0.015 M Na₃citrate pH 7.0}) to the nucleotide sequences presented herein.

The present invention also relates to nucleotide sequences that can hybridise to the nucleotide sequences of the present invention (including complementary sequences of those presented herein).

The present invention also relates to nucleotide sequences that are complementary to sequences that can hybridise to the nucleotide sequences of the present invention (including complementary sequences of those presented herein).

Also included within the scope of the present invention are polynucleotide sequences that are capable of hybridising to the nucleotide sequences presented herein under conditions of intermediate to maximal stringency.

In a preferred aspect, the present invention covers nucleotide sequences that can hybridise to the nucleotide sequence of the present invention, or the complement thereof, under stringent conditions (e.g. 50°C and 0.2xSSC).

In a more preferred aspect, the present invention covers nucleotide sequences that can hybridise to the nucleotide sequence of the present invention, or the complement thereof, under high stringent conditions (e.g. 65°C and 0.1xSSC).

The mutations have been described with reference to a reference sequence but it will be appreciated that the mutations may be at a corresponding position in another sequence. Thus it will be appreciated that the nucleotide referred to may differ in the indicated number but still have similar neighbouring nucleotides.

It will be appreciated that mutated nucleic acid sequences of the present invention can be introduced into plants or parts thereof using routine techniques. Thus, the present invention also encompasses vectors, host cells, plants and parts of plants comprising the mutated nucleic acid sequences. The introduction of the nucleic acid sequences into plants or parts thereof is more particularly relevant to the second aspect of the present invention where one is in general seeking to increase the FAD2 enzymatic activity to increase the level of polyunsaturated fatty acid.

According to a further aspect of the present invention, there is provided a fragment of a mutated nucleic acid sequence of the invention which comprises a specified mutation. Preferred fragments include fragments having at least 5, 10, 15, 20, 30, 40, 50 or 100 contiguous nucleic acid from a mutated nucleic acid sequence of the present invention or fragments having at least 5, 10, 15, 20, 30, 40, 50 or 100 contiguous nucleic acids truncated or deleted from a mutated nucleic acid sequences of the present invention. A fragment may be part of a longer nucleic acid sequence. In one embodiment the fragment comprises at least 5 contiguous wild-type bases on either side of the mutation according to the present invention.

Preferably the fragment is identical in sequence to a portion of a modified *BnaC.FAD2.b* gene.

Preferably the fragment is selected from the group consisting of
*SEQ ID. 52*: GTCTCCTCCCTCCAAAAAGT
*SEQ ID. 53:* GTGTCTCCTCCCTCCAAA
*SEQ ID. 54*: CTACAGAAACAAACATGGGC
*SEQ ID. 55:* GTCTCTCCTCCCTCCAGC
*SEQ ID. 56:* CTCTCCTCCCTCCAGCTCCC
*SEQ ID. 57*: CTCTTCGACATCCTCCTCTC
*SEQ ID. 58:* CCTCGTCCCTTACTTCTCCTG
*SEQ ID. 59:* CCTCATAACTTATTGTTGTACCAG
*SEQ ID. 60:* CAAGACGACCAGAGACAGC
*SEQ ID. 61:* GAACTCGACAAATTTGCCTG
SEQ ID. 62: GGGTGCAGGTGGAAGAATG probe f,
SEQ ID. 63: TTGTTGTACCAGTACACACC probe r,
SEQ ID. 64: conserved f GAGGGAGGCGAAGGAGTGTATC,
SEQ ID. 65: conserved r CAGGAGAAGTAAGGGACGAGG,
SEQ ID. 66: degenerate f ATTCCTTCCTNCTNCTNGTNCC,
SEQ ID. 67: degenerate r GCTAAGTACAANGGNCANCC.

The nucleic acids of the present invention, including the fragments thereof, can usefully be used as markers, primers or probes. They can usefully be employed in combination.

The nucleic acids of the present invention can thus be used as markers in the identification of genes encoding desirable phenotypic traits in *Brassica.* The present invention thus allows a skilled worker to advantageously reliably breed for the markers of the present invention and hence reliably breed for an economically important fatty acid composition.

In one aspect, the invention provides use of a nucleic acid sequence to guide site-specific mutation in a regulatory region of a *FAD2* gene. For example, sequence from the upstream region of the *FAD2* gene may be used to guide site-specific mutations in the *FAD2* regulatory region such as the TATA box in order to down-regulate expression of the *FAD2* gene. Similarly, sequence from the upstream region of the *FAD2* gene could be used to guide site-specific mutations in the *FAD2* regulatory region to down-regulate expression of the *FAD2* gene. This may be done *in vitro* or *in vivo.*

In one aspect, the invention provides amplification primers or probes that may be used to identify *FAD2* nucleic acid sequences of the invention, or the region upstream from the genes from other nucleic acid sequences. For example, primers or probes may be synthesised that are complementary to portions of the naturally occurring oleate desaturase coding sequence. Selected primers may be capable of distinguishing plants having high oleic acid content from plants having low oleic acid content or vice versa.

In another aspect of the invention, selective hybridisation and amplification, using *FAD2* locus-specific probes and primer pairs of the invention, may be used to generate an amplification pattern that may contribute to a collection of DNA fingerprints to identify the *FAD2* genotype of a germ-plasm. *FAD2* probes may for example include primers or probes synthesised from complementary portions of the naturally occurring coding sequences of the oleate desaturase *FAD2* genes and from complementary portions upstream of the *FAD2* genes.

According to the first aspect the invention comprises a method of selecting plants having a high oleic acid content by utilizing PCR primers to selectively amplify a desired gene. This method may be used, for example, to ensure the selected progeny carry a desired coding sequence conferring a high oleic acid oil phenotype. According to the second aspect the invention comprises a method of selecting plants having a high PUFA content by utilizing PCR primers to selectively amplify a desired gene. This method may be used, for example, to ensure the selected progeny carry a desired coding sequence conferring a high PUFA oil phenotype.

In accordance with an embodiment of the method, seedlings of a first segregating backcross population, may be subjected to a PCR analysis to detect the mutant *FAD2* nucleic acid, and the selected plants backcrossed again to a recurrent parental line. The backcrossing and PCR analysis of the first seedling population may, for example, proceed through at least two more cycles to create a third segregating backcross seedling population, which may be self-pollinated to create a third seedling population. The third seedling population may be subjected to PCR analysis for the mutant nucleic acid, and homozygotes may be selected for further pedigree breeding, such as breeding of an elite, high oleic acid content strain.

In another aspect there is also provided a method of selecting plants having a desired fatty acid content comprising the following steps:
crossing a plant or part thereof of the present invention with a parent plant;
selecting from the progeny plants or parts thereof those comprising at least one sequence of the present invention.

Crossing can be carried out using methods well known to skilled workers.

In one embodiment the selection is carried out using a nucleic acid of the present invention, in particular a fragment thereof.

The present invention also relates to a plant obtainable using the methods of the present invention.

The present invention also relates to a kit comprising a nucleic acid sequence or fragment of the present invention, particularly for the selection of plants with a desirable fatty acid profile.

In another embodiment of the first aspect the present invention relates to a method of providing a plant having increased monounsaturated fatty acid content, particularly oleic acid content, comprising the steps of:
inactivating at least one FAD2-encoding nucleic acid sequence of the present invention in a plant or part thereof,
selecting a plant or part thereof or regenerated plant comprising said inactivation.

In a preferred method of producing a plant which is subject to the inactivation is pre-selected such that it contains only one active *FAD2* gene or enzyme.

The present invention enables a skilled worker to provide a new plant in which all nucleic acid sequences encoding FAD2 or the enzyme are inactivated.

Thus in one preferred embodiment of the first aspect the present invention relates to a method of producing a plant or a part thereof, said method selected from the group consisting of (a) silencing each of the homologues of the gene encoding that enzyme (b) producing mutants in the genome to reduce or destroy the activity of any encoded enzyme (c) selecting a variety of *Brassica* in which only one active *FAD2* gene exists, mutating that gene and producing a new variety in which all genes encoding oleate-12 desaturase are modified as compared with wild-type of that variety such that any oleate desaturase protein encoded is compromised in activity or is devoid of activity and (d) marker assisted selection of crosses between particular varieties to produce a variety which encodes little or no active FAD2 enzymatic activity.

In another embodiment of the first aspect the present invention relates to a method of providing a plant having increased PUFA content, particularly linoleic acid and/or linolenic fatty acid content, comprising the steps of:
activating at least one FAD2-encoding nucleic acid sequence of the present invention in a plant or part thereof,
selecting a plant or part thereof or regenerated plant comprising said activation.

Thus in one preferred embodiment of the second aspect the present invention relates to a method of producing a plant or a part thereof, said method selected from the group consisting of (a) introducing or upregulating expression of more or more of the homologues of the gene encoding that enzyme (b) producing mutants in the genome to increase the activity of any encoded enzyme (c) selecting a variety of *Brassica* in which a *FAD2* gene exists, mutating that gene and producing a new variety in which a gene and preferably all genes encoding oleate-12 desaturase are modified as compared with wild-type of that variety such that any oleate desaturase protein encoded is improved or gains activity and (d) marker assisted selection of crosses between particular varieties to produce a variety which encodes active FAD2 enzymatic activity.

By activated we include that the protein has FAD2 enzymatic activity. By activating we include that the protein has increased FAD2 activity compared to the parent protein or the plant or part thereof.

According to another aspect of the present invention there is provided a protein or polypeptide comprising an amino acid sequence encoded by a mutated nucleic acid sequences of the present invention or fragment thereof.

The protein or polypeptide may be a fragment of FAD2.

The mutated nucleic acid sequences and fragments thereof, as well as proteins or polypeptides encoded by said mutated nucleic acid sequences and fragments may be used to produce the desirable fatty acid composition of a plant or part thereof.

According to further aspect of the present invention there is provided a plant or part thereof in which more than two FAD2-encoding nucleic acid sequences or the corresponding FAD2 enzymes are inactivated such that the enzymatic activity is reduced compared to the non-inactivated version.

In one embodiment, the plant or part thereof has more than three FAD2-encoding nucleic acid sequences or the corresponding FAD2 enzymes are inactivated.

In another embodiment, the plant or part thereof has all of the FAD2 enzymes or the nucleic acid sequences encoding therefore are inactivated.

Plants or parts thereof according to the invention are provided in which the nucleic acid sequence is one or more of the mutated nucleic acid sequences of the invention or fragments thereof.

It will be appreciated that the aspects of the present invention which are based on the technical teaching of the surprising number of *FAD2* genes are generally applicable, particularly for all oil producing crops. Such crops include sunflower, soybean, palm, corn etc., but are preferably *Brassica* species. *Brassica* is particularly preferred where the present invention involves the sequences and mutations of the present invention.

The *Brassica* plant of part thereof according to the invention may be selected from the group consisting of *Brassica juncea, napus, rapa, oleracea, campestris, carinata.*

The *Brassica* plant of part thereof according to the invention may be *Brassica napus.* In one embodiment, the *Brassica napus* plant or part thereof is derivable from the variety Cabriolet or Tapidor.

In another embodiment there is provided a *Brassica napus* plant or part thereof in which each of the four FAD2-encoding nucleic acid sequences or the corresponding FAD2 enzymes are inactivated. The nucleic acid sequences may be selected from the group consisting of:
*BnaA.FAD2.a*
*BnaC.FAD2.a*
*BnaA.FAD2.b* or
*BnaC.FAD2.b.*

The FAD2-encoding nucleic acid sequences or the corresponding FAD2 enzymes may be inactivated to increase the 18:1 (oleic acid) content of the *Brassica* plant or part thereof.

The FAD2-encoding nucleic acid sequences or the corresponding FAD2 enzymes may be inactivated to reduce the polyunsaturated fatty acid (PUFA) content of the *Brassica* plant or part thereof. Preferably, the 18:2 and 18:3 fatty acid content of the *Brassica* plant or part thereof is reduced.

As used herein in general terms "inactivation" refers to a nucleic acid which does not encode a functional FAD2 protein or it refers to a non-functional FAD2 protein and which FAD2 protein has an activity which is lower than that of the corresponding FAD2 protein, usually the natural FAD2 protein, measured in the same conditions, preferably the FAD2 protein has no enzymatic activity.

Inactivated as referred to herein refers to the outcome of methods which include *FAD2* gene silencing and the reduction or elimination of expression of a nucleic acid sequence that encodes FAD2. By elimination of expression, it is meant herein that a functional amino acid sequence encoded by the nucleic acid sequence is not produced at a detectable level. By a reduction of expression, it is meant herein that a functional amino acid sequence encoded by the nucleic acid sequence is produced at a level that is reduced compared to when a FAD2 encoding sequence is not inactivated.

Inactivation may include the reduction or elimination of transcription of a nucleic acid sequence that encodes FAD2. By elimination of transcription it is meant herein that the mRNA sequence encoded by the nucleic acid sequence is not transcribed at detectable levels. By reduction of transcription it is meant herein that the mRNA sequence encoded by the nucleic acid sequence is transcribed at levels that are reduced compared to when a FAD2 encoding sequence is not inactivated.

Inactivation may include the reduction or elimination of translation of a nucleic acid sequence that encodes FAD2. By elimination of translation it is meant herein that the mRNA sequence encoded by the nucleic acid sequence is not translated at detectable levels. By reduction of translation it is meant herein that the mRNA sequence encoded by the nucleic acid sequence is translated at levels that are reduced compared to when a FAD2 encoding sequence is not inactivated.

Inactivation may include the reduction or elimination of FAD2 enzyme activity. By elimination of FAD2 enzyme activity it is meant herein that the enzyme has no detectable activity. By reduction of FAD2 enzyme activity it is meant herein that the enzyme activity is reduced compared to when FAD2 is not inactivated. A reduction in FAD2 enzyme activity may be measured by measuring the corresponding increase in for example 18:1 (oleic acid) content in a plant or part thereof or another desirable fatty acid composition trait.

Inactivation may also include the production of a truncated amino acid sequence from a nucleic acid sequence that encodes FAD2. By production of a truncated amino acid sequence it is meant herein that the amino acid sequence encoded by the nucleic acid sequence is missing one or more amino acids of the functional amino acid sequence encoded by a wild type nucleic acid sequence. In addition, inactivation may include the production of a variant FAD2 amino acid sequence. By production of a variant amino acid sequence it is meant herein that the amino acid sequence has one or more amino acids that are different from the amino acid sequence encoded by a FAD2 nucleic acid sequence that has not been inactivated.

In general, unless otherwise specified, when referring to a "plant" it is intended to cover a plant at any stage of development, including cells and seeds and germplasm.

A plant "part thereof" includes leaves, stems, roots, flowers or flower parts, fruits, pollen, egg cells, zygotes, seeds, cuttings, cell or tissue cultures, or any other part or product of a plant.

Advantageously the plants or parts of the present invention have the desired fatty acid composition of the first aspect of the present invention, i.e. an increased monounsaturated fatty acid content, particularly an increased oleic acid content and/or a reduced PUFA character, particularly a reduced linolenic and/or linoleic fatty acid content, as compared to otherwise genetically identical plants, i.e. plant which are identical except for the presence of the mutation, and/or to corresponding wild-type plants.

Advantageously the plants or parts of the present invention have the desired fatty acid composition of the second aspect of the present invention, i.e. an increased polyunsaturated fatty acid content, particularly an increased linoleic acid content and/or linolenic acid content, and/or a reduced monounsaturated fatty acid character, particularly a reduced oleic fatty acid content, as compared to otherwise genetically identical plants, i.e. plant which are identical except for the presence of the mutation, and/or to corresponding wild-type plants.

Regeneration of plants from the plants or part thereof of the present invention can be carried out using methods well known to a skilled worker. Thus, the present invention also extends to the progeny of the plants, preferably such progeny also have the desirable fatty acid composition.

A plant or part according to the invention may be a cell or seed.

In one embodiment of the first aspect of the present invention there is provided a seed the fatty acid composition of which is greater, by at least 5%, than 74.5%, 74.7%, 75%, 76%, 77%, 78%, 79%, 80%, 81%, 82%, 83%, 84% or 85% oleic acid and which preferably also has an 18:2 and/or 18:3 fatty acid fraction which is less by at least 5% than 16.1%, 15.9%, 15.7% down to around 6%.

In one embodiment of the first aspect there is provided a seed of a plant that has at least one FAD2-encoding nucleic acid sequence or a FAD2 enzyme inactivated, wherein the oleic acid content of the seed is greater, by at least 5, 10, 15, 20, 25, 30, 50, 75, 100%, than the seed of a plant which does not have a FAD2-encoding nucleic acid sequence or a FAD2 enzyme inactivated.

In one embodiment of the first aspect there is provided a seed of a plant that has at least one FAD2-encoding nucleic acid sequence or a FAD2 enzyme inactivated, wherein the PUFA content and/or 18:2 and/or 18:3 fatty acid content of the seed is less by at least 5, 10, 15, 20, 25, 30, 50, 75, 100% than the seed of a plant which does not have a FAD2-encoding nucleic acid sequence or a FAD2 enzyme inactivated.

In one embodiment of the first aspect there is provided a seed of a plant that has at least one FAD2-encoding nucleic acid sequence or a FAD2 enzyme inactivated, wherein the oleic acid content of the seed is greater, by at least 5, 10, 15, 20, 25, 30, 50, 75, 100%, than the seed of a plant which does not have a FAD2-encoding nucleic acid sequence or a FAD2 enzyme inactivated, and wherein the PUFA content and/or 18:2 and/or 18:3 fatty acid content of the seed is less by at least 5, 10, 15, 20, 25, 30, 50, 75, 100% than the seed of a plant which does not have a FAD2-encoding nucleic acid sequence or a FAD2 enzyme inactivated.

In one embodiment of the second aspect of the present invention there is provided a seed the fatty acid composition of which is less, by at least 5%, than 74.5%, 74.7% or 75% oleic acid and which preferably also has an 18:2 and/or 18:3 fatty acid fraction which is greater by at least 5% than 16.1%, 15.9%, 15.7%. It could be envisaged that there is provided a seed the fatty acid composition of which is greater, by at least 5%, than 74.5%, 74.7%, 75%, 76%, 77%, 78%, 79%, 80%, 81%, 82%, 83%, 84% or 85% PUFA (preferably 18:2 and 18:3) and which preferably also has an 18:1 fatty acid fraction which is less by at least 5% than 16.1%, 15.9%, 15.7% down to about 6%.

In one embodiment of the second aspect there is provided a seed of a plant that has at least one FAD2-encoding nucleic acid sequence or a FAD2 enzyme, wherein the linoleic acid content and/or linolenic acid content of the seed is greater, by at least 5, 10, 15, 20, 25, 30, 50, 75, 100%, than the seed of a plant which does not have a FAD2-encoding nucleic acid sequence or has an FAD2 enzyme inactivated.

In one embodiment of the second aspect there is provided a seed of a plant that has at least one FAD2-encoding nucleic acid sequence or a FAD2 enzyme, wherein the PUFA content and/or 18:2 and/or 18:3 fatty acid content of the seed is increased by at least 5, 10, 15, 20, 25, 30, 50, 75, 100% than the seed of a plant which does not have a FAD2-encoding nucleic acid sequence or has an FAD2 enzyme inactivated.

In one embodiment of the second aspect there is provided a seed of a plant that has at least one FAD2-encoding nucleic acid sequence or a FAD2 enzyme, wherein the linoleic acid and/or linolenic acid content of the seed is greater, by at least 5, 10, 15, 20, 25, 30, 50, 75, 100%, than the seed of a plant which does not have a FAD2-encoding nucleic acid sequence or has an FAD2 enzyme inactivated, and
wherein the monounsaturated fatty acid content and/or 18:1 fatty acid content of the seed is less by at least 5, 10, 15, 20, 25, 30, 50, 75, 100% than the seed of a plant which does not have a FAD2-encoding nucleic acid sequence or has an FAD2 enzyme inactivated.

The seeds of the present invention are preferably *Brassica* seeds. These seeds according to the present invention may be from a *Brassica* plant selected from the group consisting of *Brassica juncea, napus, rapa, oleracea, campestris, carinata.*

A *Brassica* seed according to the present invention may be a *Brasica napus* seed.

According to a further aspect of the present invention there is provided fatty acid, preferably comprising oleic acid, produced from the seed or the *Brassica* plant according to the present invention. The present invention extends to a purified fatty acid composition as well as the directly extracted composition, and to the individual fatty acids present in the fatty acid composition.

According to a further aspect of the present invention there is provided a (bio)lubricant containing the fatty acid composition of the present invention.

Other objects and advantages of this invention will be appreciated by those skilled in the art from a review of the complete disclosure provided herein and the appended claims.

### BRIEF DESCRIPTION OF THE DRAWINGS

Figure 1 - PCR primer positions on the various Cabriolet *FAD2* homologues.

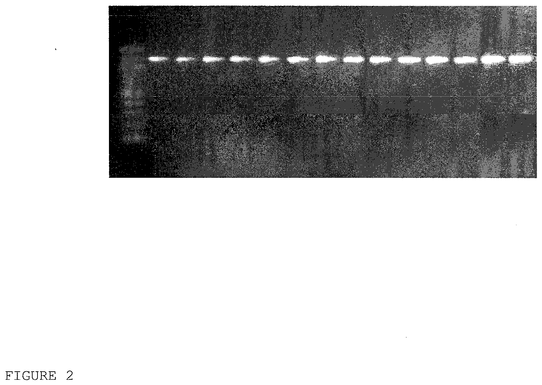

Figure 2 - Agarose gel showing *BnaC.FAD2.b* specific PCR used for mutation load screen on individuals from the population.
Figure 3 - Agarose gel showing homologue specific PCR on Tapidor (T) and Cabriolet (C), a) *BnaA.FAD2.b, b)BnaC.FAD2.a,* no amplification is seen in Cabriolet which does not possess this locus, c) and d) *BnaA.FAD2.a* generic PCR amplifies both the chromosome A1 and C1 homologues of the gene, e)*BnaA.FAD2.a*
Figure 4 shows the alignment of Tapidor and Cabriolet sequences against the different homologues. Note amplification of Cabriolet *BnaA.FAD2.a* with *BnaC.FAD2.a* primers under touchdown condition (trace highlighted).
Figure 5 mixed amplicon of Cabriolet *BnaC.FAD2.b* and *BnaA.FAD2.b* caused by the 1bp deletion occurring in *BnaA.FAD2.b* at base 160
Figure 6 shows the oleic acid (18:1) fraction compared to other fatty amino acid percentages in wild type and mutants produced according to this invention after the first year of trialling
Figure 7 - CLUSTAL 2.0.12 multiple sequence alignment Tapidor genomic
Figure 8 - CLUSTAL 2.0.12 multiple sequence alignment Tapidor protein
Figure 9 - CLUSTAL 2.0.12 multiple sequence alignment between the nucleotide sequences of Tapidor and Cabriolet
Figure 10 - alignment of Cabriolet and Tapidor Amino Acid sequences
Figure 11 - Mutants aligned against BnaA.FAD2.b from Cabriolet an Tapidor - 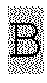 =poor quality bases **S**=position of mutation
Figure 12 - CLUSTAL 2.1 multiple sequence alignment - Coding region alignment of A5 and C5 *FAD2* homologues, *BnaA.FAD2.b* and *BnaC.FAD2.b*
FIGURE 13 - Coding region alignment of A1 and C1 *FAD2* homologues, *BnaA.FAD2.a* and *BnaC.FAD2.a*

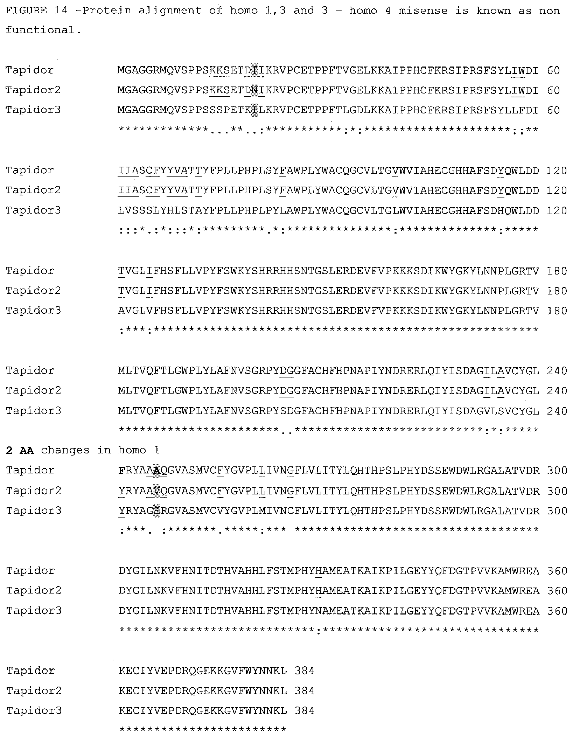

FIGURE 14 - Protein alignment of the three functional FAD2 proteins from Tapidor, BnaC.FAD2.b, BnaA.FAD2.b and BnaC.FAD2.a
FIGURE 15 - Mutations decreasing oleic acid content and increasin PUFA content

### DETAILED DISCLOSURE OF THE PREFERRED EMBODIMENTS OF THE INVENTION

The practice of the present invention will employ, unless otherwise indicated, conventional techniques of chemistry, molecular biology, microbiology, recombinant DNA and plant biology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Col d Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992. Each of these general texts is herein incorporated by reference.

In the present patent disclosure, we characterize the complete genotype of a *Brassica napus* germplasm, we identify four *FAD2* homologues, and we provide a series of mutants in which all *FAD2* homologues are inactivated. As a result, we provide certain isolates which exhibit production of high oleic acid in the rapeseed product, with, in certain instances, production in excess of 90% of the total fatty acid being oleic acid.

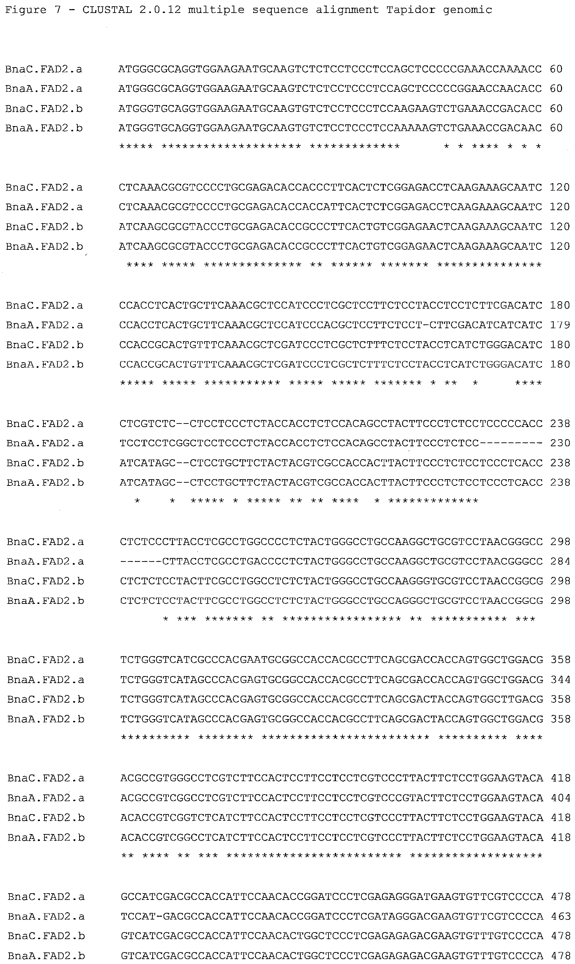

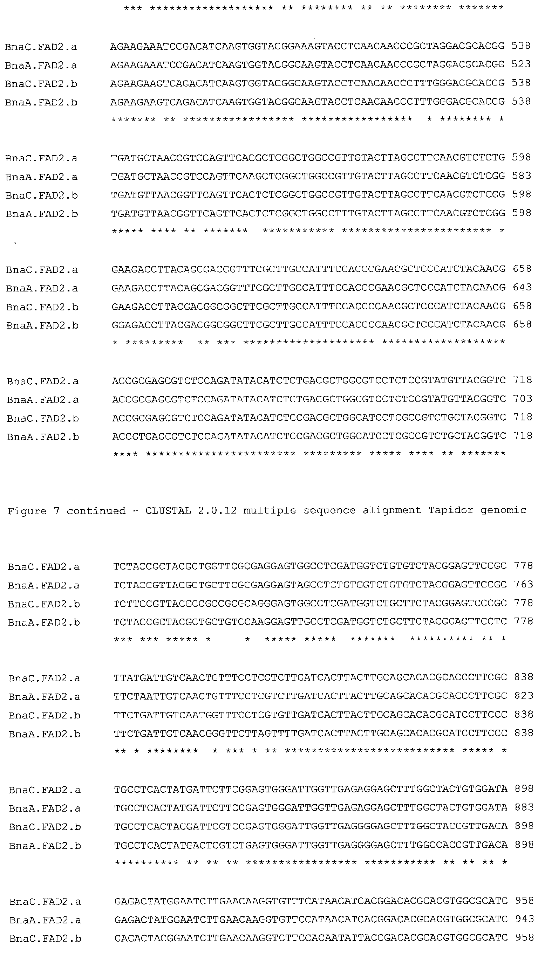

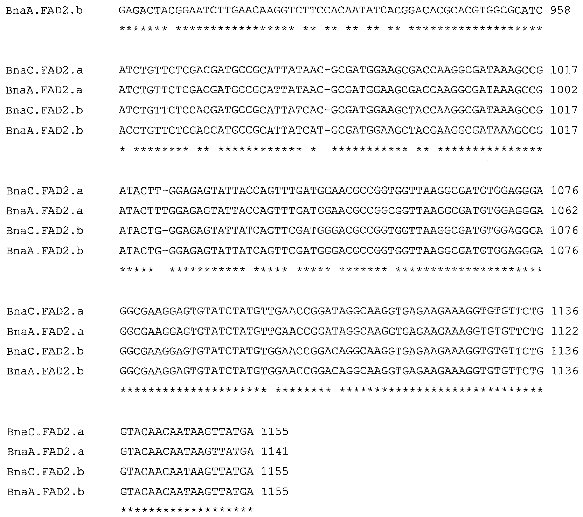

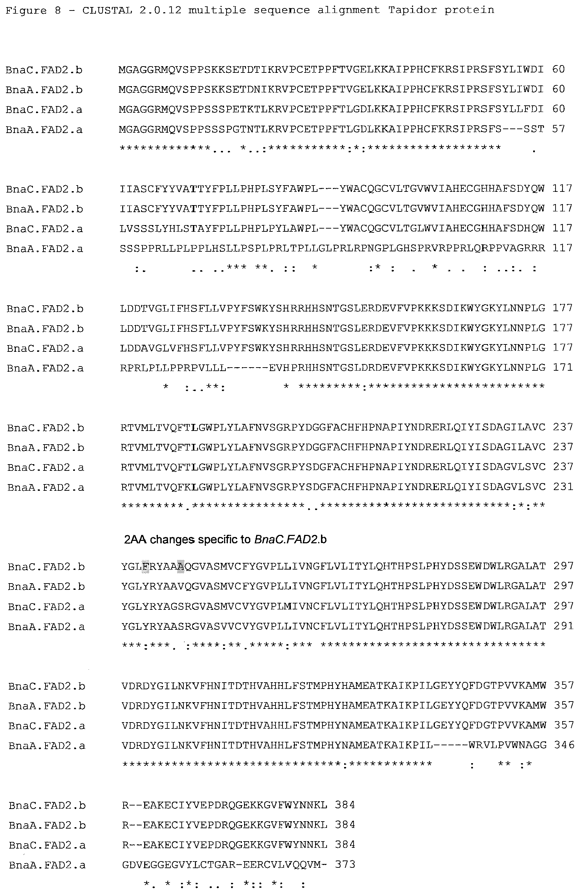

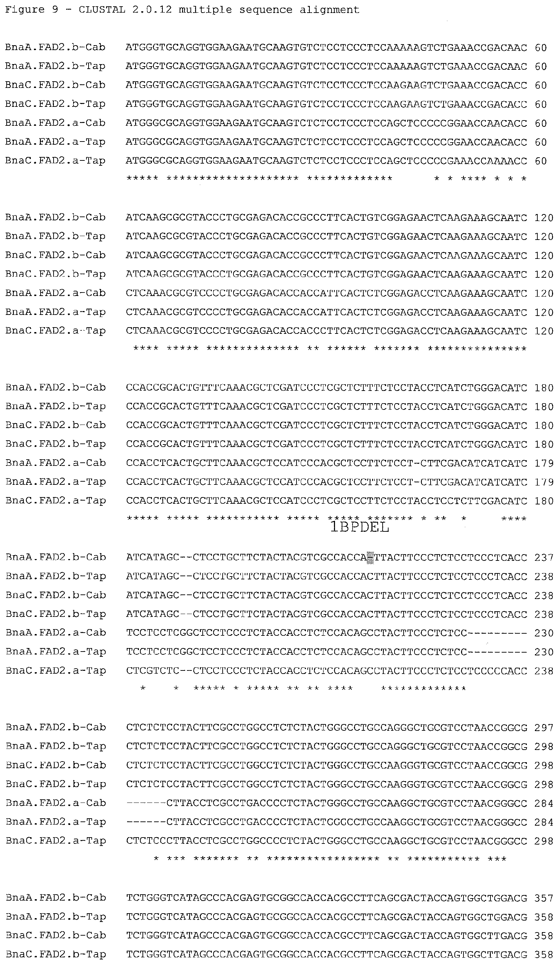

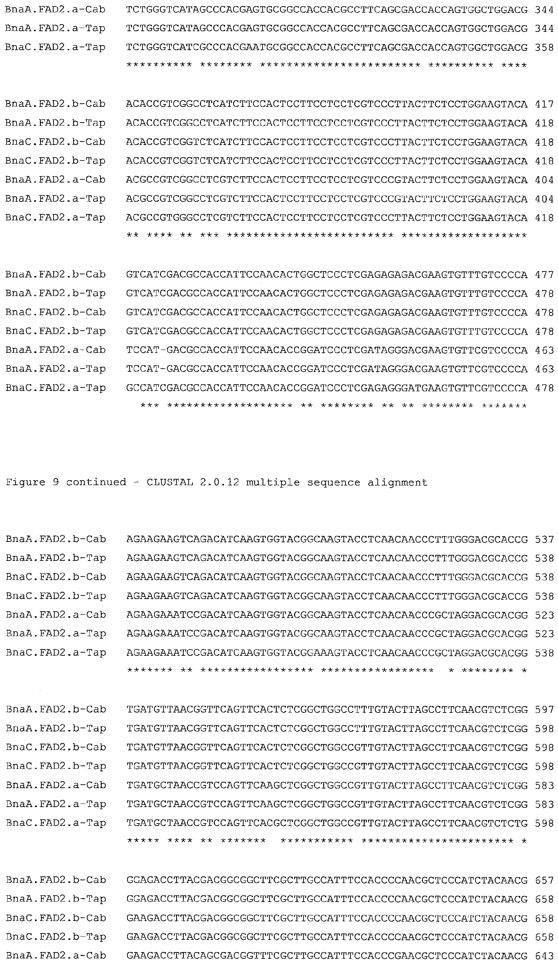

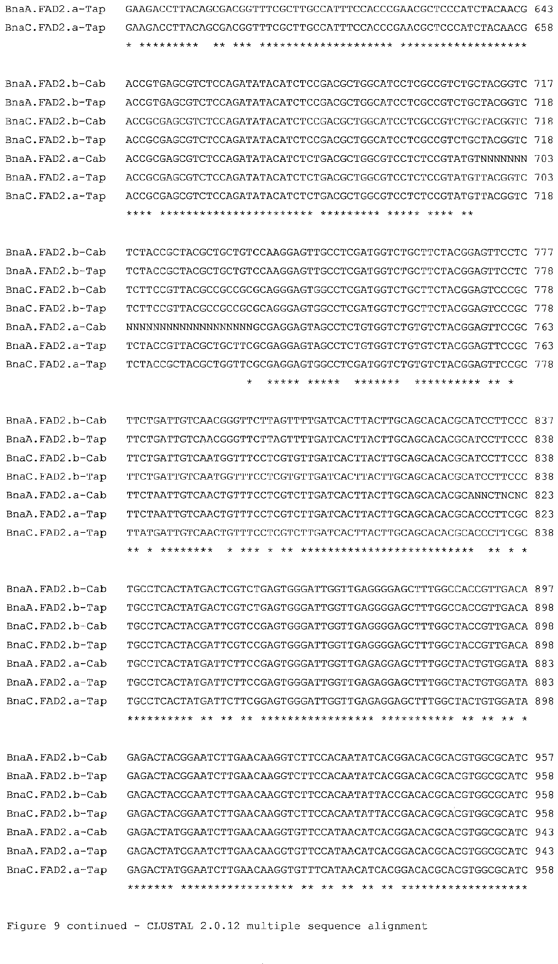

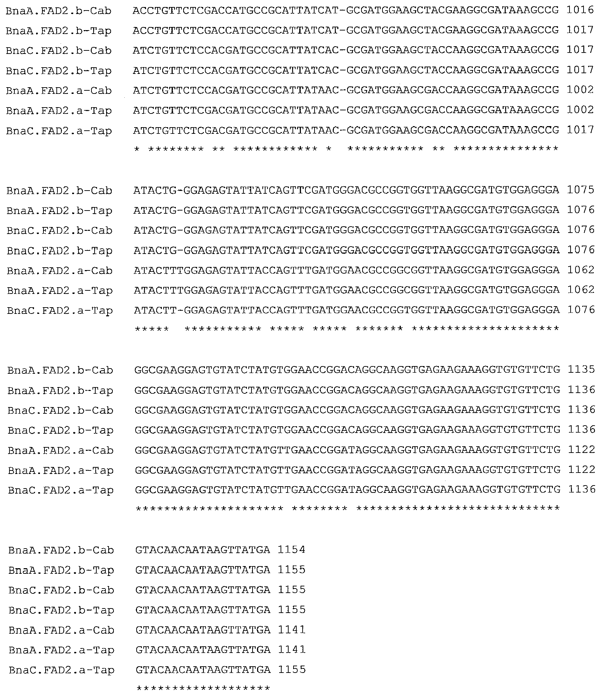

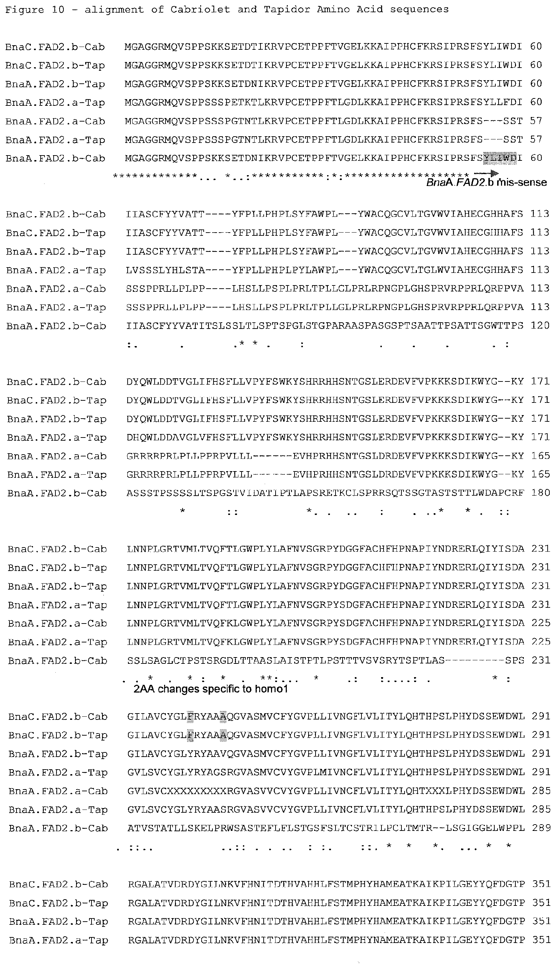

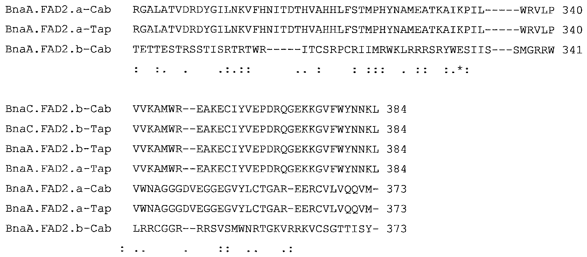

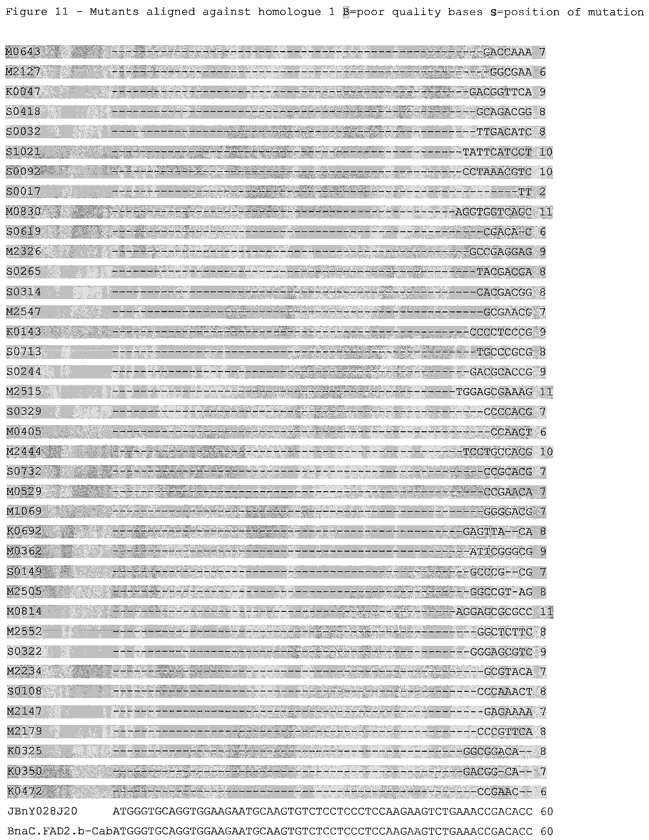

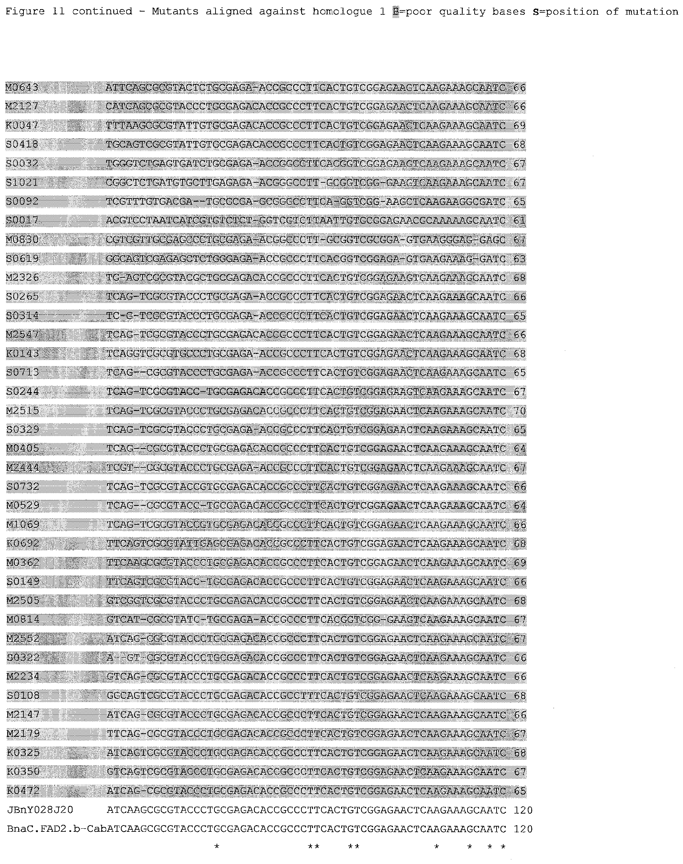

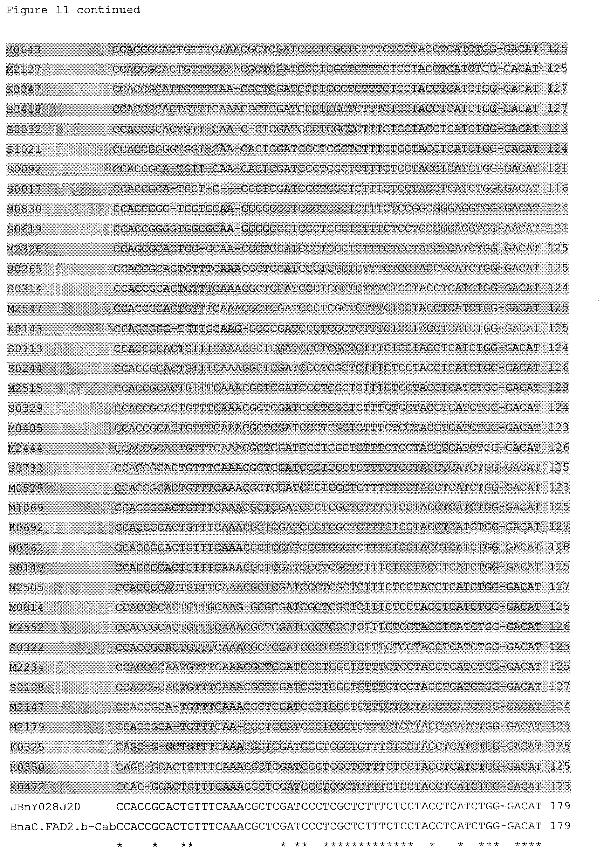

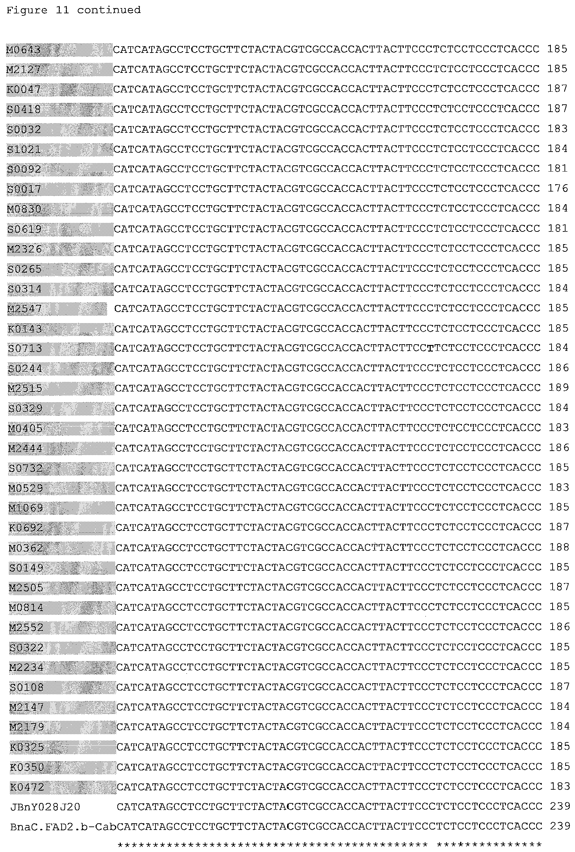

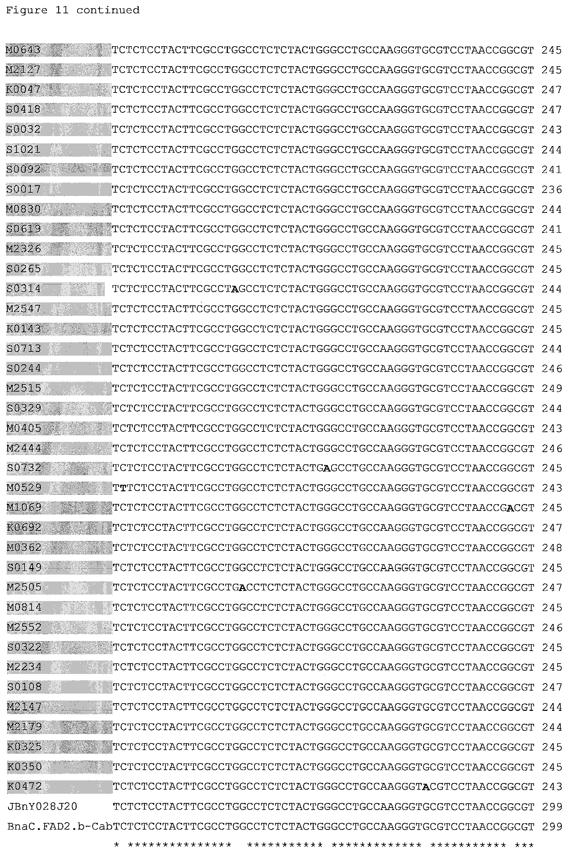

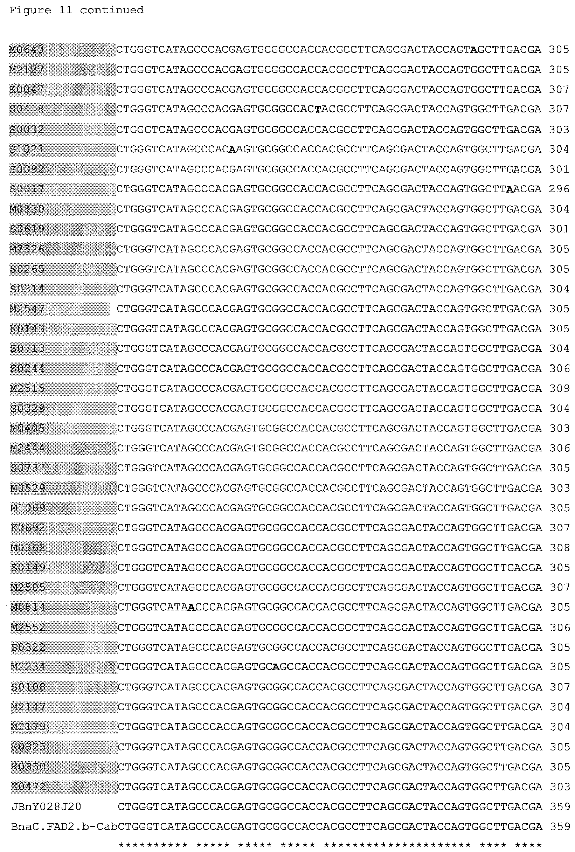

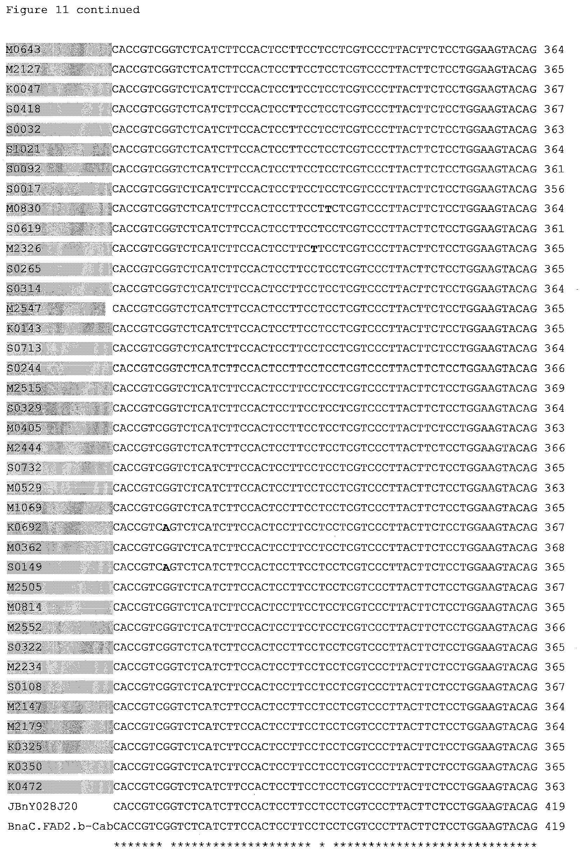

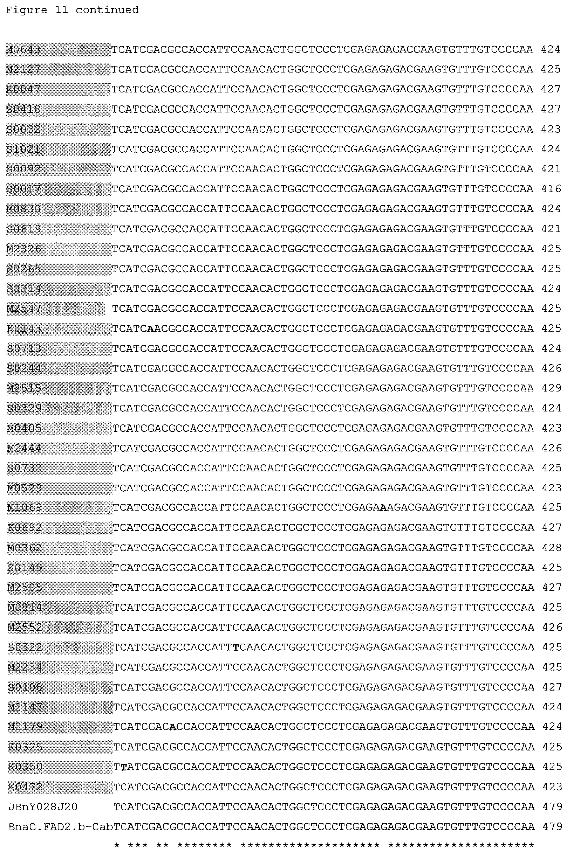

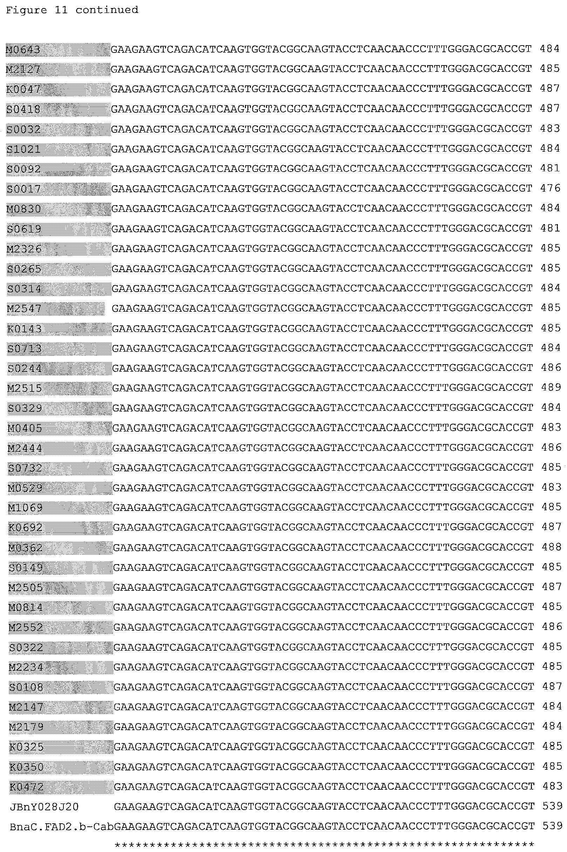

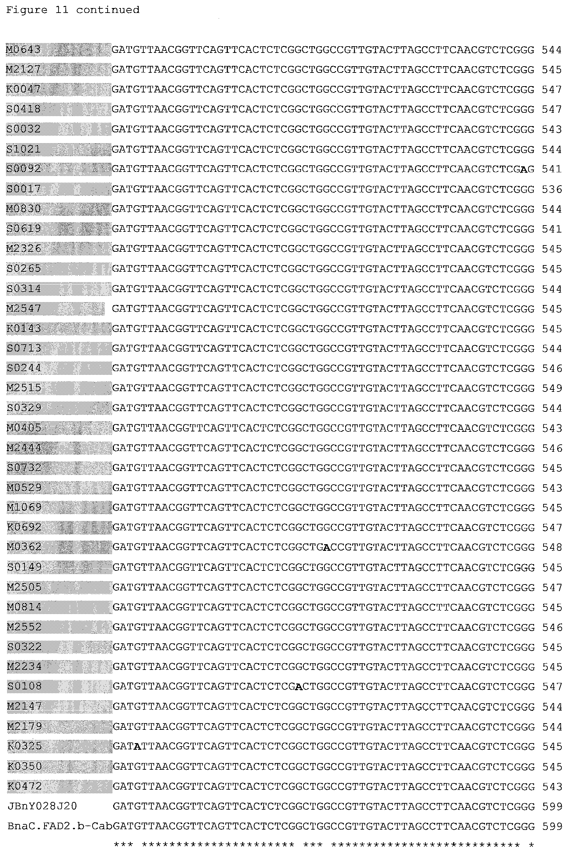

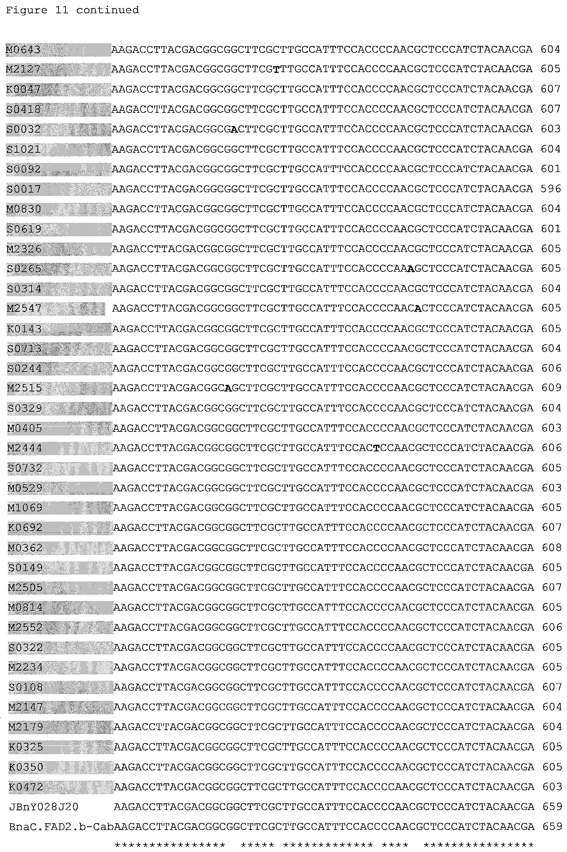

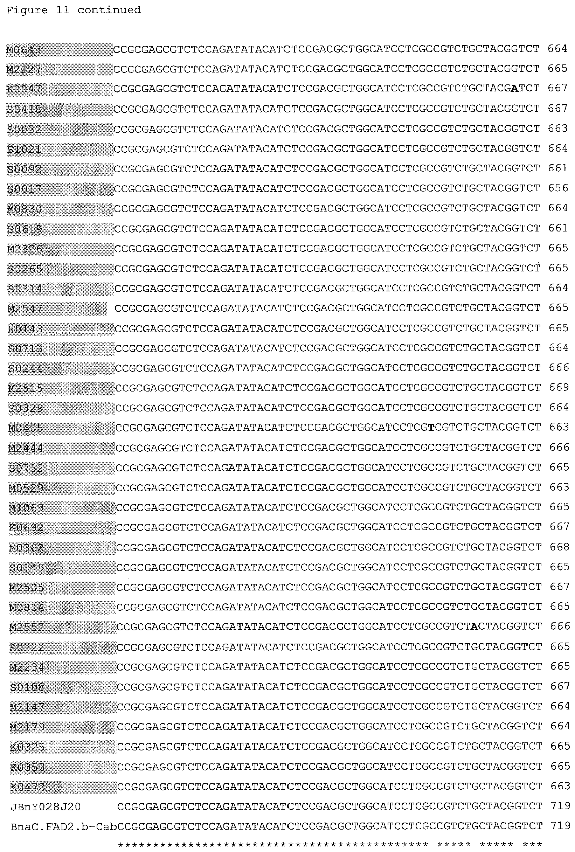

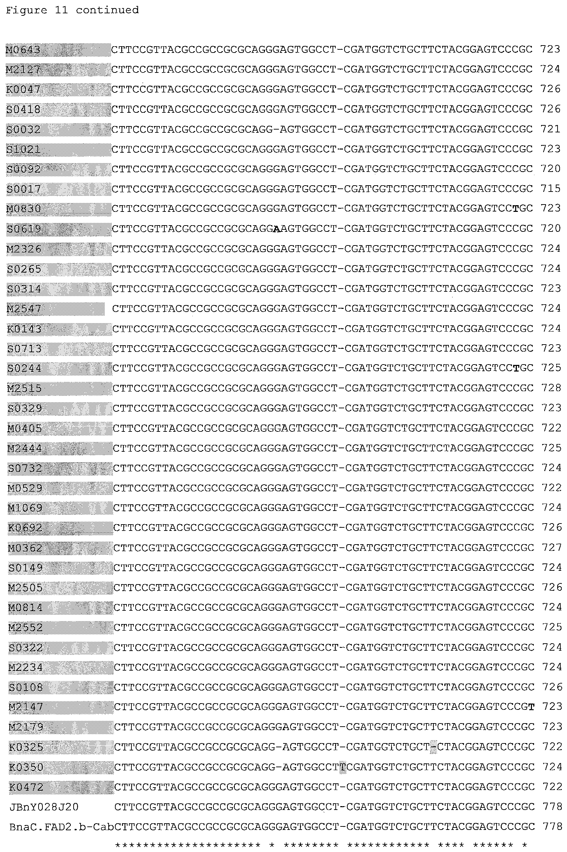

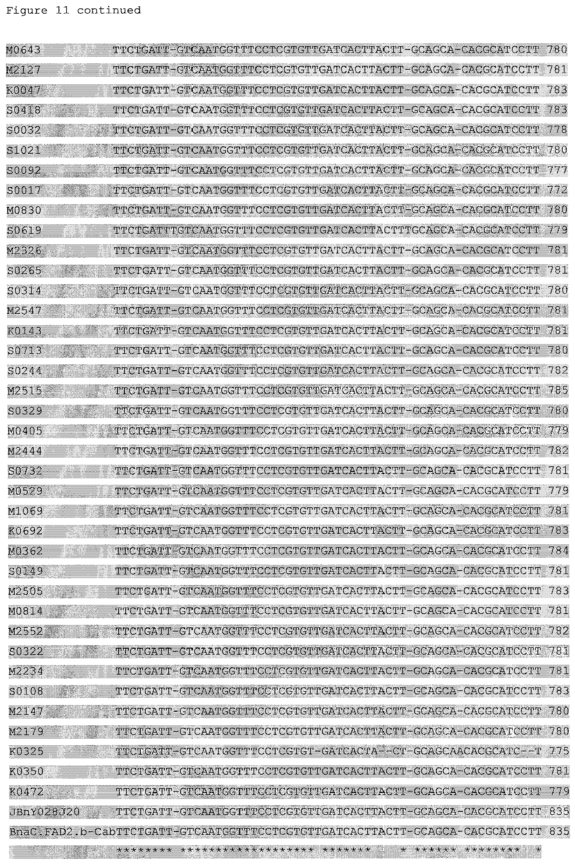

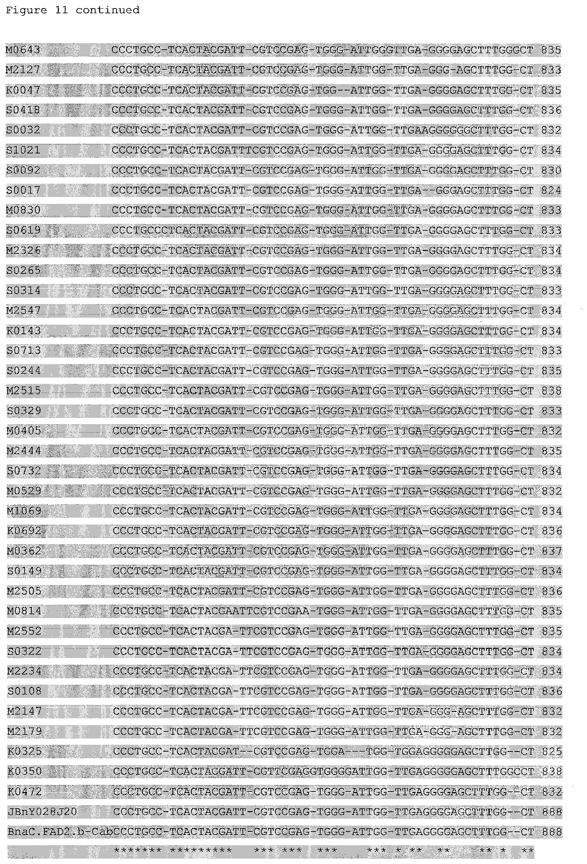

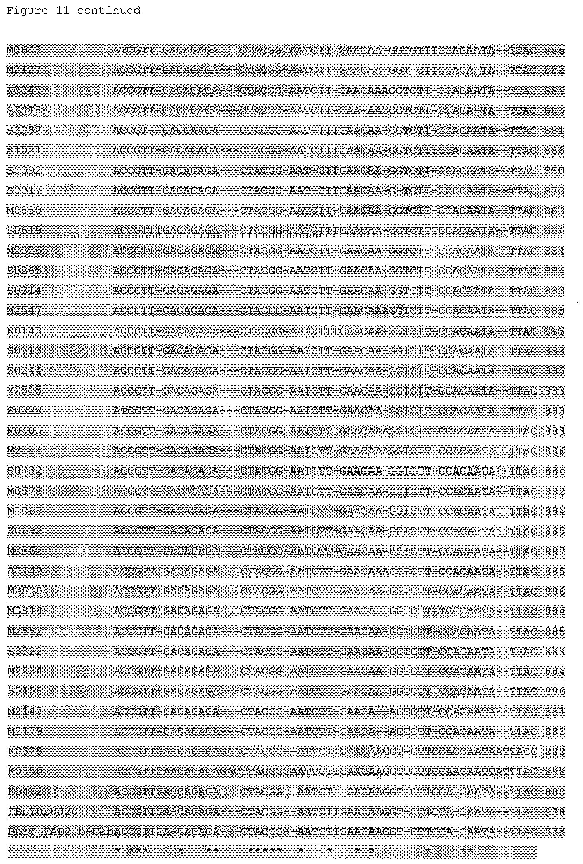

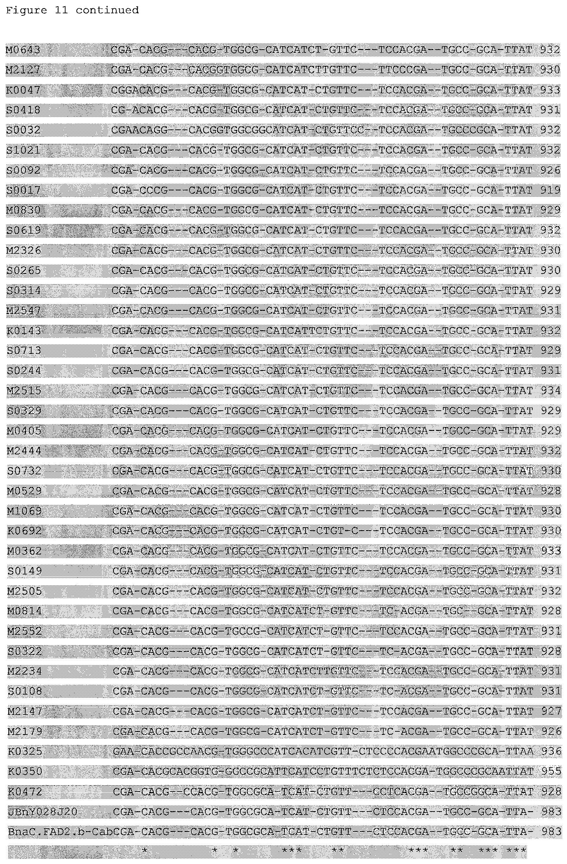

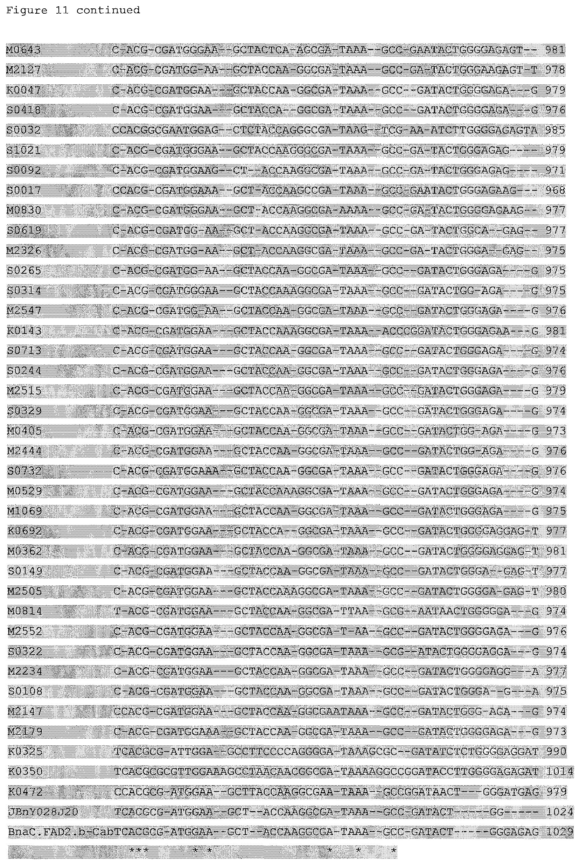

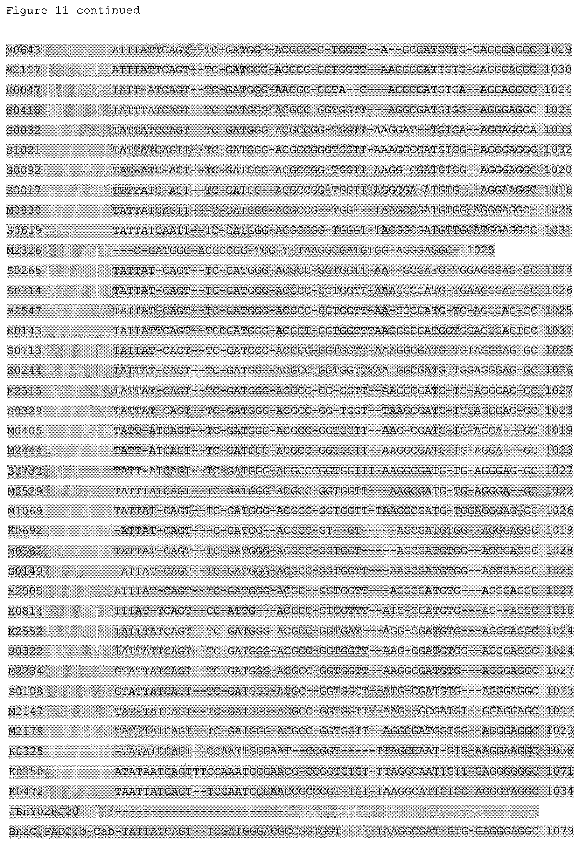

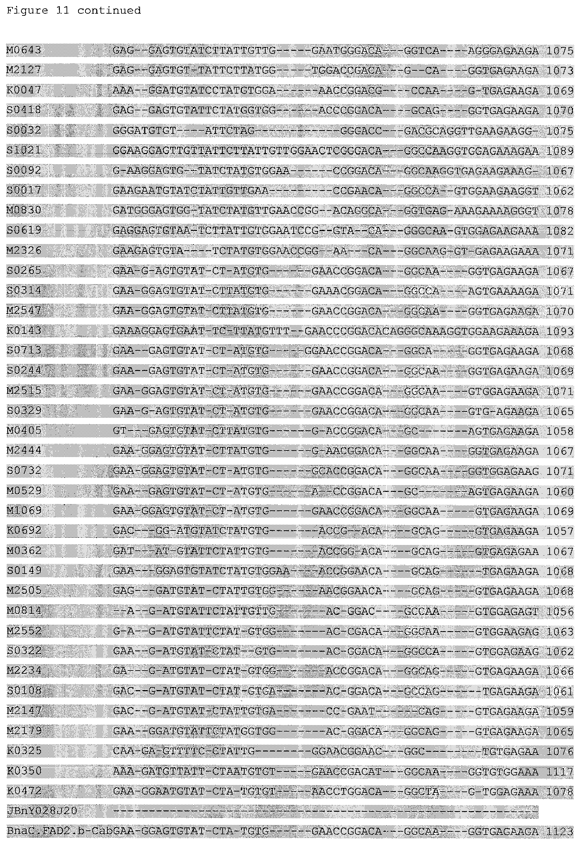

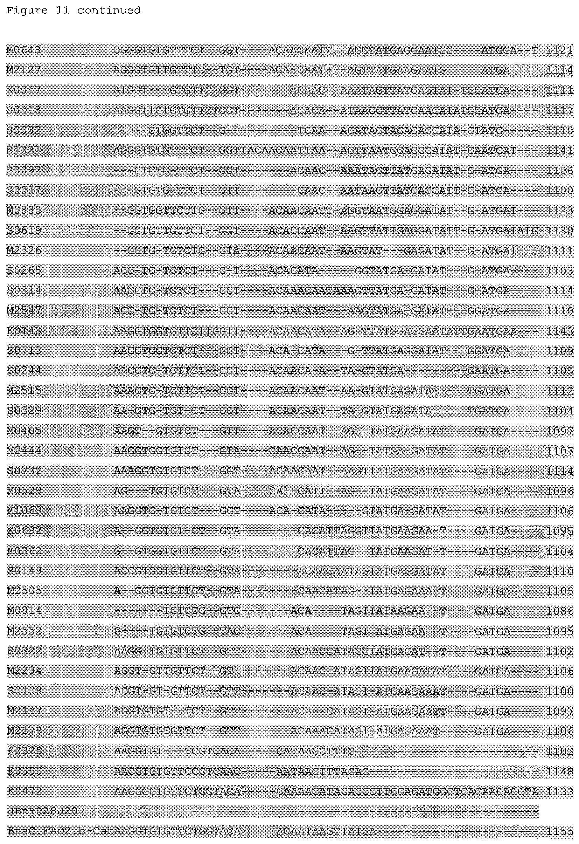

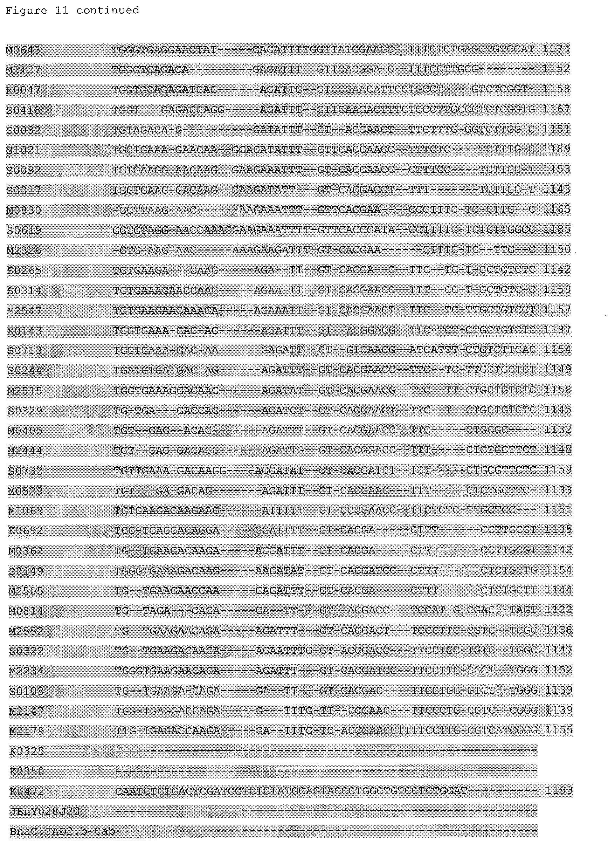

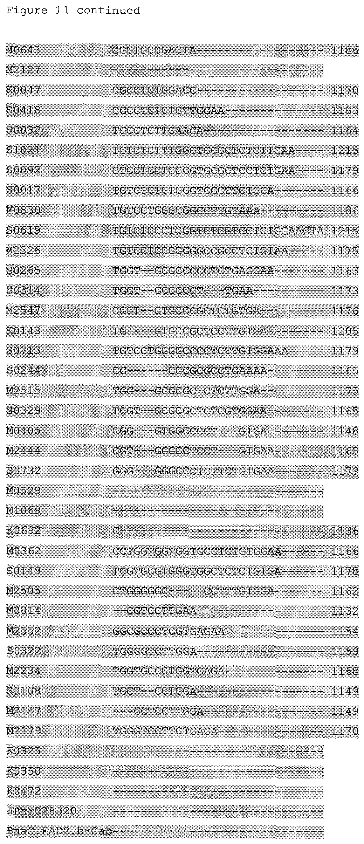

In the disclosure which follows, we demonstrate that B. *napus* has in its genome four homologues of *FAD2,* see SEQ ID. NOs. 1-4 for the nucleotide sequences of the four homologues found in *B. napus* variety Tapidor, (see Figure 7 for the nucleotide sequence alignments), with the encoded amino acid sequences provided as SEQ ID. NOs.5-8 (see Figure 8 for the amino acid sequence alignments). In addition, we demonstrate that *B. napus* variety Cabriolet has in its genome three homologues of *FAD2,* i.e. *BnaC.FAD2.a* has been deleted or replaced with *BnaA.FAD2.a* sequence, see SEQ ID. NOs. 9-11 for the nucleotide sequences of the three homologues found in B. *napus* variety Tapidor, (see Figure 9 for the nucleotide sequence alignments), with the encoded amino acid sequences provided as SEQ ID. NOs. 12-14 (see Figure 10 for the amino acid sequence alignments). Finally, we provide a wide variety of mutant sequences for the remaining active homologue of *FAD2* found in *B. napus* variety Cabriolet, see SEQ ID. NOs. 15-51, (see Figure 11 for nucleotide sequence alignments showing the various nucleotide changes found in the new varieties of *B. napus* produced herein).

Herein, we provide sequences for identifying the various homologues of delta-12 oleate desaturase enzyme, namely, *BnaA.FAD2.a, BnaC.FAD2.a, BnaA.FAD2.b,* and *BnaC.FAD2.b,* as well as methods and isolates in which all of the *FAD2* genes have been inactivated, mutated, deleted, truncated or otherwise modified, such that there is little or no production of delta-12 desaturase activity and maximal production of oleic acid.

Mutants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site. The flanking regions will contain convenient restriction sites corresponding to sites in the naturally-occurring sequence so that the sequence may be cut with the appropriate enzyme(s) and the synthetic DNA ligated into the cut. The DNA is then expressed to make the encoded FAD2. Inactivation of a FAD2-encoding nucleic acid sequence or inactivation of a FAD2 enzyme is then verified. This can be done for example by measuring the fatty acid content of a plant or part thereof compared to a control plant which has no mutation. If inactivation of a FAD2-encoding nucleic acid sequence or inactivation of a FAD2 enzyme has occurred, the level of FAD2 activity will be reduced. This can be measured by detecting an increase in 18:1 fatty acid content and/or a decrease in the PUFA (e.g. 18:2 and/or 18:3 fatty acid) content of the plant or part thereof. These methods are only illustrative of the numerous standard techniques known in the art for manipulation of DNA sequences and other known techniques may also be used.

Accordingly, in one embodiment according to the present invention, we disclose herein the increase in 18:1 (oleic acid) content from high oleic variety *B. napus* "Cabriolet" to at least 80% and decrease in polyunsaturated fatty acid content (PUFA) primarily made up for 18:2 and 18:3 to below 10%. In one embodiment, this is achieved via mutagenisis, for example by EMS (ethyl-methane sulfonate) mediated mutation of the B. *napus* genome and selection and selfing of viable plants. As noted above, the enzyme principally controlling the desaturation of 18:1 to 18:2 desaturated fatty acid is *FAD2,* and therefore, abolishing its function reduces and /or eliminates progression down the desaturation pathway.

### Existing B. napus Varieties:

### a. Phenotype:

We have characterized and compared the fatty acid profile of *B. napus* "Tapior" (an old European variety) and "Cabriolet" (a high oleic acid variety used in modern oleic acid production). The starting phenotype of these two varieties with respect to fatty acid production is summarized in Table 1 below:

**Table 1: Mean Percentages of each fatty acid**

| Tapidor | | | | | | | | | | | | | |
|---|---|---|---|---|---|---|---|---|---|---|---|---|---|
| 14:0% | 16:0% | 16:1% | 18: 0% | 18:1% | 18:2% | 18:3% | 20:0% | 20:1% | 20:2% | 22:0% | 22:1% | 24:0% | 24:1% |
| 0.08 | 4.97 | 0.32 | 1.85 | 60.4 | 21.7 | 7.71 | 0.66 | 1.08 | 0.06 | 0.34 | 0.02 | 0.20 | 0.13 |

| Cabriolet | | | | | | | | | | | | | |
|---|---|---|---|---|---|---|---|---|---|---|---|---|---|
| 14:0% | 16:0% | 16:1% | 18:0% | 18:1% | 18:2% | 18:3% | 20:0% | 20:1% | 20:2% | 22:0% | 22:1% | 24:0% | 24:1% |
| 0.07 | 4.37 | 0.35 | 1.42 | 74.5 | 8.91 | 7.21 | 0.56 | 1.38 | 0.05 | 0.31 | 0.03 | 0.21 | 0.14 |

As can be seen from Table 1, variety Cabriolet already shows an increase in 18:1 (74.5%) and decrease in 18:2 (8.2%) due to loss of function of FAD2 when compared to the old cultivar Tapidor (60.4% and 21.7%, respectively). Oleic acid percentages as high as 81-85%+ have been reported in the literature (see Rücker and Röbbelen, 1995 (81%); Wong et *al.* 1991 (>85%)). Herein, we have selected variants which exhibit improvements over the levels reported in Cabriolet where the improvement is 5% or better in 18:1, and reductions in 18:2 and/or 18:3 content is 5% or better.

### b. Genetics:

**Table 2: BAC clones used for sequencing FAD2 homologues**

| Homologue | BAC | Linkage group | Allele name | |
|---|---|---|---|---|
| | | | Tapidor | Cabriolet |
| | | | | |
| *Bna*C*.FAD2.*b | JBnY028J20 | C5 | -Tap | -Cab |
| *Bna*A*.FAD2.*b | JBnB069K15 | A5 | -Tap | -Cab |
| *Bna*C*.FAD2.*a | JBnY182P11 | C1 | -Tap | -Cab |
| *Bna*A*.FAD2.*a | JBnB090P19 | A1 | -Tap | -Cab |

The above table summarizes the BAC clones we utilized, and the relationship of the *FAD2* homologues we identified in two different *B. napus* germplasms, i.e. that of variety Tapidor and that of variety Cabriolet, see discussion and characterization of these varieties in detail below.

### i. Variety Tapidor

We have identified, by sequencing of BACs (see table above), cloned PCR product, allele specific PCR, RT-PCR and mRNA-Seq transcriptome sequencing, from the variety Tapidor, four homologues of *FAD2* in *B. napus* named homologues *BnaA.FAD2.a, BnaC.FAD2.a, BnaA.FAD2.b* and *BnaC.FAD2.b* and we have mapped the genes as noted above. We have found that *BnaA.FAD2.a,* on chromosome A1, is truncated in this variety, and is therefore non-functional. We have found that *BnaA.FAD2.b* and *BnaC.FAD2.a* on chromosomes A5 and C1, respectively, encode proteins with high sequence similarity as shown in Figure 7. These are believed to be functional in variety Tapidor. By contrast, Tapidor *BnaC.FAD2.b,* chromosome C5, contains two unique amino acid changes which are believed to affect protein function. These are amino acid no's 241 and 246. Accordingly, we have concluded that variety Tapidor contains two fully functional plus one compromised homologue of *FAD2.*

### ii. Variety Cabriolet

We have sequenced via cloned PCR product, allele specific PCR, RT-PCR and mRNA-Seq transcriptome sequencing, from variety Cabriolet, three homologues of *FAD2.* We have confirmed that *BnaA.FAD2.a* on chromosome A1, is truncated as in the variety Tapidor. We have confirmed that in variety Cabriolet, *BnaC.FAD2.a,* on chromosome C1, is absent or is, possibly, replaced by homeologous recombination a second copy of *BnaA.FAD2.a.* We believe this is the first time this has been reported for variety Cabriolet. We report herein that *BnaA.FAD2.b,* chromosome A5, contains a 1bp deletion at coding base position 215, which leads to a loss of protein function from enzyme encoded by this locus. Finally, we have confirmed that *BnaC.FAD2.b,* chromosome C5, encodes a functional but compromised FAD2 enzyme, as in variety Tapidor.

Accordingly, we concluded, based on the genetic analysis, that in variety Cabriolet, while homologues *BnaA.FAD2.a, BnaA.FAD2.b* and *BnaC.FAD2.a* are non-functional, *BnaC.FAD2.b* remains, encoding a compromised functional delta-12 oleate desaturase, and reduces the oleic acid fraction recoverable from this variety. We therefore set about developing methods for producing and screening *B*. *napus* in which *BnaC.FAD2.b* on chromosome C5 is altered in such a way that the enzyme function is further compromised or lost.

We characterized, by sequencing, wild type *FAD2* sequences and mutated sequences, produced and characterized as described in detail in the examples which follow. Mutants identified by sequencing of *BnaC.FAD2.b* PCR product.

In Figure 6, we provide a table of mutants identified, showing SNP position/changes, amino acid position/changes and phenotype with respect to fatty acid profile and fraction thereof that is oleic acid and oil profile of given mutants following 1^{st} years trials.

As noted above in the background to this invention, the following patent disclosures provide a wide and diverse series of patent applicants, relating to various efforts to achieve increased oleic acid content in a variety of different plants: US 5,840,946 (Pioneer, "Vegetable Oil Extracted From Rapeseeds Having a Genetically Controlled Unusually High Oleic Acid Content"); WO2004/072259 (Dow Agrosciences, "Altered Fad2 and Fad3 Genes in Brassica and the Molecular Marker-Assisted Detection Thereof"); WO2007/107590 (Monsanto, "FAD-2 Mutants and High Oleic Plants"); WO2007/138444 (INRA, "Genetic Markers for High Oleic Acid Content in Plants"); US 7,423,198 and US 7,605,301 (Viterra, "High Oleic Acid *Brassica juncea"* and "Plant Fad2 Coding Sequence Balancing Fatty Acid Profiling in Edible Oils", respectively). All of these published patent disclosures are herein incorporated by reference for the purpose of enabling those skilled in the art to utilize in combination the information provided herein with respect to various mutations in Cabriolet *BnaC.FAD2.b* with mutations or silencing constructs directed to all or the other of the *FAD2* homologues.

It should be noted, that there are several homologues of *FAD2* (four in oilseed rape) and all must be knocked out in order to get the optimum high oleic, low PUFA phenotype. We have been the first to demonstrate conclusively the presence of the four FAD2 homologues. Mutating any one homologue in oilseed rape would has little or no impact the oil profile; the optimum effect on the oil profile was achieved by us as disclosed herein through the introduction of mutations into or deletion of all four homologues. In that respect, the molecular markers for the three homologues already defective in cultivar Cabriolet are useful, in combination with the sequences provided herein for Cabriolet *BnaC.FAD2.b.* Breeders utilising the present invention will be interested in the lines disclosed herein with specific mutations that abolish the function of this last homologue of the gene. While Monsanto's Cabriolet high oleic variety only has one functional yet compromised *FAD2* of the four genes (as compared to the old European variety Tapidor (which has, apparently, three of four functional *FAD2* genes, with one of these being compromised), an important contribution to the art made by the present invention disclosure is that those skilled in the art, based on this disclosure, are able to identify *FAD2* mutations in the remaining functional *FAD2* gene in Cabriolet.

In addition this disclosure also enables those skilled in the art to localise mutations in all four homologues of the *FAD2* genes using the information disclosed herein.

It is further important to note that the variability of oleic acid profile with the different mutations reported herein is related to the severity of the effect of the mutation on the protein itself. Some mutations produce changes that have a greater effect on the proteins' conformation, possibly affecting the enzyme's active site or how it sits in the membrane. Mutations other than a STOP codon can have a very drastic effect. Obviously a STOP mutant should completely abolish function. While report herein the identification of STOP codon insertions into *FAD2, BnaC.FAD2.b,* only one line was tested as a homozygous STOP M0643, oleic 83.9%, PUFA 5.96%.

Finally, it is noted that *FAD2* appears not to have any additive effect, meaning that there is enough transcript produced in the heterozygous state of one functional gene to produce the amount of protein (enzyme in this case) needed to show the full wild-type phenotype. This is why a significantly reduced low PUFA phenotype, which had been keenly sought for many years, had not been achieved by incremental reduction (i.e. finding in germplasm collections knocked-out alleles of the genes one at a time based on phenotypic screens). Only the combination of three defective genes and the last functional homologue being itself somewhat impaired in enzyme function gave the phenotype of Cabriolet and other low PUFA lines currently in commercial use. Most of the *FAD2 BnaC.FAD2.b* mutants reported herein have specific changes in amino acids, the most interesting ones abolish enzyme function altogether (probably by affecting the enzyme active site). Interestingly (from the scientific point of view, at least) some mutants have increased enzyme activity, increasing the PUFA content relative to Cabriolet. It is not believed that the phenotypic impacts of these amino acid changes could have been predicted. Thus, it would be wrong to say that one could predict the effect of any given mutation, as some of them can even increase the PUFA fraction, while others reduce the PUFAs and increase the Oleic acid fraction. Figure 15 shows a selection of mutants from the population where amino acid changes increase the PUFA fraction produced. The change described in mutant M2210 reverts one of the amino acids believed to reduce FAD2 function in *BnaC.FAD2.b* to the amino acid observed in *BnaA.FAD2.b* and results in reduced oleic acid content from increased FAD2 functionality in this mutant.

### EXAMPLES

While the foregoing disclosure generally describes this invention, the following examples are provided to further describe and enable this invention. It will be appreciated, however, that these examples and the specifics provided therein are non-limiting and those skilled in the art could vary or use equivalent methods, apparatuses and systems, without departing from the heart of the invention.

### EXAMPLE 1

### IDENTIFICATION AND CHARACTERIZATION OF FAD2 HOMOLOGUES IN BRASSICA NAPUS

### i) Selection of BACs as templates for the amplification of gene-specific probes

Basic local alignment search tool (BLAST) alignment of end sequence data from *Arabidopsis* BAC clones, developed in the *Brassica* Investigating Gene Function (IGF) project (http://brassica.bbsrc.ac.uk/IGF/), have been used to calculate the 'likely' alignment of the BAC on the Arabidopsis pseudo-chromosome. Positions have also been inferred from the Munich Information Centre for Protein Sequences (MIPS) annotation of the pseudo-chromosome. Using this data it is possible to calculate which gene models the BAC clones are expected to contain.

This information, available at http://brassica.bbsrc.ac.uk/IGF/?page=body/query_clone.ht m, was used to identify *Arabidopsis* BACs containing *FAD2.* Clones predicted to contain the gene of interest were grown up overnight in 5ml LB (lOg bacto-tryptone, 5g yeast extract, 10g NaCl made up to 1 litre with ddH₂O, pH 7.5) containing 10mg/ml of kanamycin antibiotic in a 37°C shaking incubator.

### ii) Small scale BAC DNA preparation

Cells were pelleted from 2ml of overnight BAC culture by spinning for 10min at 13000rpm in a micro-centrifuge and pouring off the supernatant. DNA was prepared using the QIAprep Spin miniprep kit (Qiagen Ltd., 2005) following the manufacturers instructions. DNA was eluted into 50µl of sterile ddH₂O.

### iii) Production of candidate gene probes

Primers SEQ ID. 62: GGGTGCAGGTGGAAGAATG probe f, SEQ ID. 63: TTGTTGTACCAGTACACACC probe r,for probe production were designed from the Arabidopsis *FAD2* gene sequence.

To produce the probe, touchdown PCR was carried out in 0.2ml tubes with a reaction volume of 100µl containing:

| | |
|---|---|
| 67.6µl | ddH₂O |
| 10µl | 10 x PCR buffer |
| 3µl | MgCl₂ (50mM) |
| 10µl | dNTPs (2mM) |
| 2µl | forward primer (10mM) |
| 2µl | reverse primer (10mM) |
| 0.4µl | Platinum Taq (5u/µl - Invitrogen) |
| 5µl | BAC DNA (1/5 dilution of original DNA preparation, ∼100ng) |

PCR was carried out on MWGAG Biotech Primus96 plus thermo-cycler on a touchdown cycle as detailed below with the initial annealing temperature 1°C higher than greatest primer Tm.

| 'Touchdown' cycle | | |
|---|---|---|
| Hot start | 94°C for 5 min | |
| | | |
| 15 cycles | | |
| Denaturation | 94°C for 30s | |
| Annealing | 63°C for 30s | (-1°C/cycle) |
| Extension | 72°C for 30s | |
| 30 cycles | | |
| Denaturation | 94°C for 30s | |
| Annealing | 53°C for 30s | |
| Extension | 72°C for 30s | |
| | | |
| Final Extension | 72°C for 7min | |
| End hold temperature | 8°C forever | |

5µl of the PCR product was checked on 1.5% agarose gel for amplification. The probe was purified to remove unincorporated primer and dNTPs using the QiaQuick PCR purification kit (Qiagen Ltd., 2002) with the DNA eluted into 30µl of H₂O.

### iv) BAC library hybridisation with candidate gene probes

Initial screens for BACs containing gene homologues were performed using the *B. napus* JBnY library developed from the variety 'Tapidor'. The JBnY library is a TAC library consisting of 73,728 clones with an average insert size of 85kb constructed using pYLTAC7 as vector. A further screen was performed on a second 'Tapidor' library, JBnB. The JBnB library is a large-insert binary vector library consisting of 73,728 clones with an average insert size of 145kb constructed using pBAC/SACB1 as vector.

Colony hybridisation was performed as described by O'Neill and Bancroft (2000) Comparative physical mapping of segments of the genome of Brassica oleracea var. albogalabra that are homeologous to sequenced regions of chromosome 4 and5 of Arabidopsis thaliana. Plant J 23:233-243.

Positive BACs were selected for confirmation by Southern hybridization.

### v) 96-well BAC DNA preparation

BAC DNA preparation was performed using a method based on Sambrook, et al, 1989 Molecular cloning: a laboratory manual. Cold Spring Harbor Press, Cold Spring Harbor. BACs were picked using sterile toothpicks into Beckman 96 deep-well plates containing 500µl of 2 × YT medium (16g tryptone, 10g natural yeast extract, 5g NaCl made up to 1 litre with ddH₂O) and a selective antibiotic (for JBnB 25µg/ml chloramphenicol and JBnY 25µg/ml kanamycin). Plates were incubated overnight at 37°C in a shaking incubator.

250µl of the sample was transferred into a Greiner plate and spun in a Qiagen plate centrifuge for 4min at 2800rpm to pellet the cells. The supernatant was then discarded by gently inverting the plate onto blue roll.

Cells were re-suspended in 25µl of re-suspension solution (GTE: 50mM Glucose, 25mM Tris-Cl, 10mM EDTA, pH 8.0) by tapping the plate and left for 5min. Cells were then lysed by adding 25µl of lysis solution (0.2 M NaOH, 1% SDS), the samples mixed until clear and again left for 5min. Twenty five µl of neutralisation solution (3M potassium acetate, pH 5.5) was added and mixed until a glutinous mixture was formed, with the plate then being left to stand for a further 5min.

The supernatant was spun through a non-sterile Multiscreen GV filter plate at 2800rpm for 3min into a clean plate containing 100µl of propan-2-ol and left to precipitate at room temperature for 20min. The plate was then spun at 2800rpm for 20min to condense the DNA pellets.

The pellets were washed by re-suspending in 110µl of 70% EtOH, tapping to mix, and again condensed by spinning at 2800rpm for 10min. This final wash was repeated and pellets were then dried for 1h before digestion.

### vi) Fingerprinting digest and gel imaging

Pellets from the BAC DNA preparation were re-dissolved in 15µl of enzyme mix (5.52ml ddH₂O, 2µl Rnase (Rnase One - Promega M4261), 660µl Buffer B (supplied with *Hind* III), 220µl concentrated *Hind* III (*Hind* III HC - Roche 1274040)) and digested at 37°C for 2h. Two µl of 6 x loading dye (15% Ficoll, 0.06% Bromophenol blue, 0.06% Xylene cyanol, 10mM EDTA) was then added and plates spun uncovered at 2800rpm for 20min to reduce the volume to 10µl.

1.8µl of digested sample was loaded on to a 121 lane 1% TAE agarose (SeaKem LE, FMC Bioproducts, Rockland, ME, US) gel on the laboratory bench in a wide format system model A3-1 (Owl Scientific, US) gel rig containing 1% TAE. Point eight µl of a mixture of wide range analytical DNA ladder (Promega, catalogue no. DG1931) and molecular weight marker V ladder (Roche, catalogue number 0821705) was loaded every 5^{th} lane starting from the 1^{st} lane. The samples were run at 100V for 10min before being transferred to the cold room and then run overnight at 55V for 18h.

Gels were post-stained, shaking for 2h, in 250ml of 'staining fluid' (20mM Tris and 0.1mM EDTA) containing 25µl of Vistra Green (Amersham Life Sciences, UK). Fingerprints were visualised on the Molecular Dynamics FluorImager 595 before being used for Southern Blotting.

### vii) Southern blot and hybridisation

Southern hybridisation was conducted on positively hybridising clones as described by Rana et al.(2004) (Rana D, Boogaart T, O'Neill CM, Hynes L, Bent E, Macpherson L,Park JY, Lim YP, Bancroft I (2004) Conservation of the microstructure of genome segments in Brassica napus and its diploid relatives. Plant J 40:725-733.)

### viii) Identification of homologue number

Positively hybridising clones were plated onto LB media containing the required selective antibiotic and grown up overnight. Colony PCR was performed using both conserved and degenerate FAD2 primers (SEQ ID. 64: conserved f GAGGGAGGCGAAGGAGTGTATC, SEQ ID. 65: conserved r CAGGAGAAGTAAGGGACGAGG, SEQ ID. 66: degenerate f ATTCCTTCCTNCTNCTNGTNCC, SEQ ID. 67: degenerate r GCTAAGTACAANGGNCANCC) on the touchdown cycle described in iii.

### Colony PCR reaction:

| | |
|---|---|
| 12.5µl | ddH₂O |
| 2µl | 10 x PCR buffer |
| 1.3µl | dNTPs (2mM) |
| 2µl | forward primer (10mM) |
| 2µl | reverse primer (10mM) |
| 0.4µl | Amplitaq gold (5u/µl - Invitrogen) |

PCR product was cleaned up sequenced as described below and sequences aligned to determine homologue number.

### PCR clean up:

10µl of sample treated with SAPEXO, 1µl shrimp alkaline phosphotase (SAP) (Roche - Cat. No. 04898133001) and 0.5µl exonuclease 1 (EXO) (GE Healthcare - Cat. No. E700732). Samples incubated at 37°C for 30min before denaturing at 80°C for 10min. Samples were then submitted to the sequencing provider.

### Sequencing reaction:

Ten microlitre sequencing reactions performed in 0.2ml tubes using an adjusted protocol from the BigDye v3.1 terminator cycle sequencing kit (Applied Biosystems, 2002) .

### Reactions contained:

| | |
|---|---|
| 1µl | PCR product (2-3ng/pl per 100bp) |
| 1ul | BigDye v3.1 |
| 1.5µl | 5 x sequencing reaction buffer |
| 1µl | primer (2µM) |
| 5.5µl | ddH₂O |

### Sequencing cycle:

| | |
|---|---|
| Denaturation | 96°C for 1min |
| | |
| Cycles × 25 | |
| | |
| Denaturation | 96°C for 10s |
| Annealing | 50°C for 5s |
| Extension | 40°C for 4min |
| Cool | 4°C for 10min |
| | |
| End-hold temperature | 10°C forever |

One BAC from each identified homologue group was fully sequencing by a service provider.

### Confirmation of homologue number in B. napus

Primers had previously been developed for the generic amplification of *FAD2* as described above. A further nonspecific primer was developed to extend this product to the stop codon.

Cloning of generic PCR product to confirm all homologues of *FAD2* had been identified in the BAC library was performed using the pGEM-T cloning kit (Promega A3600). Sequencing of clones revealed no further homologues.

### Expression of homologues in B. napus

RNA from leaves and developing seeds of the varieties Cabriolet and Tapidor was extracted from developing seed 45 days after flower opening using the Qiagen RNeasy plant minikit (Qiagen 72904). Alternative buffer RLC was used for RNA extraction from seed due to the oil and starch within the starting material. Cloning of generic *FAD2* RT-PCR product produced using the Superscript III first strand synthesis system kit (Invitrogen 18080-051) showed all homologues of *FAD2* expressed in Tapidor seed. Expression of *BnaC.FAD2.a* was not observed in Cabriolet seed. Full transcriptome sequencing using single ended Illumina mRNA-Seq system (Illumina RS-100-0801) confirmed these results. Low levels of expression of the homologues seen to be expressed in the seed were observed in leaf tissue samples.

### BnaC.FAD2.a absence in Cabriolet

To determine the absence of the homologue in Cabriolet PCR was performed for both the upstream and downstream genes. PCR for Tapidor was successful for all primer combinations. Lack of amplification in Cabriolet suggests a region of at least 25kb around *FAD2* where sequence expected from the sequence of clone JBnY182P11 is absent further supporting the absence of this homologue in Cabriolet.

### Mapping of FAD2 homologues

Development of specific homologue linked markers and mapping of the homologues is described in Smooker A. M., Wells R., Morgan C., Beaudoin F., Cho K., Fraser F., Bancroft I. (2010) The identification and mapping of candidate genes and QTL involved in the fatty acid desaturation pathway in Brassica napus. Theoretical and Applied Genetics http://www.ncbi.nlm.nih.gov/pubmed/21184048. Homologue *BnaA.FAD2.a* was assigned to linkage group A1 from homology to a mapped fully sequenced *B. rapa* BAC. Alignment of *FAD2* sequences identified the four separate homologues could be divided into two separate homeologue groups (A1, C1 and A5, C5). The 6bp difference at 41bp from the start of the coding region as shown in figure 7 was utilized to produce selective primers for these two groups of homeologues. Homologue specific primers were then designed around unique SNPs specific to the individual homologues.

Primers used for conducting PCR for the selective amplification of the different FAD2 homologues are shown in Table 3 below.

**Table 3**

| No. | Primer | Sequence | Bp | GC % | Tm |
|---|---|---|---|---|---|
| 1 | Sel 1+2 f | GTCTCCTCCCTCCAAAAAGT | 20 | 50.0 | 54.9 |
| 2 | Sel 1+2 new | GTGTCTCCTCCCTCCAAA | 18 | 55.6 | 51.9 |
| 3 | FAD2 3+4 start | CTACAGAAACAAACATGGGC | 20 | 45.0 | 53.1 |
| 4 | Sel 3+4 new | GTCTCTCCTCCCTCCAGC | 18 | 66.7 | 53.4 |
| 5 | Sel 3+4 f | CTCTCCTCCCTCCAGCTCCC | 20 | 70.0 | 62.4 |
| 6 | FAD2 H3 f | CTCTTCGACATCCTCGTCTC | 20 | 55.0 | 53.3 |
| 7 | Cons f 1698 | CCTCGTCCCTTACTTCTCCTG | 21 | 57.1 | 58.2 |
| 8 | FAD2 stop | CCTCATAACTTATTGTTGTACCAG | 24 | 37.5 | 53.9 |
| 9 | FAD2grp13'UTR-r2 | CAAGACGACCAGAGACAGC | 20 | 55.3 | 55.0 |
| 10 | FAD2grp4 3'UTR2 | GAACTCGACAAATTTGCCTG | 20 | 55.7 | 45.0 |

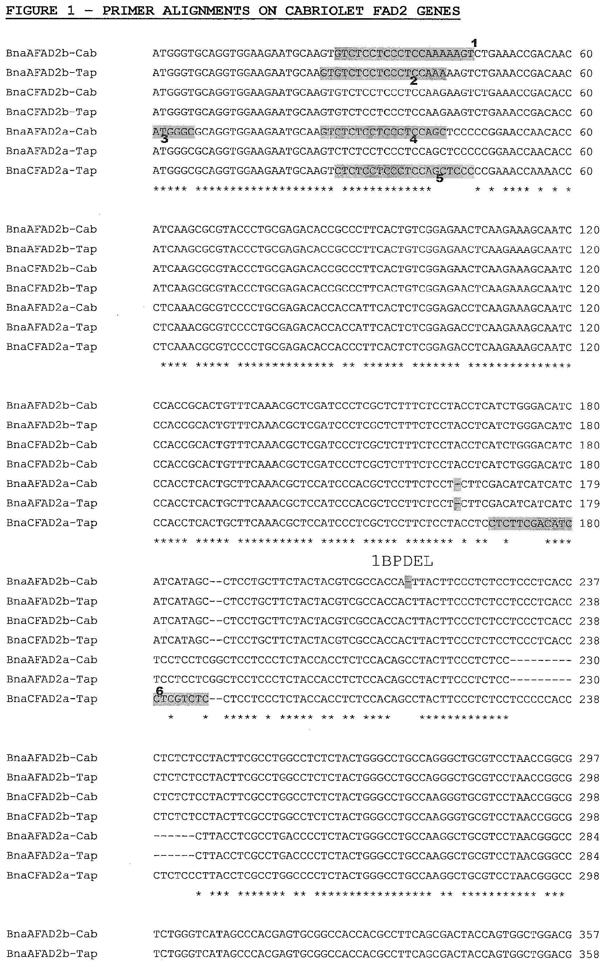

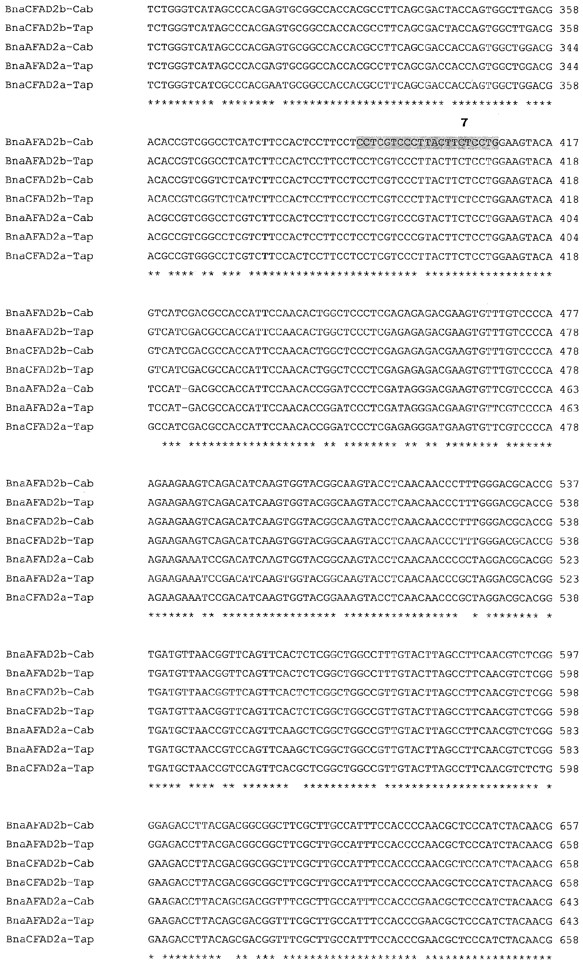

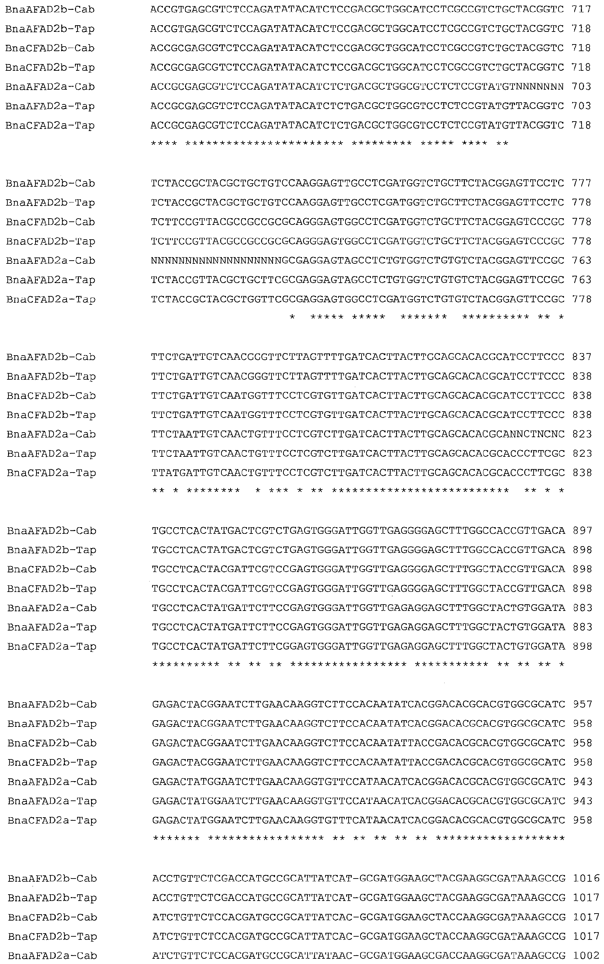

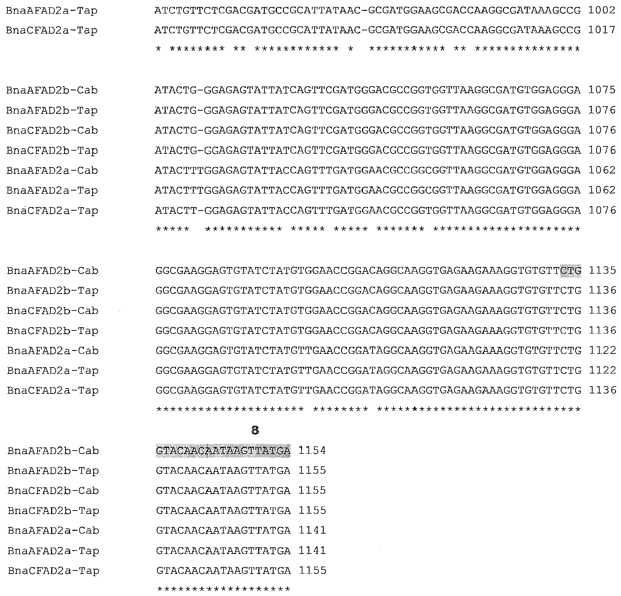

The position of primers on each gene is shown in Figure 1 (UTR primers not included). Primer combinations to amplify individual homologues are detailed in Table 4 below:

**Table 4:**

| Homologue | F primer | R primer | Amplicon size | Notes relative to Cabriolet |
|---|---|---|---|---|
| *Bna*C*.FAD2.*b | Sel 1+2 f | FAD2grp13'UTR-r2 | 1212 | |
| *Bna*A*.FAD2.*b | Sel 1+2 new | FAD2 stop | 1133 | |
| *Bna*C*.FAD2.*a | FAD2 H3 f | FAD2 stop | 991 | Will not amplify |
| *BnaA.FAD2.*a | FAD2 3+4 start | FAD2 stop | 1173 | May produced mixed *Bna.FAD2.* a amplicon in other genotypes |
| *BnaA.FAD2.*a | Sel 3+4 f | FAD2 stop | 1133 | May produced mixed *Bna.FAD2.* a amplicon in other genotypes |
| *BnaA.FAD2.* a | Cons f 1698 | FAD2grp4 3'UTR2 | 966 | |

The PCR protocol was as follows:

### PCR mix

DNAx (100ng or 1µl of qiagen 96 well DNA extraction) Primer f (2µM) 2µl
Primer r (2µM) 2µl
10 x PCR buffer 2µl
Amplitaq gold (5u/µl) 0.2µl
dNTPs (2mM) 1.3
ddH₂O y
Total 20ul

### PCR cycle

| | | |
|---|---|---|
| Hotstart | 94°C for 5min | |
| | | |
| Cycles × 35 | | |
| | | |
| Denature | Ramp 0.5c/s to 94°C | |
| | Temp 94°C for 30s | |
| Anneal | | Ramp 0.5c/s to 57°C |
| | Temp 57°C for 30sec | |
| Extend | | Ramp 0.5C/s to 72°C |
| | Temp 72°C for 1min | |
| Final extend | | 72°C for 10min |

### Visualisation of product:

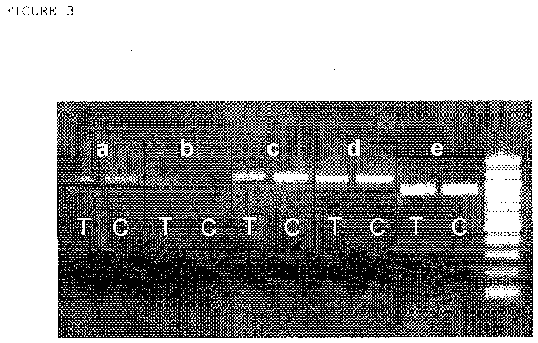

5µl run on 1.5% agarose gel - see Figure 3

### Alternative PCR conditions:

Improved amplification for some amplicons can be achieved by using a 1KB touchdown PCR protocol however this may lead to a reduction in specificity. Under these conditions *BnaC.FAD2.a* specific primers will readily amplify *BnaA.FAD2.a.*

### 'Touchdown' cycle

| | | |
|---|---|---|
| Hot start | 94°C for 5 min | |
| | | |
| Cycles × 15 | | |
| Denaturation | 94°C for 30s | |
| Annealing | 63°C for 30s | (-1°C/cycle) |
| Extension | 72°C for 1min | |
| | | |
| Cycles × 30 | | |
| Denaturation | 94°C for 30s | |
| Annealing | 53°C for 30s | |
| Extension | 72°C for 1min | |
| Final Extension | 72°C for 7min | |

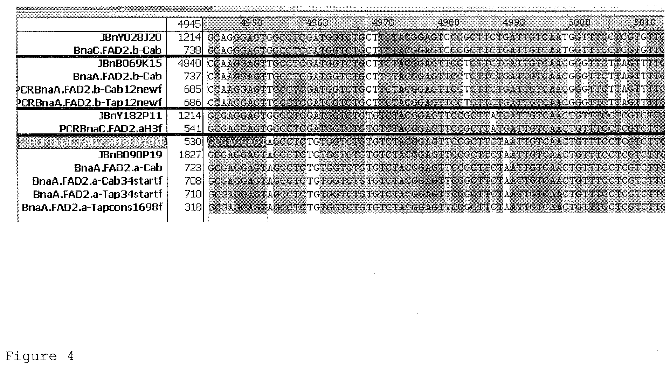

See the alignment of Tapidor and Cabriolet sequences against the different homologues. Note amplification of Cabriolet *BnaA.FAD2.a* with *BnaC.FAD2.a* primers under touchdown condition (trace highlighted) in figure 4.

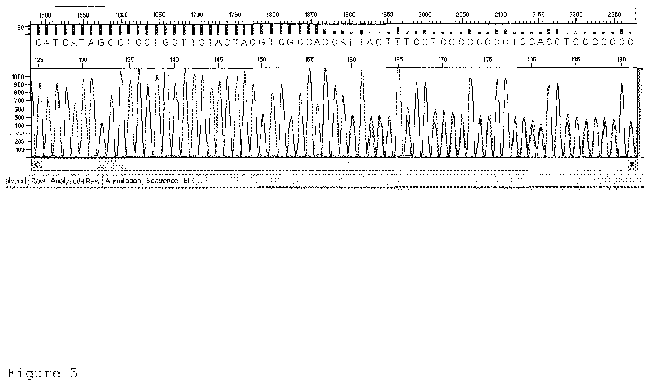

Sequencing should be performed on amplicons to determine they are clean. Figure 5 shows a mixed amplicon.

### EXAMPLE 2

### EMS MUTATION OF BRASSICA NAPUS SEEDS AND DEVELOPMENT OF MUTAGENISED POPULATION

We utilized *B. napus* variety Cabriolet for genetic mutagenesis by EMS to produce a starting population to screen. We utilized EMS levels of 0.4, 0.6 and 0.8%. We detected mutations via sequencing *BnaC.FAD2.b* allele specific PCR product with the forward specific primer (see Example 1 for details, PCR cycles etc.). We aligned sequences and compared mutagenized sequences with wild type sequences using the Mutation Surveyor software. We screened approximately 3000 lines, prioritising the higher EMS treatment levels. The mutagenesis was carried out as follows:
Chemical mutagenesis of ∼ 25,000 *Brassica napus* seeds variety Cabriolet using EMS (ethyl methanesulphonate) to produce a mutatagenised population, JBnCAB_E (the JIC consortium *Brassica napus* Cabriolet EMS population). Before carrying out the method stated here all safety material should be considered. EMS is a mutagen with limited evidence of carcinogenic effects. Equipment required/utilized, includes: Tube rotator (e.g. blood tube rotator - Stuart Scientific) with modified containment box attachment.

Spill tray; 15ml tubes (Corning BV Cat. No. 430790); Centrifuge tubes (Corning BV Cat. No. 430776); Tea strainer; Dry waste container; Small squares of blue towel; Large liquid waste container; Parafilm (R and L Slaughter Ltd. Cat. No. 291-1214).

Approximately 33000 *B. napus* seeds *var.* Cabriolet (breeders seed) were treated. Positive/negative controls: 2.5g each (∼ 500 seed) in 15ml falcon tube. Treatments x 5: 30g each (-6400 seed) in tubes.

Reagents utilized included:
0.02% Tween 20, Tween 20 (Sigma Aldridge Cat No. TS700-500ML). Tween 20 is a non-ionic detergent used as a base for the wetting out of seeds for the EMS treatment and for the subsequent washes. It is a very viscous liquid and, therefore, it is probably more accurate to make a higher percentage dilution first and dilute from here. For this protocol a 20% stock solution was produced. Therefore a 1:1000 dilution is required for the 0.02% solution. 1 litre 0.02% Tween 20 is required for the EMS treatment. That is: 1 ml 20% Tween 20 solution made up to 1 litre. 6 litres 0.02% Tween 20 is required for the washes.

10M NaOH, prepared from Sodium hydroxide pellets GPR 1Kg (Sigma Aldridge Cat. No. 06203). This is used for the neutralisation of the EMS. EMS is inactivated at 1M NaOH. Therefore, a final concentration of 2M can ensure inactivation. Approximately 3000ml of 10M NaOH is required to neutralise all of the EMS in the initial treatment and subsequent seed washing solutions. MW = 40g; 1M requires 40g made up to 1 litre. Therefore 10M requires 400g made up to 1 litre. This production of the solution is highly exothermic and therefore releases a great deal of heat. The NaOH should be added to gently stirring ice cold water and kept on ice until fully dissolved and cool.

EMS (Ethyl methanesulfonate), (Sigma Aldridge Cat. No. M0880). EMS is a well studied chemical mutagen that generates single base pair changes. It is highly toxic and can cause carcinogenic effects. EMS is sold by weight and has a density of 1.16g/ml. Therefore, 1g EMS=0.856ml. Before a full scale mutagenesis experiment, the level and time of exposure should be decided. This can be based on examples in the literature, although the strength of effect of EMS can vary between batches. Therefore a small scale trial is advised. EMS has a short half life (<100h in aqueous solution @20°C), therefore the procedure should be carried out with fresh dilutions only. For this work, 5 levels of EMS were determined based on those used for the production of current *B. napus* mutagenised populations. A positive and a negative control were also included in the work, as follows:
Negative control (No EMS):
   1 x 15ml tube, 2.5g seed at 0% EMS, 0.02% Tween 20 solution only
Positive control (2% EMS):
   1 x 15ml tube, 2.5g seed at 2% EMS, 1ml EMS up to 10ml with 0.02% Tween 20 solution.
Treatment 1 (0.2% EMS):
   30g seed, 150ml solution, 0.3ml EMS up to 150ml with 0.02% Tween 20 solution
Treatment 2 (0.4% EMS):
   30g seed, 150ml solution, 0.6ml EMS up to 150ml with 0.02% Tween 20 solution
Treatment 3 (0.6% EMS):
   30g seed, 150ml solution, 0.9ml EMS up to 150ml with 0.02% Tween 20 solution
Treatment 4 (0.8% EMS):
   30g seed, 150ml solution, 1.2 EMS up to 150ml with 0.02% Tween 20 solution
Treatment 5 (1% EMS):
   30g seed, 150ml solution, 1.5ml EMS up to 150ml with 0.02% Tween 20 solution

The EMS treatment was carried out as follows:

### Overnight treatment:

Set up a work area in a fume hood. Notify everyone in the lab you are about to use EMS and label area clearly. Label tubes clearly. Weigh out 2.5g seed into the positive and negative control tubes and 30g into the 5 treatment tubes. Create the treatment solutions by adding the required EMS to the 0.02% Tween 20 solution. Add 12.5ml of solution to the positive and negative control tubes and 150ml of solution to the treatment tubes. Catch any drips with the blue paper squares which are disposed of into the dry waste container. Replace the caps, ensure they are tightly closed and seal round the lids with parafilm. Fit into the rotating box and set to turn slowly overnight. Add waste EMS solutions to a 2 litre wide necked disposal bottle containing 400ml10M NaOH. Decontaminate the EMS/Tween 20 bottles by adding ∼200ml 2M NaOH. Seal lid tightly and shake well to ensure that all of the surface has been treated. Leave overnight. Close fume hood and leave overnight.

### EMS removal and washes:

Take the two litre disposal bottle from the previous day and slowly decant the EMS treatment solutions into the NaOH. Do this slowly to avoid losing seed or use a tea strainer to prevent seed loss. Rinse the seeds with 0.02% Tween 20 and decant the liquid into the waste bottle. Perform 10 washes of 150ml 0.02% Tween 20 for 20 minutes/wash, turning slowly. Decant waste into 10MNaOH to give a final volume of 2M NaOH. After the final wash seeds transferred to KWS for sowing. Wallpaper paste was added to the seeds to aid in dispersal the mixture sown by piping onto tray of peat and sand mix. Trays kept at 4°C for 2 days to stratify before transferring to glasshouse. Seedlings transplanted when large enough to handle.

### Development of the mutagenised population

### M₁ generation

At the four-leaf stage transplanted M₁ seedlings were vernalised at 4°C for six weeks before transferral to the glasshouse under 16h day length at 12-18°C, 65-75 % relative humidity. Plants were selfed to produce M₂ seed.

### M₂ generation

Two tramlines of M₂ seed/line of -7000 lines were drilled in the field (Sept 08). One plant/line was selected for tissue sampling and bagged for self seed production. Approximately four leaf disks/selected line were taken and Qiagen DNeasy 96 well DNA preparation (Cat. No. 69181) were performed to produce DNA stocks for mutation screening.

### Identification of mutants with altered FAD2 function

Following the genetic analysis described above which determined only one homologue, *BnaC.FAD2.b,* was functional in Cabriolet, PCR specific for this homologue, as described in Example 1 was performed on M₂ DNA from -3000 lines and sequenced using the standard protocol described previously. Lines containing both heterozygous and homozygous mutations in this homologue were detected by alignment of the sequence against a wild type trace using SoftGenetics Mutation Surveyor DNA Variant Analysis Software.

### Selection, growth and phenotyping of M₃ lines

Twelve M₃ seed from each line containing a mutation was sown and mutation status determined as for the M₂ generation. Where available homozygous, heterozygous and wild type outsegregant lines were trialled in a fully replicated glasshouse trial in the UK under standard *B*. *napus* growth conditions to compare oil profiles from the various mutation states. Further replicates of lines were grown for analysis in France. Selfed seed was collected from all lines and analysed for oil profile using NIR. Analysis of fatty acid methyl esters (FAMEs) by gas chromatography (GC) as detailed in Smooker and Wells et al. 2010 can be performed as an alternative.

### EXAMPLE 3

### PROCESS FOR PRODUCING HOMOZYGOUS PLANTS WITH NO FAD2 ACTIVITY

Once the methods disclosed herein are practiced on a given germplasm, a large number of seeds are produced with mutated genotypes. The seeds are germinated and grown, the genome is sequenced and the phenotype confirmed, as shown in figure 6. Those new varieties that grow to seed are selfed to produce homozygous plants for field testing and commercial cultivation and oleic acid production.

The crossing and backcrossing of mutant lines with a given conventional germplasm will result in populations segregating for the non-functional alleles of *FAD2* present within the mutant lines. PCR and sequencing of the products produced from these lines using the homologue specific primers described within this document will allow the selction of progeny carrying the non-functional alleles and thus these alleles can be tracked through a breeding program. Lines can be phenotyped to confirm the effect of these alleles on the plant phenotype. Lines possessing the non-functional alleles and superior agronomic phenotypes are bulked for field testing, commercial cultivation and oleic acid production.

### EXAMPLE 4

### PRODUCTION OF VARIOUS GERMPLASMS WITH ALTERED FAD2 ACTIVITY

Following the detailed procedures of Examples 1 and 2, and the more general teachings provided herein above, those skilled in the art will appreciate that, starting with any *B. napus* germplasm, (and, indeed, other *Brassicas*) one is able to produce a new germplasm in which the same or different mutations to those disclosed herein for *B. napus* variety Cabriolet are introduced. This will produce a new *B. napus* variety in which *BnaC.FAD2.b* is altered, resulting in little or no activity of the enzyme otherwise encoded by that homologue of the *FAD2* gene. By similarly introducing known mutations, truncations, deletions or the like into any remaining *FAD2* homologues, such as those already known in the art for such varieties, those skilled in the art are in a position, to rationally produce new varieties of *B. napus,* in which any or all of the FAD2 activity from any or all of the homologues of the genese encoding that activity has been reduced or removed entirely. Methods of directed gene deletion, silencing and the like, known in the art, may be utilized using the information provided herein, and that already known in the art, to quickly produce desired genotypes and/or, using for example DNA directed siRNA methods, desired phenotypes without even necessarily modifying the genotype. The sequences provided herein may further be used by those skilled in the art to design probes for marker assisted breeding, TILLING and other methods known in the art or which hereafter come into being, to identify and produce varieties of *B. napus,* and indeed, varieities of other species which have desired oleic acid profiles as compared to other fatty acid compositions.

Based on the disclosure provided herein, and the known art, those skilled in the art are enabled to follow a similar approach in other *Brasicas* to identify the full complement of *FAD2* genes in their genomes and to produce, for example, (*Brassica juncea, rapa, oleracea, campestris, carinata*) in which their full complement of *FAD2* genes have been compromised, silenced, mutated, as disclosed herien for the *FAD2* genes of *Brasica napus.* Various preferred features and embodiments of the present invention will now be described with reference to the following numbered paragraphs (para).
1. A mutated nucleic acid sequence of a *Brassica* FAD2-encoding nucleic acid sequence, wherein said nucleic acid sequence has one or more of the mutations shown in Figure 6 in relation to the *BnaC.FAD2.b* gene, or
   a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or
   a complementary sequence of one of said mutated, homologous or hybridizing nucleic acid sequences.
2. The nucleic acid sequence of para 1 wherein said mutation is one of the following mutations:
   224C to T (75 Pro to Leu), 241C to T (81 Leu to Phe),
   257G to A (86 Trp to **Stop**), 258G to A (86 Trp to **Stop**),
   270G to A (90 Trp to **Stop**), 284G to A (95 Cys to Tyr),
   296G to A (99 Gly to Asp), 310G to A (104 Ala to Thr),
   316G to A (106 Glu to Lys), 322G to A (108 Gly to Ser),
   328C to T (110 His to Tyr), 350G to A (117 Trp to **Stop**),
   355G to A (119 Asp to Asn), 367G to A (123 Gly to Ser),
   388C to T (130 Leu to Phe), 421C to T (141 His to Tyr),
   425G to A (143 Arg to Gln), 428G to A (143 Arg to His),
   437C to T (146 Ser to Phe), 458G to A (153 Arg to Lys),
   543G to A (181 Met to Ile), 566G to A (189 Gly to Asp),
   570G to A (190 Trp to **Stop**), 598G to A (200 Gly to Arg),
   616G to A (206 Gly to Tyr), 617G to A (206 Gly to Asp),
   623C to T (208 Ala to Val), 637C to T (213 Pro to Ser),
   642C to A (214 Asn to Lys), 643G to A (215 Ala to Thr),
   704C to T (235 Ala to Val), 710G to A (237 Cys to Tyr),
   716G to A (239 Gly to Asp), 743G to A (248 Gly to Glu),
   776C to T (259 Pro to Leu), 778C to T (260 Leu to Phe),
   890C to T (297 Thr to Ile).
3. The nucleic acid sequence of para 1 or 2 wherein, in relation to the *BnaA.FAD2.b* gene, the mutation is not one of the mutations shown in Figure 15.
4. A mutated nucleic acid sequence of a *Brassica* FAD2-encoding nucleic acid sequence, wherein said nucleic acid sequence encodes a FAD2 enzyme in which amino acid position 241 and/or 246 are changed in relation to the FAD2 enzyme encoded by *BnaC.FAD2.b* gene, or
   a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or
   a complementary sequence of one of said mutated, homologous or hybridizing nucleic acid sequences.
5. A mutated nucleic acid sequence of a *Brassica* FAD2-encoding nucleic acid sequence, wherein said nucleic acid sequence has a 1bp deletion at coding base position 215 in relation to the *BnaA.FAD2.b* gene, or
   a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or
   a complementary sequence of one of said mutated, homologous or hybridizing nucleic acid sequences.
6. A mutated nucleic acid sequence of a *Brassica* FAD2-encoding nucleic acid sequence, wherein said nucleic acid sequence has one or more of the mutations shown in Figure 15 in relation to the *BnaC.FAD2.b* gene, or
   a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence encodes an active FAD2 enzyme, or a hybridizing nucleic acid sequence which hybridizes under stringent conditions to said mutated nucleic acid sequence, provided that the complementary sequence of said hybridizing nucleic acid sequence encodes an active FAD2 enzyme, or
   a complementary sequence of one of said mutated, homologous or hybridizing nucleic acid sequences.
7. A protein comprising an amino acid sequence encoded by the nucleic acid of any preceding para.
8. A plant or part thereof in which more than two FAD2-encoding nucleic acid sequences or the corresponding FAD2 enzymes are inactivated such that the enzymatic activity is reduced compared to the non-inactivated version.
9. The plant or part thereof according to para 8 in which more than three FAD2-encoding nucleic acid sequences or the corresponding FAD2 enzymes are inactivated.
10. The plant or part thereof in which all of the FAD2 enzymes or the nucleic acid sequences encoding therefore are inactivated.
11. The plant or part thereof according to any one of paras 8 to 10 in which the nucleic acid sequence is one or more of those defined in paras 1 to 5.
12. The plant or part thereof according to any one of paras 8 to 11 which is *Brassica.*
13. The plant of part thereof according to para 12 which is selected from the group consisting of *Brassica juncea, napus, rapa, oleracea, campestris, carinata.*
14. The plant or part thereof according to para 13 which is *Brassica napus.*
15. The plant or part thereof according to para 14 which is derivable from the variety Cabriolet or Tapior.
16. The plant or part thereof of any one of paras 13 to 15 in which each of the four FAD2-encoding nucleic acid sequences or the corresponding FAD2 enzymes are inactivated.
17. The plant or part thereof of any one of paras 13 to 16 in which nucleic acid sequences are selected from the group consisting of:
   *BnaA.FAD2.a*
   *BnaC.FAD2.a*
   *BnaA.FAD2.b* or
   *BnaC.FAD2.b.*
18. The plant or a part thereof according to any one of paras 8 to 17 in which the part is a cell or seed.
19. A *Brassica* seed the fatty acid composition of which is greater, by at least 5%, than 74.5% oleic acid and which also has an 18:2 and/or 18:3 fatty acid fraction which is less by at least 5% than 16.1%.
20. A *Brassica* seed the fatty acid composition of which is less, by at least 5%, than 74.5% oleic acid and which also has an 18:2 and/or 18:3 fatty acid fraction which is greater by at least 5% than 16.1%.
21. The *Brassica* seed according to para 19 or para 20 wherein said *Brassica* is selected from the group consisting of *Brassica juncea, napus, rapa, oleracea, campestris, carinata.*
22. The *Brassica* seed according to para 21 which is a *Brasica napus* seed.
23. Fatty acid produced from the seed or the plant according to any one of paras 8-22.
24. A lubricant comprising the fatty acid of para 23.
25. A method of selecting plants having a desired fatty acid content comprising the following steps:
   crossing a plant or part thereof of the present invention with a parent plant;
   selecting from the progeny plants or parts thereof those comprising at least one sequence of any one of paras 1 to 6.
26. A method of providing a plant having increased monounsaturated fatty acid content, particularly oleic acid content, comprising the steps of:
   inactivating at least one FAD2-encoding nucleic acid sequence in a plant or part thereof,
   selecting a plant or part thereof or regenerated plant comprising said inactivation.
27. A method of producing a *Brassica* plant or a part thereof in which FAD2 is inactivated, said method selected from the group consisting of (a) silencing each of the homologues of the gene encoding FAD2 (b) producing mutants in the genome to reduce or destroy the activity of any encoded enzyme (c) selecting a variety of *Brassica* in which only one active *FAD2* gene exists, mutating that gene and producing a new variety in which all genes encoding oleate-12 desaturase are modified as compared with wild-type of that variety such that any oleate desaturase protein encoded is compromised in activity or is devoid of activity and (d) marker assisted selection of crosses between particular varieties to produce a variety which encodes little or no active FAD2 enzymatic activity.
28. A method of providing a plant having increased PUFA content, particularly linoleic acid and/or linolenic fatty acid content, comprising the steps of:
   activating at least one FAD2-encoding nucleic acid sequence of the present invention in a plant or part thereof,
   selecting a plant or part thereof or regenerated plant comprising said activation.
29. A method of producing a plant or a part thereof, said method selected from the group consisting of (a) upregulating expression of more or more of the homologues of the gene encoding FAD2 (b) producing mutants in the genome to increase the activity of any encoded FAD2 (c) selecting a variety of *Brassica* in which a *FAD2* gene exists, mutating that gene and producing a new variety in which a gene and preferably all genes encoding oleate-12 desaturase are modified as compared with wild-type of that variety such that any oleate desaturase protein encoded is improved in activity and (d) marker assisted selection of crosses between particular varieties to produce a variety which encodes active FAD2 enzymatic activity.
30. The method of any one of paras 25 to 29 wherein said plant or part thereof is *Brassica.*
31. The method according to para 30 wherein said *Brassica* is selected from the group consisting of *Brassica juncea, napus, rapa, oleracea, campestris, carinata.*
32. The method according to para 31 wherein said *Brasica* is *Brasica napus.*
33. A nucleic acid sequence which is identical in sequence to a portion of a modified *BnaC.FAD2.b* gene.
34. A nucleic acid sequence comprising at least 5 contiguous wild-type bases on either side of the mutation according to any one of paras 1 to 6.
35. The nucleic acid sequence selected from the group consisting of
   *SEQ ID.* 52: GTCTCCTCCCTCCAAAAAGT
   *SEQ ID. 53:* GTGTCTCCTCCCTCCAAA
   *SEQ ID.* 54: CTACAGAAACAAACATGGGC
   *SEQ ID. 55:* GTCTCTCCTCCCTCCAGC
   *SEQ ID. 56:* CTCTCCTCCCTCCAGCTCCC
   *SEQ ID.* 57: CTCTTCGACATCCTCCTCTC
   *SEQ ID. 58:* CCTCGTCCCTTACTTCTCCTG
   *SEQ ID. 59:* CCTCATAACTTATTGTTGTACCAG
   *SEQ ID. 60:* CAAGACGACCAGAGACAGC
   *SEQ ID. 61:* GAACTCGACAAATTTGCCTG
   SEQ ID. 62: GGGTGCAGGTGGAAGAATG probe f,
   SEQ ID. 63: TTGTTGTACCAGTACACACC probe r,
   SEQ ID. 64: conserved f GAGGGAGGCGAAGGAGTGTATC,
   SEQ ID. 65: conserved r CAGGAGAAGTAAGGGACGAGG,
   SEQ ID. 66: degenerate f ATTCCTTCCTNCTNCTNGTNCC,
   SEQ ID. 67: degenerate r GCTAAGTACAANGGNCANCC.
36. Use of a nucleic acid sequence according to para 35 in combination with a primer or probe which is identical in sequence to a portion of a modified *BnaA.FAD2.b, BnaC.FAD2a* or *BnaA.FAD2.a* gene.

## Claims

1. A mutated nucleic acid sequence of a *Brassica* FAD2-encoding nucleic acid sequence, wherein said nucleic acid sequence has one or more mutations selected from the group consisting of: 284G to A (95 Cys to Tyr) and 716G to A (239 Gly to Asp), in relation to the *BnaC.FAD2.b* gene, or
a homologous nucleic acid sequence derived from said mutated nucleic acid sequence by substitution, insertion or deletion of at least one nucleotide, and presenting at least 90% homology with said mutated nucleic acid sequence, provided that said homologous nucleic acid sequence does not encode an active FAD2 enzyme, or a hybridizing nucleic acid sequence which hybridizes under stringent conditions to said mutated nucleic acid sequence, provided that the complementary sequence of said hybridizing nucleic acid sequence does not encode an active FAD2 enzyme, or
a complementary sequence of one of said mutated, homologous or hybridizing nucleic acid sequences.

2. The nucleic acid sequence of claim 1, wherein said nucleic acid sequence further has one or more mutations selected from the group consisting of:
224C to T (75 Pro to Leu), 241C to T (81 Leu to Phe),
257G to A (86 Trp to **Stop**), 258G to A (86 Trp to **Stop**),
270G to A (90 Trp to **Stop**), 284G to A (95 Cys to Tyr),
296G to A (99 Gly to Asp), 310G to A (104 Ala to Thr),
316G to A (106 Glu to Lys), 322G to A (108 Gly to Ser),
328C to T (110 His to Tyr), 350G to A (117 Trp to **Stop**),
355G to A (119 Asp to Asn), 367G to A (123 Gly to Ser),
388C to T (130 Leu to Phe), 421C to T (141 His to Tyr),
425G to A (143 Arg to Gln), 428G to A (143 Arg to His),
437C to T (146 Ser to Phe), 458G to A (153 Arg to Lys),
543G to A (181 Met to Ile), 566G to A (189 Gly to Asp),
570G to A (190 Trp to **Stop**), 598G to A (200 Gly to Arg),
616G to A (206 Gly to Tyr), 617G to A (206 Gly to Asp),
623C to T (208 Ala to Val), 637C to T (213 Pro to Ser),
642C to A (214 Asn to Lys), 643G to A (215 Ala to Thr),
704C to T (235 Ala to Val), 710G to A (237 Cys to Tyr),
716G to A (239 Gly to Asp), 743G to A (248 Gly to Glu),
776C to T (259 Pro to Leu), 778C to T (260 Leu to Phe),
890C to T (297 Thr to Ile).

3. The nucleic acid sequence of claim 1 or 2, wherein, in relation to the *BnaA.FAD2.b* gene, the mutation is not one of the mutations shown in Figure 15.

4. A protein comprising an amino acid sequence encoded by the nucleic acid of any preceding claim.

5. A plant or part thereof in which more than two, preferably more than three, FAD2-encoding nucleic acid sequences or the corresponding FAD2 enzymes are inactivated such that the enzymatic activity is reduced compared to the non-inactivated version.

6. The plant or part thereof according to claim 5 in which all of the FAD2 enzymes or the nucleic acid sequences encoding therefore are inactivated.

7. The plant or part thereof according to claim 5 or 6, wherein one or more of the inactivated FAD2-encoding nucleic acid sequences is a nucleic acid sequence defined in claims 1-3.

8. The plant or part thereof according to any one of claims 5-7 which is *Brassica,* optionally selected from the group consisting of *Brassica juncea, napus, rapa, oleracea, campestris, carinata,* preferably *Brassica napus,* preferably *Brassica napus* derivable from the variety Cabriolet or Tapior.

9. The plant or part thereof of claim 8 in which each of the four FAD2-encoding nucleic acid sequences or the corresponding FAD2 enzymes are inactivated.

10. The plant or part thereof of claim 8 or 9 in which nucleic acid sequences are selected from the group consisting of:
*BnaA.FAD2.a*
*BnaC.FAD2.a*
*BnaA.FAD2.b* or
*BnaC.FAD2.b.*

11. The plant or a part thereof according to any one of claims 5-10 in which the part is a cell or seed.

12. A method of selecting plants having a desired fatty acid content comprising the following steps:
crossing a plant or part thereof of the present invention with a parent plant;
selecting from the progeny plants or parts thereof those comprising at least one sequence of any one of claims 1-3.

13. A method of providing a plant having increased monounsaturated fatty acid content, particularly oleic acid content, comprising the steps of:
inactivating at least one FAD2-encoding nucleic acid sequence in a plant or part thereof,
selecting a plant or part thereof or regenerated plant comprising said inactivation.

14. A method of producing a *Brassica* plant or a part thereof in which FAD2 is inactivated, said method selected from the group consisting of (a) silencing each of the homologues of the gene encoding FAD2 (b) producing mutants in the genome to reduce or destroy the activity of any encoded enzyme (c) selecting a variety of *Brassica* in which only one active *FAD2* gene exists, mutating that gene and producing a new variety in which all genes encoding oleate-12 desaturase are modified as compared with wild-type of that variety such that any oleate desaturase protein encoded is compromised in activity or is devoid of activity and (d) marker assisted selection of crosses between particular varieties to produce a variety which encodes little or no active FAD2 enzymatic activity.

15. The method of any one of claims 12-14, wherein said plant or part thereof is *Brassica,* optionally selected from the group consisting of *Brassica juncea, napus, rapa, oleracea, campestris, carinata,* preferably *Brassica napus.*

16. A nucleic acid sequence comprising at least 5 contiguous wild-type bases on either side of the mutation according to any one of claims 1-3.


SEQUENCE LISTING

<110>  Plant Bioscience Limited

<120>  MUTANTS

<130>  P043560EP

<140>  PCT/GB2012/050471
<141>  2012-03-02

<150>  GB 1103569.8
<151>  2011-03-02

<160>  83

<170>  PatentIn version 3.5

<210>  1
<211>  1155
<212>  DNA
<213>  Brassica napus

<400>  1
atgggtgcag gtggaagaat gcaagtgtct cctccctcca agaagtctga aaccgacacc      60

atcaagcgcg taccctgcga gacaccgccc ttcactgtcg gagaactcaa gaaagcaatc     120

ccaccgcact gtttcaaacg ctcgatccct cgctctttct cctacctcat ctgggacatc     180

atcatagcct cctgcttcta ctacgtcgcc accacttact tccctctcct ccctcaccct     240

ctctcctact tcgcctggcc tctctactgg gcctgccaag ggtgcgtcct aaccggcgtc     300

tgggtcatag cccacgagtg cggccaccac gccttcagcg actaccagtg gcttgacgac     360

accgtcggtc tcatcttcca ctccttcctc ctcgtccctt acttctcctg gaagtacagt     420

catcgacgcc accattccaa cactggctcc ctcgagagag acgaagtgtt tgtccccaag     480

aagaagtcag acatcaagtg gtacggcaag tacctcaaca accctttggg acgcaccgtg     540

atgttaacgg ttcagttcac tctcggctgg ccgttgtact tagccttcaa cgtctcggga     600

agaccttacg acggcggctt cgcttgccat ttccacccca acgctcccat ctacaacgac     660

cgcgagcgtc tccagatata catctccgac gctggcatcc tcgccgtctg ctacggtctc     720

ttccgttacg ccgccgcgca gggagtggcc tcgatggtct gcttctacgg agtcccgctt     780

ctgattgtca atggtttcct cgtgttgatc acttacttgc agcacacgca tccttccctg     840

cctcactacg attcgtccga gtgggattgg ttgaggggag ctttggctac cgttgacaga     900

gactacggaa tcttgaacaa ggtcttccac aatattaccg acacgcacgt ggcgcatcat     960

ctgttctcca cgatgccgca ttatcacgcg atggaagcta ccaaggcgat aaagccgata    1020

ctgggagagt attatcagtt cgatggaacg ccggtggtta aggcgatgtg gagggaggcg    1080

aaggagtgta tctatgtgga accggacagg caaggtgaga agaaaggtgt gttctggtac    1140

aacaataagt tatga                                                     1155

<210>  2
<211>  1155
<212>  DNA
<213>  Brassica napus

<400>  2
atgggtgcag gtggaagaat gcaagtgtct cctccctcca aaaagtctga aaccgacaac      60

atcaagcgcg taccctgcga gacaccgccc ttcactgtcg gagaactcaa gaaagcaatc     120

ccaccgcact gtttcaaacg ctcgatccct cgctctttct cctacctcat ctgggacatc     180

atcatagcct cctgcttcta ctacgtcgcc accacttact tccctctcct ccctcaccct     240

ctctcctact tcgcctggcc tctctactgg gcctgccagg gctgcgtcct aaccggcgtc     300

tgggtcatag cccacgagtg cggccaccac gccttcagcg actaccagtg gctggacgac     360

accgtcggcc tcatcttcca ctccttcctc ctcgtccctt acttctcctg gaagtacagt     420

catcgacgcc accattccaa cactggctcc ctcgagagag acgaagtgtt tgtccccaag     480

aagaagtcag acatcaagtg gtacggcaag tacctcaaca accctttggg acgcaccgtg     540

atgttaacgg ttcagttcac tctcggctgg cctttgtact tagccttcaa cgtctcgggg     600

agaccttacg acggcggctt cgcttgccat ttccacccca acgctcccat ctacaacgac     660

cgtgagcgtc tccagatata catctccgac gctggcatcc tcgccgtctg ctacggtctc     720

taccgctacg ctgctgtcca aggagttgcc tcgatggtct gcttctacgg agttcctctt     780

ctgattgtca acgggttctt agttttgatc acttacttgc agcacacgca tccttccctg     840

cctcactatg actcgtctga gtgggattgg ttgaggggag ctttggccac cgttgacaga     900

gactacggaa tcttgaacaa ggtcttccac aatatcacgg acacgcacgt ggcgcatcac     960

ctgttctcga ccatgccgca ttatcatgcg atggaagcta cgaaggcgat aaagccgata    1020

ctgggagagt attatcagtt cgatgggacg ccggtggtta aggcgatgtg gagggaggcg    1080

aaggagtgta tctatgtgga accggacagg caaggtgaga agaaaggtgt gttctggtac    1140

aacaataagt tatga                                                     1155

<210>  3
<211>  1155
<212>  DNA
<213>  Brassica napus

<400>  3
atgggcgcag gtggaagaat gcaagtctct cctccctcca gctcccccga aaccaaaacc      60

ctcaaacgcg tcccctgcga gacaccaccc ttcactctcg gagacctcaa gaaagcaatc     120

ccacctcact gcttcaaacg ctccatccct cgctccttct cctacctcct cttcgacatc     180

ctcgtctcct cctccctcta ccacctctcc acagcctact tccctctcct cccccaccct     240

ctcccttacc tcgcctggcc cctctactgg gcctgccaag gctgcgtcct aacgggcctc 300

tgggtcatcg cccacgaatg cggccaccac gccttcagcg accaccagtg gctggacgac 360

gccgtgggcc tcgtcttcca ctccttcctc ctcgtccctt acttctcctg gaagtacagc 420

catcgacgcc accattccaa caccggatcc ctcgagaggg atgaagtgtt cgtccccaag 480

aagaaatccg acatcaagtg gtacggaaag tacctcaaca acccgctagg acgcacggtg 540

atgctaaccg tccagttcac gctcggctgg ccgttgtact tagccttcaa cgtctctgga 600

agaccttaca gcgacggttt cgcttgccat ttccacccga acgctcccat ctacaacgac 660

cgcgagcgtc tccagatata catctctgac gctggcgtcc tctccgtatg ttacggtctc 720

taccgctacg ctggttcgcg aggagtggcc tcgatggtct gtgtctacgg agttccgctt 780

atgattgtca actgtttcct cgtcttgatc acttacttgc agcacacgca cccttcgctg 840

cctcactatg attcttcgga gtgggattgg ttgagaggag ctttggctac tgtggataga 900

gactatggaa tcttgaacaa ggtgtttcat aacatcacgg acacgcacgt ggcgcatcat 960

ctgttctcga cgatgccgca ttataacgcg atggaagcga ccaaggcgat aaagccgata 1020

cttggagagt attaccagtt tgatggaacg ccggtggtta aggcgatgtg gagggaggcg 1080

aaggagtgta tctatgttga accggatagg caaggtgaga agaaaggtgt gttctggtac 1140

aacaataagt tatga 1155

<210> 4
<211> 1141
<212> DNA
<213> Brassica napus

<400> 4
atgggcgcag gtggaagaat gcaagtctct cctccctcca gctcccccgg aaccaacacc 60

ctcaaacgcg tcccctgcga gacaccacca ttcactctcg gagacctcaa gaaagcaatc 120

ccacctcact gcttcaaacg ctccatccca cgctccttct cctcttcgac atcatcatct 180

cctcctcggc tcctccctct accacctctc cacagcctac ttccctctcc cttacctcgc 240

ctgacccctc tactgggcct gccaaggctg cgtcctaacg ggcctctggg tcatagccca 300

cgagtgcggc caccacgcct tcagcgacca ccagtggctg gacgacgccg tcggcctcgt 360

cttccactcc ttcctcctcg tcccgtactt ctcctggaag tacatccatg acgccaccat 420

tccaacaccg gatccctcga tagggacgaa gtgttcgtcc ccaagaagaa atccgacatc 480

aagtggtacg gcaagtacct caacaacccg ctaggacgca cggtgatgct aaccgtccag 540

ttcaagctcg gctggccgtt gtacttagcc ttcaacgtct cgggaagacc ttacagcgac 600

ggtttcgctt gccatttcca cccgaacgct cccatctaca acgaccgcga gcgtctccag 660

atatacatct ctgacgctgg cgtcctctcc gtatgttacg gtctctaccg ttacgctgct 720

tcgcgaggag tagcctctgt ggtctgtgtc tacggagttc cgcttctaat tgtcaactgt 780

ttcctcgtct tgatcactta cttgcagcac acgcaccctt cgctgcctca ctatgattct 840

tccgagtggg attggttgag aggagctttg gctactgtgg atagagacta tggaatcttg 900

aacaaggtgt tccataacat cacggacacg cacgtggcgc atcatctgtt ctcgacgatg 960

ccgcattata acgcgatgga agcgaccaag gcgataaagc cgatactttg gagagtatta 1020

ccagtttgat ggaacgccgg cggttaaggc gatgtggagg gaggcgaagg agtgtatcta 1080

tgttgaaccg gataggcaag gtgagaagaa aggtgtgttc tggtacaaca ataagttatg 1140

a 1141

<210> 5
<211> 384
<212> PRT
<213> Brassica napus

<400> 5

Met Gly Ala Gly Gly Arg Met Gln Val Ser Pro Pro Ser Lys Lys Ser
1               5                   10                  15

Glu Thr Asp Thr Ile Lys Arg Val Pro Cys Glu Thr Pro Pro Phe Thr
            20                  25                  30

Val Gly Glu Leu Lys Lys Ala Ile Pro Pro His Cys Phe Lys Arg Ser
        35                  40                  45

Ile Pro Arg Ser Phe Ser Tyr Leu Ile Trp Asp Ile Ile Ile Ala Ser
    50                  55                  60

Cys Phe Tyr Tyr Val Ala Thr Thr Tyr Phe Pro Leu Leu Pro His Pro
65                  70                  75                  80

Leu Ser Tyr Phe Ala Trp Pro Leu Tyr Trp Ala Cys Gln Gly Cys Val
                85                  90                  95

Leu Thr Gly Val Trp Val Ile Ala His Glu Cys Gly His His Ala Phe
            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Lys Lys Ser Asp Ile Lys Trp Tyr Gly Lys Tyr Leu Asn Asn Pro Leu
                165                 170                 175

Gly Arg Thr Val Met Leu Thr Val Gln Phe Thr Leu Gly Trp Pro Leu
            180                 185                 190

Tyr Leu Ala Phe Asn Val Ser Gly Arg Pro Tyr Asp Gly Gly Phe Ala
        195                 200                 205

Cys His Phe His Pro Asn Ala Pro Ile Tyr Asn Asp Arg Glu Arg Leu
    210                 215                 220

Gln Ile Tyr Ile Ser Asp Ala Gly Ile Leu Ala Val Cys Tyr Gly Leu
225                 230                 235                 240

Phe Arg Tyr Ala Ala Ala Gln Gly Val Ala Ser Met Val Cys Phe Tyr
                245                 250                 255

Gly Val Pro Leu Leu Ile Val Asn Gly Phe Leu Val Leu Ile Thr Tyr
            260                 265                 270

Leu Gln His Thr His Pro Ser Leu Pro His Tyr Asp Ser Ser Glu Trp
        275                 280                 285

Asp Trp Leu Arg Gly Ala Leu Ala Thr Val Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr His Ala Met Glu Ala Thr Lys Ala
                325                 330                 335

Ile Lys Pro Ile Leu Gly Glu Tyr Tyr Gln Phe Asp Gly Thr Pro Val
            340                 345                 350

Val Lys Ala Met Trp Arg Glu Ala Lys Glu Cys Ile Tyr Val Glu Pro
        355                 360                 365

Asp Arg Gln Gly Glu Lys Lys Gly Val Phe Trp Tyr Asn Asn Lys Leu
    370                 375                 380

<210>  6
<211>  384
<212>  PRT
<213>  Brassica napus

<400>  6

Met Gly Ala Gly Gly Arg Met Gln Val Ser Pro Pro Ser Lys Lys Ser
1               5                   10                  15

Glu Thr Asp Asn Ile Lys Arg Val Pro Cys Glu Thr Pro Pro Phe Thr
            20                  25                  30

Val Gly Glu Leu Lys Lys Ala Ile Pro Pro His Cys Phe Lys Arg Ser
        35                  40                  45

Ile Pro Arg Ser Phe Ser Tyr Leu Ile Trp Asp Ile Ile Ile Ala Ser
    50                  55                  60

Cys Phe Tyr Tyr Val Ala Thr Thr Tyr Phe Pro Leu Leu Pro His Pro
65                  70                  75                  80

Leu Ser Tyr Phe Ala Trp Pro Leu Tyr Trp Ala Cys Gln Gly Cys Val
                85                  90                  95

Leu Thr Gly Val Trp Val Ile Ala His Glu Cys Gly His His Ala Phe
            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Lys Lys Ser Asp Ile Lys Trp Tyr Gly Lys Tyr Leu Asn Asn Pro Leu
                165                 170                 175

Gly Arg Thr Val Met Leu Thr Val Gln Phe Thr Leu Gly Trp Pro Leu
            180                 185                 190

Tyr Leu Ala Phe Asn Val Ser Gly Arg Pro Tyr Asp Gly Gly Phe Ala
        195                 200                 205

Cys His Phe His Pro Asn Ala Pro Ile Tyr Asn Asp Arg Glu Arg Leu
    210                 215                 220

Gln Ile Tyr Ile Ser Asp Ala Gly Ile Leu Ala Val Cys Tyr Gly Leu
225                 230                 235                 240

Tyr Arg Tyr Ala Ala Val Gln Gly Val Ala Ser Met Val Cys Phe Tyr
                245                 250                 255

Gly Val Pro Leu Leu Ile Val Asn Gly Phe Leu Val Leu Ile Thr Tyr
            260                 265                 270

Leu Gln His Thr His Pro Ser Leu Pro His Tyr Asp Ser Ser Glu Trp
        275                 280                 285

Asp Trp Leu Arg Gly Ala Leu Ala Thr Val Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr His Ala Met Glu Ala Thr Lys Ala
                325                 330                 335

Ile Lys Pro Ile Leu Gly Glu Tyr Tyr Gln Phe Asp Gly Thr Pro Val
            340                 345                 350

Val Lys Ala Met Trp Arg Glu Ala Lys Glu Cys Ile Tyr Val Glu Pro
        355                 360                 365

Asp Arg Gln Gly Glu Lys Lys Gly Val Phe Trp Tyr Asn Asn Lys Leu
    370                 375                 380

<210>  7
<211>  384
<212>  PRT
<213>  Brassica napus

<400>  7

Met Gly Ala Gly Gly Arg Met Gln Val Ser Pro Pro Ser Ser Ser Pro
1               5                   10                  15

Glu Thr Lys Thr Leu Lys Arg Val Pro Cys Glu Thr Pro Pro Phe Thr
            20                  25                  30

Leu Gly Asp Leu Lys Lys Ala Ile Pro Pro His Cys Phe Lys Arg Ser
        35                  40                  45

Ile Pro Arg Ser Phe Ser Tyr Leu Leu Phe Asp Ile Leu Val Ser Ser
    50                  55                  60

Ser Leu Tyr His Leu Ser Thr Ala Tyr Phe Pro Leu Leu Pro His Pro
65                  70                  75                  80

Leu Pro Tyr Leu Ala Trp Pro Leu Tyr Trp Ala Cys Gln Gly Cys Val
                85                  90                  95

Leu Thr Gly Leu Trp Val Ile Ala His Glu Cys Gly His His Ala Phe
                100                 105                 110

Ser Asp His Gln Trp Leu Asp Asp Ala Val Gly Leu Val Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Lys Lys Ser Asp Ile Lys Trp Tyr Gly Lys Tyr Leu Asn Asn Pro Leu
                165                 170                 175

Gly Arg Thr Val Met Leu Thr Val Gln Phe Thr Leu Gly Trp Pro Leu
            180                 185                 190

Tyr Leu Ala Phe Asn Val Ser Gly Arg Pro Tyr Ser Asp Gly Phe Ala
        195                 200                 205

Cys His Phe His Pro Asn Ala Pro Ile Tyr Asn Asp Arg Glu Arg Leu
    210                 215                 220

Gln Ile Tyr Ile Ser Asp Ala Gly Val Leu Ser Val Cys Tyr Gly Leu
225                 230                 235                 240

Tyr Arg Tyr Ala Gly Ser Arg Gly Val Ala Ser Met Val Cys Val Tyr
                245                 250                 255

Gly Val Pro Leu Met Ile Val Asn Cys Phe Leu Val Leu Ile Thr Tyr
            260                 265                 270

Leu Gln His Thr His Pro Ser Leu Pro His Tyr Asp Ser Ser Glu Trp
        275                 280                 285

Asp Trp Leu Arg Gly Ala Leu Ala Thr Val Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr Asn Ala Met Glu Ala Thr Lys Ala
                325                 330                 335

Ile Lys Pro Ile Leu Gly Glu Tyr Tyr Gln Phe Asp Gly Thr Pro Val

340                 345                 350

Val Lys Ala Met Trp Arg Glu Ala Lys Glu Cys Ile Tyr Val Glu Pro
        355                 360                 365

Asp Arg Gln Gly Glu Lys Lys Gly Val Phe Trp Tyr Asn Asn Lys Leu
    370                 375                 380

<210>  8
<211>  373
<212>  PRT
<213>  Brassica napus

<400>  8

Met Gly Ala Gly Gly Arg Met Gln Val Ser Pro Pro Ser Ser Ser Pro
1               5                   10                  15

Gly Thr Asn Thr Leu Lys Arg Val Pro Cys Glu Thr Pro Pro Phe Thr
            20                  25                  30

Leu Gly Asp Leu Lys Lys Ala Ile Pro Pro His Cys Phe Lys Arg Ser
        35                  40                  45

Ile Pro Arg Ser Phe Ser Ser Ser Thr Ser Ser Ser Pro Pro Arg Leu
    50                  55                  60

Leu Pro Leu Pro Pro Leu His Ser Leu Leu Pro Ser Pro Leu Pro Arg
65                  70                  75                  80

Leu Thr Pro Leu Leu Gly Leu Pro Arg Leu Arg Pro Asn Gly Pro Leu
                85                  90                  95

Gly His Ser Pro Arg Val Arg Pro Pro Arg Leu Gln Arg Pro Pro Val
            100                 105                 110

Ala Gly Arg Arg Arg Arg Pro Arg Leu Pro Leu Leu Pro Pro Arg Pro
        115                 120                 125

Val Leu Leu Leu Glu Val His Pro Arg His His Ser Asn Thr Gly Ser
    130                 135                 140

Leu Asp Arg Asp Glu Val Phe Val Pro Lys Lys Lys Ser Asp Ile Lys
145                 150                 155                 160

Trp Tyr Gly Lys Tyr Leu Asn Asn Pro Leu Gly Arg Thr Val Met Leu
                165                 170                 175

Thr Val Gln Phe Lys Leu Gly Trp Pro Leu Tyr Leu Ala Phe Asn Val

180                 185                 190

Ser Gly Arg Pro Tyr Ser Asp Gly Phe Ala Cys His Phe His Pro Asn
        195                 200                 205

Ala Pro Ile Tyr Asn Asp Arg Glu Arg Leu Gln Ile Tyr Ile Ser Asp
    210                 215                 220

Ala Gly Val Leu Ser Val Cys Tyr Gly Leu Tyr Arg Tyr Ala Ala Ser
225                 230                 235                 240

Arg Gly Val Ala Ser Val Val Cys Val Tyr Gly Val Pro Leu Leu Ile
                245                 250                 255

Val Asn Cys Phe Leu Val Leu Ile Thr Tyr Leu Gln His Thr His Pro
            260                 265                 270

Ser Leu Pro His Tyr Asp Ser Ser Glu Trp Asp Trp Leu Arg Gly Ala
        275                 280                 285

Leu Ala Thr Val Asp Arg Asp Tyr Gly Ile Leu Asn Lys Val Phe His
    290                 295                 300

Asn Ile Thr Asp Thr His Val Ala His His Leu Phe Ser Thr Met Pro
305                 310                 315                 320

His Tyr Asn Ala Met Glu Ala Thr Lys Ala Ile Lys Pro Ile Leu Trp
                325                 330                 335

Arg Val Leu Pro Val Trp Asn Ala Gly Gly Gly Asp Val Glu Gly Gly
            340                 345                 350

Glu Gly Val Tyr Leu Cys Thr Gly Ala Arg Glu Glu Arg Cys Val Leu
        355                 360                 365

Val Gln Gln Val Met
    370

<210>  9
<211>  1155
<212>  DNA
<213>  Brassica napus

<400>  9
atgggtgcag gtggaagaat gcaagtgtct cctccctcca agaagtctga aaccgacacc      60

atcaagcgcg taccctgcga gacaccgccc ttcactgtcg gagaactcaa gaaagcaatc     120

ccaccgcact gtttcaaacg ctcgatccct cgctctttct cctacctcat ctgggacatc     180

atcatagcct cctgcttcta ctacgtcgcc accacttact tccctctcct ccctcaccct 240

ctctcctact tcgcctggcc tctctactgg gcctgccaag ggtgcgtcct aaccggcgtc 300

tgggtcatag cccacgagtg cggccaccac gccttcagcg actaccagtg gcttgacgac 360

accgtcggtc tcatcttcca ctccttcctc ctcgtccctt acttctcctg gaagtacagt 420

catcgacgcc accattccaa cactggctcc ctcgagagag acgaagtgtt tgtccccaag 480

aagaagtcag acatcaagtg gtacggcaag tacctcaaca accctttggg acgcaccgtg 540

atgttaacgg ttcagttcac tctcggctgg ccgttgtact tagccttcaa cgtctcggga 600

agaccttacg acggcggctt cgcttgccat ttccacccca acgctcccat ctacaacgac 660

cgcgagcgtc tccagatata catctccgac gctggcatcc tcgccgtctg ctacggtctc 720

ttccgttacg ccgccgcgca gggagtggcc tcgatggtct gcttctacgg agtcccgctt 780

ctgattgtca atggtttcct cgtgttgatc acttacttgc agcacacgca tccttccctg 840

cctcactacg attcgtccga gtgggattgg ttgaggggag ctttggctac cgttgacaga 900

gactacggaa tcttgaacaa ggtcttccac aatattaccg acacgcacgt ggcgcatcat 960

ctgttctcca cgatgccgca ttatcacgcg atggaagcta ccaaggcgat aaagccgata 1020

ctgggagagt attatcagtt cgatgggacg ccggtggtta aggcgatgtg gagggaggcg 1080

aaggagtgta tctatgtgga accggacagg caaggtgaga agaaaggtgt gttctggtac 1140

aacaataagt tatga 1155

<210> 10
<211> 1154
<212> DNA
<213> Brassica napus

<400> 10
atgggtgcag gtggaagaat gcaagtgtct cctccctcca aaaagtctga aaccgacaac 60

atcaagcgcg taccctgcga gacaccgccc ttcactgtcg gagaactcaa gaaagcaatc 120

ccaccgcact gtttcaaacg ctcgatccct cgctctttct cctacctcat ctgggacatc 180

atcatagcct cctgcttcta ctacgtcgcc accattactt ccctctcctc cctcaccctc 240

tctcctactt cgcctggcct ctctactggg cctgccaggg ctgcgtccta accggcgtct 300

gggtcatagc ccacgagtgc ggccaccacg ccttcagcga ctaccagtgg ctggacgaca 360

ccgtcggcct catcttccac tccttcctcc tcgtccctta cttctcctgg aagtacagtc 420

atcgacgcca ccattccaac actggctccc tcgagagaga cgaagtgttt gtccccaaga 480

agaagtcaga catcaagtgg tacggcaagt acctcaacaa ccctttggga cgcaccgtga 540

tgttaacggt tcagttcact ctcggctggc ctttgtactt agccttcaac gtctcgggga 600

gaccttacga cggcggcttc gcttgccatt tccaccccaa cgctcccatc tacaacgacc 660

gtgagcgtct ccagatatac atctccgacg ctggcatcct cgccgtctgc tacggtctct 720

accgctacgc tgctgtccaa ggagttgcct cgatggtctg cttctacgga gttcctcttc 780

tgattgtcaa cgggttctta gttttgatca cttacttgca gcacacgcat ccttccctgc 840

ctcactatga ctcgtctgag tgggattggt tgaggggagc tttggccacc gttgacagag 900

actacggaat cttgaacaag gtcttccaca atatcacgga cacgcacgtg gcgcatcacc 960

tgttctcgac catgccgcat tatcatgcga tggaagctac gaaggcgata aagccgatac 1020

tgggagagta ttatcagttc gatgggacgc cggtggttaa ggcgatgtgg agggaggcga 1080

aggagtgtat ctatgtggaa ccggacaggc aaggtgagaa gaaaggtgtg ttctggtaca 1140

acaataagtt atga 1154

<210> 11
<211> 1141
<212> DNA
<213> Brassica napus

<220>
<221> misc_feature
<222> (697)..(722)
<223> n is a, c, g, or t

<220>
<221> misc_feature
<222> (816)..(817)
<223> n is a, c, g, or t

<220>
<221> misc_feature
<222> (820)..(820)
<223> n is a, c, g, or t

<220>
<221> misc_feature
<222> (822)..(822)
<223> n is a, c, g, or t

<400> 11
atgggcgcag gtggaagaat gcaagtctct cctccctcca gctcccccgg aaccaacacc 60

ctcaaacgcg tcccctgcga gacaccacca ttcactctcg gagacctcaa gaaagcaatc 120

ccacctcact gcttcaaacg ctccatccca cgctccttct cctcttcgac atcatcatct 180

cctcctcggc tcctccctct accacctctc cacagcctac ttccctctcc cttacctcgc 240

ctgacccctc tactgggcct gccaaggctg cgtcctaacg ggcctctggg tcatagccca 300

cgagtgcggc caccacgcct tcagcgacca ccagtggctg gacgacgccg tcggcctcgt 360

cttccactcc ttcctcctcg tcccgtactt ctcctggaag tacatccatg acgccaccat 420

tccaacaccg gatccctcga tagggacgaa gtgttcgtcc ccaagaagaa atccgacatc 480

aagtggtacg gcaagtacct caacaacccg ctaggacgca cggtgatgct aaccgtccag 540

ttcaagctcg gctggccgtt gtacttagcc ttcaacgtct cgggaagacc ttacagcgac 600

ggtttcgctt gccatttcca cccgaacgct cccatctaca acgaccgcga gcgtctccag 660

atatacatct ctgacgctgg cgtcctctcc gtatgtnnnn nnnnnnnnnn nnnnnnnnnn 720

nngcgaggag tagcctctgt ggtctgtgtc tacggagttc cgcttctaat tgtcaactgt 780

ttcctcgtct tgatcactta cttgcagcac acgcannctn cnctgcctca ctatgattct 840

tccgagtggg attggttgag aggagctttg gctactgtgg atagagacta tggaatcttg 900

aacaaggtgt tccataacat cacggacacg cacgtggcgc atcatctgtt ctccacgatg 960

ccgcattata acgcgatgga agcgaccaag gcgataaagc cgatactttg gagagtatta 1020

ccagtttgat ggaacgccgg cggttaaggc gatgtggagg gaggcgaagg agtgtatcta 1080

tgttgaaccg gataggcaag gtgagaagaa aggtgtgttc tggtacaaca ataagttatg 1140

a 1141

<210> 12
<211> 384
<212> PRT
<213> Brassica napus

<400> 12

Met Gly Ala Gly Gly Arg Met Gln Val Ser Pro Pro Ser Lys Lys Ser
1               5                   10                  15

Glu Thr Asp Thr Ile Lys Arg Val Pro Cys Glu Thr Pro Pro Phe Thr
            20                  25                  30

Val Gly Glu Leu Lys Lys Ala Ile Pro Pro His Cys Phe Lys Arg Ser
        35                  40                  45

Ile Pro Arg Ser Phe Ser Tyr Leu Ile Trp Asp Ile Ile Ile Ala Ser
    50                  55                  60

Cys Phe Tyr Tyr Val Ala Thr Thr Tyr Phe Pro Leu Leu Pro His Pro
65                  70                  75                  80

Leu Ser Tyr Phe Ala Trp Pro Leu Tyr Trp Ala Cys Gln Gly Cys Val
                85                  90                  95

Leu Thr Gly Val Trp Val Ile Ala His Glu Cys Gly His His Ala Phe
            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Lys Lys Ser Asp Ile Lys Trp Tyr Gly Lys Tyr Leu Asn Asn Pro Leu
                165                 170                 175

Gly Arg Thr Val Met Leu Thr Val Gln Phe Thr Leu Gly Trp Pro Leu
            180                 185                 190

Tyr Leu Ala Phe Asn Val Ser Gly Arg Pro Tyr Asp Gly Gly Phe Ala
        195                 200                 205

Cys His Phe His Pro Asn Ala Pro Ile Tyr Asn Asp Arg Glu Arg Leu
    210                 215                 220

Gln Ile Tyr Ile Ser Asp Ala Gly Ile Leu Ala Val Cys Tyr Gly Leu
225                 230                 235                 240

Phe Arg Tyr Ala Ala Ala Gln Gly Val Ala Ser Met Val Cys Phe Tyr
                245                 250                 255

Gly Val Pro Leu Leu Ile Val Asn Gly Phe Leu Val Leu Ile Thr Tyr
            260                 265                 270

Leu Gln His Thr His Pro Ser Leu Pro His Tyr Asp Ser Ser Glu Trp
        275                 280                 285

Asp Trp Leu Arg Gly Ala Leu Ala Thr Val Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr His Ala Met Glu Ala Thr Lys Ala
                325                 330                 335

Ile Lys Pro Ile Leu Gly Glu Tyr Tyr Gln Phe Asp Gly Thr Pro Val
            340                 345                 350

Val Lys Ala Met Trp Arg Glu Ala Lys Glu Cys Ile Tyr Val Glu Pro
        355                 360                 365

Asp Arg Gln Gly Glu Lys Lys Gly Val Phe Trp Tyr Asn Asn Lys Leu
    370                 375                 380

<210>  13
<211>  373
<212>  PRT
<213>  Brassica napus

<400>  13

Met Gly Ala Gly Gly Arg Met Gln Val Ser Pro Pro Ser Lys Lys Ser
1               5                   10                  15

Glu Thr Asp Asn Ile Lys Arg Val Pro Cys Glu Thr Pro Pro Phe Thr
            20                  25                  30

Val Gly Glu Leu Lys Lys Ala Ile Pro Pro His Cys Phe Lys Arg Ser
        35                  40                  45

Ile Pro Arg Ser Phe Ser Tyr Leu Ile Trp Asp Ile Ile Ile Ala Ser
    50                  55                  60

Cys Phe Tyr Tyr Val Ala Thr Ile Thr Ser Leu Ser Ser Leu Thr Leu
65                  70                  75                  80

Ser Pro Thr Ser Pro Gly Leu Ser Thr Gly Pro Ala Arg Ala Ala Ser
                85                  90                  95

Pro Ala Ser Gly Ser Pro Thr Ser Ala Ala Thr Thr Pro Ser Ala Thr
            100                 105                 110

Thr Ser Gly Trp Thr Thr Pro Ser Ala Ser Ser Ser Thr Pro Ser Ser
        115                 120                 125

Ser Ser Leu Thr Ser Pro Gly Ser Thr Val Ile Asp Ala Thr Ile Pro
    130                 135                 140

Thr Leu Ala Pro Ser Arg Glu Thr Lys Cys Leu Ser Pro Arg Arg Ser
145                 150                 155                 160

Gln Thr Ser Ser Gly Thr Ala Ser Thr Ser Thr Thr Leu Trp Asp Ala
                165                 170                 175

Pro Cys Arg Phe Ser Ser Leu Ser Ala Gly Leu Cys Thr Pro Ser Thr
            180                 185                 190

Ser Arg Gly Asp Leu Thr Thr Ala Ala Ser Leu Ala Ile Ser Thr Pro
        195                 200                 205

Thr Leu Pro Ser Thr Thr Thr Val Ser Val Ser Arg Tyr Thr Ser Pro
    210                 215                 220

Thr Leu Ala Ser Ser Pro Ser Ala Thr Val Ser Thr Ala Thr Leu Leu
225                 230                 235                 240

Ser Lys Glu Leu Pro Arg Trp Ser Ala Ser Thr Glu Phe Leu Phe Leu
                245                 250                 255

Ser Thr Gly Ser Phe Ser Leu Thr Cys Ser Thr Arg Ile Leu Pro Cys
            260                 265                 270

Leu Thr Met Thr Arg Leu Ser Gly Ile Gly Gly Glu Leu Trp Pro Pro
        275                 280                 285

Leu Thr Glu Thr Thr Glu Ser Thr Arg Ser Ser Thr Ile Ser Arg Thr
    290                 295                 300

Arg Thr Trp Arg Ile Thr Cys Ser Arg Pro Cys Arg Ile Ile Met Arg
305                 310                 315                 320

Trp Lys Leu Arg Arg Arg Ser Arg Tyr Trp Glu Ser Ile Ile Ser Ser
                325                 330                 335

Met Gly Arg Arg Trp Leu Arg Arg Cys Gly Gly Arg Arg Arg Ser Val
            340                 345                 350

Ser Met Trp Asn Arg Thr Gly Lys Val Arg Arg Lys Val Cys Ser Gly
        355                 360                 365

Thr Thr Ile Ser Tyr
    370

<210>  14
<211>  373
<212>  PRT
<213>  Brassica napus

<220>
<221>  misc_feature
<222>  (232)..(240)
<223>  Xaa can be any naturally occurring amino acid

<220>
<221>  misc_feature
<222>  (271)..(273)
<223>  Xaa can be any naturally occurring amino acid

<400>  14

Met Gly Ala Gly Gly Arg Met Gln Val Ser Pro Pro Ser Ser Ser Pro
1               5                   10                  15

Gly Thr Asn Thr Leu Lys Arg Val Pro Cys Glu Thr Pro Pro Phe Thr
            20                  25                  30

Leu Gly Asp Leu Lys Lys Ala Ile Pro Pro His Cys Phe Lys Arg Ser
        35                  40                  45

Ile Pro Arg Ser Phe Ser Ser Ser Thr Ser Ser Ser Pro Pro Arg Leu
    50                  55                  60

Leu Pro Leu Pro Pro Leu His Ser Leu Leu Pro Ser Pro Leu Pro Arg
65                  70                  75                  80

Leu Thr Pro Leu Leu Gly Leu Pro Arg Leu Arg Pro Asn Gly Pro Leu
                85                  90                  95

Gly His Ser Pro Arg Val Arg Pro Pro Arg Leu Gln Arg Pro Pro Val
            100                 105                 110

Ala Gly Arg Arg Arg Arg Pro Arg Leu Pro Leu Leu Pro Pro Arg Pro
        115                 120                 125

Val Leu Leu Leu Glu Val His Pro Arg His His Ser Asn Thr Gly Ser
    130                 135                 140

Leu Asp Arg Asp Glu Val Phe Val Pro Lys Lys Lys Ser Asp Ile Lys
145                 150                 155                 160

Trp Tyr Gly Lys Tyr Leu Asn Asn Pro Leu Gly Arg Thr Val Met Leu
                165                 170                 175

Thr Val Gln Phe Lys Leu Gly Trp Pro Leu Tyr Leu Ala Phe Asn Val
            180                 185                 190

Ser Gly Arg Pro Tyr Ser Asp Gly Phe Ala Cys His Phe His Pro Asn
        195                 200                 205

Ala Pro Ile Tyr Asn Asp Arg Glu Arg Leu Gln Ile Tyr Ile Ser Asp
    210                 215                 220

Ala Gly Val Leu Ser Val Cys Xaa Xaa Xaa Xaa Xaa Xaa Xaa Xaa Xaa
225                 230                 235                 240

Arg Gly Val Ala Ser Val Val Cys Val Tyr Gly Val Pro Leu Leu Ile
                245                 250                 255

Val Asn Cys Phe Leu Val Leu Ile Thr Tyr Leu Gln His Thr Xaa Xaa
            260                 265                 270

Xaa Leu Pro His Tyr Asp Ser Ser Glu Trp Asp Trp Leu Arg Gly Ala
        275                 280                 285

Leu Ala Thr Val Asp Arg Asp Tyr Gly Ile Leu Asn Lys Val Phe His
    290                 295                 300

Asn Ile Thr Asp Thr His Val Ala His His Leu Phe Ser Thr Met Pro
305                 310                 315                 320

His Tyr Asn Ala Met Glu Ala Thr Lys Ala Ile Lys Pro Ile Leu Trp
                325                 330                 335

Arg Val Leu Pro Val Trp Asn Ala Gly Gly Gly Asp Val Glu Gly Gly
            340                 345                 350

Glu Gly Val Tyr Leu Cys Thr Gly Ala Arg Glu Glu Arg Cys Val Leu
        355                 360                 365

Val Gln Gln Val Met
    370

<210> 15
<211> 1166
<212> DNA
<213> Brassica napus

<400> 15
ttacgtccta atcatcgtgt ctctggtcgt cttaattgtg cggagaacgc aaaaagcaat 60

cccaccgcat gctcccctcg atccctcgct ctttctccta cctcatctgg cgacatcatc 120

atagcctcct gcttctacta cgtcgccacc acttacttcc ctctcctccc tcaccctctc 180

tcctacttcg cctggcctct ctactgggcc tgccaagggt gcgtcctaac cggcgtctgg 240

gtcatagccc acgagtgcgg ccaccacgcc ttcagcgact accagtggct taacgacacc 300

gtcggtctca tcttccactc cttcctcctc gtcccttact tctcctggaa gtacagtcat 360

cgacgccacc attccaacac tggctccctc gagagagacg aagtgtttgt ccccaagaag 420

aagtcagaca tcaagtggta cggcaagtac ctcaacaacc ctttgggacg caccgtgatg 480

ttaacggttc agttcactct cggctggccg ttgtacttag ccttcaacgt ctcgggaaga 540

ccttacgacg gcggcttcgc ttgccatttc caccccaacg ctcccatcta caacgaccgc 600

gagcgtctcc agatatacat ctccgacgct ggcatcctcg ccgtctgcta cggtctcttc 660

cgttacgccg ccgcgcaggg agtggcctcg atggtctgct tctacggagt cccgcttctg 720

attgtcaatg gtttcctcgt gttgatcact tacttgcagc acacgcatcc ttccctgcct 780

cactacgatt cgtccgagtg ggattggttg agggagcttt ggctaccgtt gacagagact 840

acggaatctt gaacaagtct tccccaatat taccgacccg cacgtggcgc atcatctgtt 900

ctccacgatg ccgcattatc cacgcgatgg aaagctacca agccgataaa gccgaatact 960

gggagaagtt ttatcagttc gatggacgcc ggtggttagg cgaatgtgag gaaggcgaag 1020

aatgtatcta ttgttgaacc gaacaggcca gtggaagaag gtgtgtgttc tgttcaacaa 1080

taagttatga ggattgatga tggtgaagga caagcaagat attgtcacga cctttttctt 1140

gcttgtctct gtgggtcgct tctgga 1166

<210> 16
<211> 1164
<212> DNA
<213> Brassica napus

<400> 16
ttgacatctg ggtctgagtg atctgcgaga accggccttc acggtcggag aagtcaagaa 60

agcaatccca ccgcactgtt caacctcgat ccctcgctct ttctcctacc tcatctggga 120

catcatcata gcctcctgct tctactacgt cgccaccact tacttccctc tcctccctca 180

ccctctctcc tacttcgcct ggcctctcta ctgggcctgc caaggtgtcg tcctaaccgg 240

cgtctgggtc atagcccacg agtgcggcca ccacgccttc agcgactacc agtggcttga 300

cgacaccgtc ggtctcatct tccactcctt cctcctcgtc ccttacttct cctggaagta 360

cagtcatcga cgccaccatt ccaacactgg ctccctcgag agagacgaag tgtttgtccc 420

caagaagaag tcagacatca agtggtacgg caagtacctc aacaaccctt tgggacgcac 480

cgtgatgtta acggttcagt tcactctcgg ctggccgttg tacttagcct tcaacgtctc 540

gggaagacct tacgacggcg acttcgcttg ccatttccac cccaacgctc ccatctacaa 600

cgaccgcgag cgtctccaga tatacatctc cgacgctggc atcctcgccg tctgctacgg 660

tctcttccgt tacgccgccg cgcaggagtg gcctcgatgg tctgcttcta cggagtcccg 720

cttctgattg tcaatggttt cctcgtgttg atcacttact tgcagcacac gcatccttcc 780

ctgcctcact acgattcgtc cgagtgggat tggttgaagg ggggctttgg ctaccgtgac 840

gaagactacg gaattttgaa caaggtcttc cacaatatta ccgaacaggc acggtggcgg 900

catcatctgt tcctccacga tgcccgcatt atccacggcg aatggagctc taccagggcg 960

ataagtcgaa atcttgggga gagtatatta tccagttcga tgggacgccg gtggttaagg 1020

attgtgaagg aggcagggat gtgtattcta ggggaccgac gcaggttgaa gaagggtggt 1080

tctgtcaaac atagtagaga ggatagtatg tgtagacagg atatttgtac gaactttctt 1140

tgggtcttgg ctgcgtcttg aaga 1164

<210> 17
<211> 1179
<212> DNA

<213>  Brassica napus

<400>  17
cctaaacgtc tcgtttgtga cgatgcgcga gcgggccttc aggtcggaag ctcaagaagg      60

cgatcccacc gcatgttcaa cactcgatcc ctcgctcttt ctcctacctc atctgggaca     120

tcatcatagc ctcctgcttc tactacgtcg ccaccactta cttccctctc ctccctcacc     180

ctctctccta cttcgcctgg cctctctact gggcctgcca agggtgcgtc ctaaccggcg     240

tctgggtcat agcccacgag tgcggccacc acgccttcag cgactaccag tggcttgacg     300

acaccgtcgg tctcatcttc cactccttcc tcctcgtccc ttacttctcc tggaagtaca     360

gtcatcgacg ccaccattcc aacactggct ccctcgagag agacgaagtg tttgtcccca     420

agaagaagtc agacatcaag tggtacggca agtacctcaa caaccctttg ggacgcaccg     480

tgatgttaac ggttcagttc actctcggct ggccgttgta cttagccttc aacgtctcga     540

gaagacctta cgacggcggc ttcgcttgcc atttccaccc caacgctccc atctacaacg     600

accgcgagcg tctccagata tacatctccg acgctggcat cctcgccgtc tgctacggtc     660

tcttccgtta cgccgccgcg cagggagtgg cctcgatggt ctgcttctac ggagtcccgc     720

ttctgattgt caatggtttc ctcgtgttga tcacttactt gcagcacacg catccttccc     780

tgcctcacta cgattcgtcc gagtgggatt ggttgagggg agctttggct accgttgaca     840

gagactacgg aatcttgaac aaggtcttcc acaatattac cgacacgcac gtggcgcatc     900

atctgttctc cacgatgccg cattatcacg cgatggaagc taccaaggcg ataaagccga     960

tactgggaga gtatatcagt tcgatgggac gccggtggtt aaggcgatgt ggagggaggc    1020

gaaggagtgt atctatgtgg aaccggacag gcaaggtgag aagaaaggtg tgttctggta    1080

caacaaatag ttatgagata tgatgatgtg aaggaacaag gaagaaattt gtcacgaacc    1140

ctttcctctt gctgtcctcc tggggtgcgc tcctctgaa                           1179


<210>  18
<211>  1170
<212>  DNA
<213>  Brassica napus

<400>  18
gacggttcat ttaagcgcgt attgtgcgag acaccgccct tcactgtcgg agaactcaag      60

aaagcaatcc caccgcattg ttttaacgct cgatccctcg ctctttctcc tacctcatct     120

gggacatcat catagcctcc tgcttctact acgtcgccac cacttacttc cctctcctcc     180

ctcaccctct ctcctacttc gcctggcctc tctactgggc ctgccaaggg tgcgtcctaa     240

ccggcgtctg ggtcatagcc cacgagtgcg gccaccacgc cttcagcgac taccagtggc     300

ttgacgacac cgtcggtctc atcttccact ccttcctcct cgtcccttac ttctcctgga     360

agtacagtca tcgacgccac cattccaaca ctggctccct cgagagagac gaagtgtttg     420

tccccaagaa gaagtcagac atcaagtggt acggcaagta cctcaacaac cctttgggac 480

gcaccgtgat gttaacggtt cagttcactc tcggctggcc gttgtactta gccttcaacg 540

tctcgggaag accttacgac ggcggcttcg cttgccattt ccaccccaac gctcccatct 600

acaacgaccg cgagcgtctc cagatataca tctccgacgc tggcatcctc gccgtctgct 660

acgatctctt ccgttacgcc gccgcgcagg gagtggcctc gatggtctgc ttctacggag 720

tcccgcttct gattgtcaat ggtttcctcg tgttgatcac ttacttgcag cacacgcatc 780

cttccctgcc tcactacgat tcgtccgagt ggattggttg aggggagctt tggctaccgt 840

tgacagagac tacggaatct tgaacaaagg tcttccacaa tattaccgga cacgcacgtg 900

gcgcatcatc tgttctccac gatgccgcat tatcacgcga tggaagctac caaggcgata 960

aagccgatac tggggagagt attatcagtt cgatgggaac gcggtacagg cgatgtgaag 1020

gaggcgaaag gatgtatcct atgtggaaac cggacgccaa gtgagaagaa tggtgtgttc 1080

ggtacaacaa atagttatga gtattggatg atggtgcaga gatcagagat tggtccgaac 1140

attcctgcct gtctcggtcg cctctggacc 1170

<210> 19
<211> 1205
<212> DNA
<213> Brassica napus

<400> 19
cccctcccgt caggtcgcgt gccctgcgag aaccgccctt cactgtcgga gaactcaaga 60

aagcaatccc agcgggtgtt gcaaggcgcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc aacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt tgaacaaggt cttccacaat attaccgaca cgcacgtggc 900

gcatcattct gttctccacg atgccgcatt atcacgcgat ggaagctacc aaaggcgata 960

aaacccggat actgggagaa gtattattca gttccgatgg gacgctggtg gtttaagggc 1020

gatggtggag ggagtgcgaa aggagtgaat tcttatgttt gaacccggac acagggcaaa 1080

ggtggaagaa agaaaggtgg tgttcttggt tacaacataa gttatggagg aatattgaat 1140

gaatggtgaa agacagagat ttgtacggac gttctctctg ctgtctctgg tgccgctcct 1200

tgtga 1205

<210> 20
<211> 1148
<212> DNA
<213> Brassica napus

<400> 20
ccaacttcag cgcgtaccct gcgagacacc gcccttcact gtcggagaac tcaagaaagc 60

aatcccaccg cactgtttca aacgctcgat ccctcgctct ttctcctacc tcatctggga 120

catcatcata gcctcctgct tctactacgt cgccaccact tacttccctc tcctccctca 180

ccctctctcc tacttcgcct ggcctctcta ctgggcctgc caagggtgcg tcctaaccgg 240

cgtctgggtc atagcccacg agtgcggcca ccacgccttc agcgactacc agtggcttga 300

cgacaccgtc ggtctcatct tccactcctt cctcctcgtc ccttacttct cctggaagta 360

cagtcatcga cgccaccatt ccaacactgg ctccctcgag agagacgaag tgtttgtccc 420

caagaagaag tcagacatca agtggtacgg caagtacctc aacaaccctt tgggacgcac 480

cgtgatgtta acggttcagt tcactctcgg ctggccgttg tacttagcct tcaacgtctc 540

gggaagacct tacgacggcg gcttcgcttg ccatttccac cccaacgctc ccatctacaa 600

cgaccgcgag cgtctccaga tatacatctc cgacgctggc atcctcgtcg tctgctacgg 660

tctcttccgt tacgccgccg cgcagggagt ggcctcgatg gtctgcttct acggagtccc 720

gcttctgatt gtcaatggtt tcctcgtgtt gatcacttac ttgcagcaca cgcatccttc 780

cctgcctcac tacgattcgt ccgagtggga ttggttgagg ggagctttgg ctaccgttga 840

cagagactac ggaatcttga acaaaggtct tccacaatat taccgacacg cacgtggcgc 900

atcatctgtt ctccacgatg ccgcattatc acgcgatgga agctaccaag gcgataaagc 960

cgatactgga gagtattatc agttcgatgg gacgccggtg gttaagcgat gtgaggagcg 1020

tgagtgtatc ttatgtggac cggacagcag tgagaagaaa gtgtgtctgt tacaccaata 1080

gtatgaagat atgatgatgt gagacagaga tttgtcacga accttcctgc gccgggtggc 1140

ccctgtga 1148

<210> 21
<211> 1133

<212>  DNA
<213>  Brassica napus

<400>  21
ccgaacatca gcgcgtacct gcgagacacc gcccttcact gtcggagaac tcaagaaagc      60

aatcccaccg cactgtttca aacgctcgat ccctcgctct ttctcctacc tcatctggga     120

catcatcata gcctcctgct tctactacgt cgccaccact tacttccctc tcctccctca     180

ccctttctcc tacttcgcct ggcctctcta ctgggcctgc caagggtgcg tcctaaccgg     240

cgtctgggtc atagcccacg agtgcggcca ccacgccttc agcgactacc agtggcttga     300

cgacaccgtc ggtctcatct tccactcctt cctcctcgtc ccttacttct cctggaagta     360

cagtcatcga cgccaccatt ccaacactgg ctccctcgag agagacgaag tgtttgtccc     420

caagaagaag tcagacatca agtggtacgg caagtacctc aacaaccctt tgggacgcac     480

cgtgatgtta acggttcagt tcactctcgg ctggccgttg tacttagcct tcaacgtctc     540

gggaagacct tacgacggcg gcttcgcttg ccatttccac cccaacgctc ccatctacaa     600

cgaccgcgag cgtctccaga tatacatctc cgacgctggc atcctcgccg tctgctacgg     660

tctcttccgt tacgccgccg cgcagggagt ggcctcgatg gtctgcttct acggagtccc     720

gcttctgatt gtcaatggtt tcctcgtgtt gatcacttac ttgcagcaca cgcatccttc     780

cctgcctcac tacgattcgt ccgagtggga ttggttgagg ggagctttgg ctaccgttga     840

cagagactac ggaatcttga acaaggtctt ccacaatatt accgacacgc acgtggcgca     900

tcatctgttc tccacgatgc cgcattatca cgcgatggaa gctaccaaag cgataaagcc     960

cgatactggg agagtattta tcagttcgat gggacgccgg tggttaagcg atgtgaggga    1020

gcgaagagtg tatctatgtg accggacagc agtgagaaga agtgtgtctg tacacattag    1080

tatgaagata tgatgatgtg agacagagat ttgtcacgaa ctttctctgc ttc           1133

<210>  22
<211>  1186
<212>  DNA
<213>  Brassica napus

<400>  22
gaccaaaatt cagcgcgtac tctgcgagaa ccgcccttca ctgtcggaga agtcaagaaa      60

gcaatcccac cgcactgttt caaacgctcg atccctcgct ctttctccta cctcatctgg     120

gacatcatca tagcctcctg cttctactac gtcgccacca cttacttccc tctcctccct     180

caccctctct cctacttcgc ctggcctctc tactgggcct gccaagggtg cgtcctaacc     240

ggcgtctggg tcatagccca cgagtgcggc caccacgctt tcagcgacta ccagtagctt     300

gacgacaccg tcggtctcat cttccctctt tcctcctcgt cccttacttc tcctggaagt     360

acagtcatcg acgccaccat tccaacactg gctccctcga gagagacgaa gtgtttgtcc     420

cccagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

cgcttctgat tgtcaatggt ttcctcgtgt tgatcactta cttgcagcac acgcatcctt 780

ccctgcctca ctacgattcg tccgagtggg attgggttga ggggagcttt gggctatcgt 840

tgacagagac tacggaatct tgaacaaggt gtttccacaa tattaccgac acgcacgtgg 900

cgcatcatct gttctccacg atgccgcatt atcacgcgat gggaagctac tcaagcgata 960

aagccgaata ctggggagag tatttattca gttcgatgga cgccgtggtt agcgatggtg 1020

gagggaggcg aggagtgtat cttattgttg gaatgggaca ggtcaaggga gaagacgggt 1080

gtgtttctgg tacaacaatt agctatgagg aatggatgga ttgggtgagg aactatgaga 1140

ttttggttat cgaagctttc tctgagctgt ccatcggtgc cgacta 1186

<210> 23
<211> 1132
<212> DNA
<213> Brassica napus

<400> 23
aggagcgcgc cgtcatcgcg tatctgcgag aaccgccctt cacggtcggg aagtcaagaa 60

agcaatccca ccgcactgtt gcaaggcgcg atcgctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcataaccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaagga gtggcctcga tggtctgctt ctactgagtc 720

ccgcttctga ttgtcaatgg tttcctcgtg ttgatcactt acttgcagca cacgcatcct 780

tccctgcctc actacgaatt cgtccgaatg ggattggttg aggggagctt tggctaccgt 840

tgacagagac tacggaatct tgaacaggtc tttcccaata ttaccgacac gcacgtggcg 900

catcatctgt tctcacgatg cgcattatta cgcgatggaa gctaccaagg cgattaagcg 960

aataactggg ggagtttatt cagtccattg acgccgtcgt ttatgcgatg tgagaggcag 1020

atgtattcta ttgttgacgg acgccaagtg gagagttgtc tggtcacata gttataagaa 1080

tgatgatgta gacagagatt gtacgacctc catgcgacta gtcgtccttg aa 1132

<210> 24
<211> 1186
<212> DNA
<213> Brassica napus

<400> 24
aggtggtcag ccgtcgttgc gagccctgcg agaacggccc ttgcggtcgc ggagtgaagg 60

gaggagccca gcgggtggtg caaggcgggg tcggtcgctc tttctccggc gggaggtggg 120

acatcatcat agcctcctgc ttctactacg tcgccaccac ttacttccct ctcctccctc 180

accctctctc ctacttcgcc tggcctctct actgggcctg ccaagggtgc gtcctaaccg 240

gcgtctgggt catagcccac gagtgcggcc accacgcctt cagcgactac cagtggcttg 300

acgacaccgt cggtctcatc ttccactcct tccttctcgt cccttacttc tcctggaagt 360

acagtcatcg acgccaccat tccaacactg gctccctcga gagagacgaa gtgtttgtcc 420

ccaagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

tgcttctgat tgtcaatggt ttcctcgtgt tgatcactta cttgcagcac acgcatcctt 780

ccctgcctca ctacgattcg tccgagtggg attggttgag gggagctttg gctaccgttg 840

acagagacta cggaatcttg aacaaggtct tccacaatat taccgacacg cacgtggcgc 900

atcatctgtt ctccacgatg ccgcattatc acgcgatggg aagctaccaa ggcgaaaaag 960

ccgatactgg ggagaagtat tatcagttcg atgggacgcc gtggtaagcc gatgtggagg 1020

gaggcgatgg gagtggtatc tatgttgaac cggacaggca ggtgagaaag aaaagggtgg 1080

tggttcttgg ttacaacaat taggtaatgg aggatatgat gatgcttaag aacaagaaat 1140

ttgttcacga accctttctc cttgctgtcc tgggcggcct tgtaaa 1186

<210> 25
<211> 1151
<212> DNA
<213> Brassica napus

<400> 25
ggggacgtca gtcgcgtacc gtgcgagaca ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

gacgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agaaagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaggtc ttccacaata ttaccgacac gcacgtggcg 900

catcatctgt tctccacgat gccgcattat cacgcgatgg aagctaccaa ggcgataaag 960

ccgatactgg gagagtatta tcagttcgat gggacgccgg tggtttaagg cgatgtggag 1020

ggaggcgaag gagtgtatct atgtggaacc ggacaggcaa gtgagaagaa aggtgtgtct 1080

ggtacacata gtatgagata tgatgatgtg aagacaagaa gatttttgtc ccgaaccttc 1140

tctcttgctc c 1151

<210> 26
<211> 1175
<212> DNA
<213> Brassica napus

<400> 26
gccgaggagt gagtcgcgta cgctgcgaga caccgccctt cactgtcgga gaagtgaaga 60

aagcaatccc agcgcactgg gcaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ttcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaggtc ttccacaata ttaccgacac gcacgtggcg 900

catcatctgt tctccacgat gccgcattat cacgcgatgg aagctaccaa ggcgataaag 960

ccgatactgg gagagtatta tcagttcgat gggacgccgg tggttaaggc gatgtggagg 1020

gaggcgaaga gtgtatctat gtggaaccgg aacaggcaag gtgagaagaa aggtgtgtct 1080

ggtaacaaca ataagtatga gatatgatga tgtgaagaac aaagaagatt tgtcacgaac 1140

tttctcttgc tgtcctccgg gggccgcctc tgtaa 1175

<210> 27
<211> 1165
<212> DNA
<213> Brassica napus

<400> 27
tcctgccacg tcgtcgcgta ccctgcgaga accgcccttc actgtcggag aactcaagaa 60

agcaatccca ccgcactgtt tcaaacgctc gatccctcgc tctttctcct acctcatctg 120

ggacatcatc atagcctcct gcttctacta cgtcgccacc acttacttcc ctctcctccc 180

tcaccctctc tcctacttcg cctggcctct ctactgggcc tgccaagggt gcgtcctaac 240

cggcgtctgg gtcatagccc acgagtgcgg ccaccacgcc ttcagcgact accagtggct 300

tgacgacacc gtcggtctca tcttccactc cttcctcctc gtcccttact tctcctggaa 360

gtacagtcat cgacgccacc attccaacac tggctccctc gagagagacg aagtgtttgt 420

ccccaagaag aagtcagaca tcaagtggta cggcaagtac ctcaacaacc ctttgggacg 480

caccgtgatg ttaacggttc agttcactct cggctggccg ttgtacttag ccttcaacgt 540

ctcgggaaga ccttacgacg gcggcttcgc ttgccatttc cactccaacg ctcccatcta 600

caacgaccgc gagcgtctcc agatatacat ctccgacgct ggcatcctcg ccgtctgcta 660

cggtctcttc cgttacgccg ccgcgcaggg agtggcctcg atggtctgct tctacggagt 720

cccgcttctg attgtcaatg gtttcctcgt gttgatcact tacttgcagc acacgcatcc 780

ttccctgcct cactacgatt cgtccgagtg ggattggttg aggggagctt tggctaccgt 840

tgacagagac tacggaatct tgaacaaagg tcttccacaa tattaccgac acgcacgtgg 900

cgcatcatct gttctccacg atgccgcatt atcacgcgat ggaagctacc aaggcgataa 960

agccgatact ggagagtatt atcagttcga tgggacgccg gtggttaagg cgatgtgagg 1020

agcgaaggag tgtatcttat gtggaacgga caggcaaggt gagaagaaag gtggtgtctg 1080

tacaaccaat agtatgagat atgatgatgt gaggacagga gattggtcac ggacctttct 1140

ctgcttctcg tgggcctcct gtgaa 1165

<210> 28
<211> 1175
<212> DNA
<213> Brassica napus

<400> 28
tggagcgaaa gtcagtcgcg taccctgcga gacaccgccc ttcactgtcg gagaactcaa 60

gaaagcaatc ccaccgcact gtttcaaacg ctcgatccct cgctctttct cctacctcat 120

ctgggacatc atcatagcct cctgcttcta ctacgtcgcc accacttact tccctctcct 180

ccctcaccct ctctcctact tcgcctggcc tctctactgg gcctgccaag ggtgcgtcct 240

aaccggcgtc tgggtcatag cccacgagtg cggccaccac gccttcagcg actaccagtg 300

gcttgacgac accgtcggtc tcatcttcca ctccttcctc ctcgtccctt acttctcctg 360

gaagtacagt catcgacgcc accattccaa cactggctcc ctcgagagag acgaagtgtt 420

tgtccccaag aagaagtcag acatcaagtg gtacggcaag tacctcaaca accctttggg 480

acgcaccgtg atgttaacgg ttcagttcac tctcggctgg ccgttgtact tagccttcaa 540

cgtctcggga agaccttacg acggcagctt cgcttgccat ttccacccca acgctcccat 600

ctacaacgac cgcgagcgtc tccagatata catctccgac gctggcatcc tcgccgtctg 660

ctacggtctc ttccgttacg ccgccgcgca gggagtggcc tcgatggtct gcttctacgg 720

agtcccgctt ctgattgtca atggtttcct cgtgttgatc acttacttgc agcacacgca 780

tccttccctg cctcactacg attcgtccga gtgggattgg ttgaggggag ctttggctac 840

cgttgacaga gactacggaa tcttgaacaa ggtcttccac aatattaccg acacgcacgt 900

ggcgcatcat ctgttctcca cgatgccgca ttatcacgcg atggaagcta ccaaggcgat 960

aaagccgata ctgggagagt attatcagtt cgatgggacg ccggggttaa ggcgatgtga 1020

gggaggcgaa ggagtgtatc tatgtggaac cggacaggca agtggagaag aaaagtgtgt 1080

tctggtacaa caataagtat gagatatgat gatggtgaaa ggacaagaga tatgtcacga 1140

acgttcttct gctgtctctg ggcgcgcctc ttgga 1175

<210> 29
<211> 1176
<212> DNA
<213> Brassica napus

<400> 29
gcgaacgtca gtcgcgtacc ctgcgagaca ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacac tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaaggt cttccacaat attaccgaca cgcacgtggc 900

gcatcatctg ttctccacga tgccgcatta tcacgcgatg gaagctacca aggcgataaa 960

gccgatactg ggagagtatt atcagttcga tgggacgccg gtggttaagg cgatgtgagg 1020

gaggcgaagg agtgtatctt atgtggaacc ggacaggcaa ggtgagaaga aggtgtgtct 1080

ggtacaacaa taagtatgag atatggatga tgtgaagaac aaagaagaaa ttgtcacgaa 1140

ctttctcttg ctgtcctcgg tgtgcccgct ctgtga 1176

<210> 30
<211> 1154
<212> DNA
<213> Brassica napus

<400> 30
ggctcttcat cagcgcgtac cctgcgagac accgcccttc actgtcggag aactcaagaa 60

agcaatccca ccgcactgtt tcaaacgctc gatccctcgc tctttctcct acctcatctg 120

ggacatcatc atagcctcct gcttctacta cgtcgccacc acttacttcc ctctcctccc 180

tcaccctctc tcctacttcg cctggcctct ctactgggcc tgccaagggt gcgtcctaac 240

cggcgtctgg gtcatagccc acgagtgcgg ccaccacgcc ttcagcgact accagtggct 300

tgacgacacc gtcggtctca tcttccactc cttcctcctc gtcccttact tctcctggaa 360

gtacagtcat cgacgccacc attccaacac tggctccctc gagagagacg aagtgtttgt 420

ccccaagaag aagtcagaca tcaagtggta cggcaagtac ctcaacaacc ctttgggacg 480

caccgtgatg ttaacggttc agttcactct cggctggccg ttgtacttag ccttcaacgt 540

ctcgggaaga ccttacgacg gcggcttcgc ttgccatttc caccccaacg ctcccatcta 600

caacgaccgc gagcgtctcc agatatacat ctccgacgct ggcatcctcg ccgtctacta 660

cggtctcttc cgttacgccg ccgcgcaggg agtggcctcg atggtctgct tctacggagt 720

cccgcttctg attgtcaatg gtttcctcgt gttgatcact tacttgcagc acacgcatcc 780

ttccctgcct cactacgatt cgtccgagtg ggattggttg aggggagctt tggctaccgt 840

tgacagagac tacggaatct tgaacaaggt cttccacaat attaccgaca cgcacgtgcc 900

gcatcatctg ttctccacga tgccgcatta tcacgcgatg gaagctacca aggcgataag 960

ccgatactgg ggagagtatt tatcagttcg atgggacgcc ggtgataggc gatgtgaggg 1020

aggcgagatg tattctatgt ggaccgacag gcaagtggaa gaggtgtgtc tgtacacata 1080

gtatgagaat gatgatgtga agaacagaag atttgtcacg acttcccttg cgtctcgcgg 1140

cgccctcgtg agaa 1154

<210> 31
<211> 1165
<212> DNA
<213> Brassica napus

<400> 31
gacgcaccgt cagtcgcgta cctgcgagac accgcccttc actgtcggag aagtcaagaa 60

agcaatccca ccgcactgtt tcaaaggctc gatccctcgc tctttctcct acctcatctg 120

ggacatcatc atagcctcct gcttctacta cgtcgccacc acttacttcc ctctcctccc 180

tcaccctctc tcctacttcg cctggcctct ctactgggcc tgccaagggt gcgtcctaac 240

cggcgtctgg gtcatagccc acgagtgcgg ccaccacgcc ttcagcgact accagtggct 300

tgacgacacc gtcggtctca tcttccactc cttcctcctc gtcccttact tctcctggaa 360

gtacagtcat cgacgccacc attccaacac tggctccctc gagagagacg aagtgtttgt 420

ccccaagaag aagtcagaca tcaagtggta cggcaagtac ctcaacaacc ctttgggacg 480

caccgtgatg ttaacggttc agttcactct cggctggccg ttgtacttag ccttcaacgt 540

ctcgggaaga ccttacgacg gcggcttcgc ttgccatttc caccccaacg ctcccatcta 600

caacgaccgc gagcgtctcc agatatacat ctccgacgct ggcatcctcg ccgtctgcta 660

cggtctcttc cgttacgccg ccgcgcaggg agtggcctcg atggtctgct tctacggagt 720

cctgcttctg attgtcaatg gtttcctcgt gttgatcact tacttgcagc acacgcatcc 780

ttccctgcct cactacgatt cgtccgagtg ggattggttg aggggagctt tggctaccgt 840

tgacagagac tacggaatct tgaacaaggt cttccacaat attaccgaca cgcacgtggc 900

gcatcatctg ttctccacga tgccgcatta tcacgcgatg gaagctacca aggcgataaa 960

gccgatactg ggagagtatt atcagttcga tggacgccgg tggtttaagg cgatgtggag 1020

ggaggcgaag agtgtatcta tgtggaaccg gacaggcaag gtgagaagaa aggtgtgttc 1080

tggtacaaca atagtatgag aatgatgatg tgagacagag atttgtcacg aaccttctct 1140

tgctgctctc gggcgcgcct gaaaa 1165

<210> 32
<211> 1163
<212> DNA
<213> Brassica napus

<400> 32
tacgacgatc agtcgcgtac cctgcgagaa ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaaagc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaggtc ttccacaata ttaccgacac gcacgtggcg 900

catcatctgt tctccacgat gccgcattat cacgcgatgg aagctaccaa ggcgataaag 960

ccgatactgg gagagtatta tcagttcgat gggacgccgg tggttaagcg atgtggaggg 1020

aggcgaagag tgtatctatg tggaaccgga caggcaaggt gagaagaacg tgtgtctgta 1080

cacataggta tgagatatga tgatgtgaag acaagagatt gtcacgactt ctctgctgtc 1140

tctggtgcgc cccctctgag gaa 1163

<210> 33
<211> 1173
<212> DNA
<213> Brassica napus

<400> 33
   cacgacggtc gtcgcgtacc ctgcgagaac cgcccttcac tgtcggagaa ctcaagaaag 60
   caatcccacc gcactgtttc aaacgctcga tccctcgctc tttctcctac ctcatctggg 120
   acatcatcat agcctcctgc ttctactacg tcgccaccac ttacttccct ctcctccctc 180
   accctctctc ctacttcgcc tagcctctct actgggcctg ccaagggtgc gtcctaaccg 240
   gcgtctgggt catagcccac gagtgcggcc accacgcctt cagcgactac cagtggcttg 300
   acgacaccgt cggtctcatc ttccactcct tcctcctcgt cccttacttc tcctggaagt 360
   acagtcatcg acgccaccat tccaacactg gctccctcga gagagacgaa gtgtttgtcc 420
   ccaagaagaa gtcagacatc aagtggtacg gcaagtacct caacaaccct ttgggacgca 480
   ccgtgatgtt aacggttcag ttcactctcg gctggccgtt gtacttagcc ttcaacgtct 540
   cgggaagacc ttacgacggc ggcttcgctt gccatttcca ccccaacgct cccatctaca 600
   acgaccgcga gcgtctccag atatacatct ccgacgctgg catcctcgcc gtctgctacg 660
   gtctcttccg ttacgccgcc gcgcagggag tggcctcgat ggtctgcttc tacggagtcc 720
   cgcttctgat tgtcaatggt ttcctcgtgt tgatcactta cttgcagcac acgcatcctt 780
   ccctgcctca ctacgattcg tccgagtggg attggttgag gggagctttg gctaccgttg 840
   acagagacta cggaatcttg aacaaggtct tccacaatat taccgacacg cacgtggcgc 900
   atcatctgtt ctccacgatg ccgcattatc acgcgatggg aagctaccaa aggcgataaa 960
   gccgatactg gagagtatta tcagttcgat gggacgccgg tggttaaggc cgatgtgaag 1020
   ggaggcgaag gagtgtatct tatgtggaaa cggacaggcc aagtgaaaag aaaggtgtgt 1080
   ctggtacaaa caataaagtt atgagatatg atgatgtgaa agaaccaaga gaattgtcac 1140
   gaacctttcc tgctgtcctg gtgcgccctt gaa 1173

<210> 34
<211> 1159
<212> DNA
<213> Brassica napus

<400> 34
gggagcgtca gtcgcgtacc ctgcgagaca ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca tttcaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaggtc ttccacaata taccgacacg cacgtggcgc 900

atcatctgtt ctcacgatgc cgcattatca cgcgatggaa gctaccaagg cgataaagcg 960

atactgggga ggagtattat tcagttcgat gggacgccgg tggttaagcg atgtggaggg 1020

aggcgaagat gtatctatgt gacggacagg ccagtggaga agaaggtgtg ttctgttaca 1080

accataggta tgagattgat gatgtgaaga caagaagaat ttggtaccga ccttcctgct 1140

gtctggctgg ggtcttgga 1159

<210> 35
<211> 1165
<212> DNA
<213> Brassica napus

<400> 35
ccccacgtca gtcgcgtacc ctgcgagaac cgcccttcac tgtcggagaa ctcaagaaag 60

caatcccacc gcactgtttc aaacgctcga tccctcgctc tttctcctac ctcatctggg 120

acatcatcat agcctcctgc ttctactacg tcgccaccac ttacttccct ctcctccctc 180

accctctctc ctacttcgcc tggcctctct actgggcctg ccaagggtgc gtcctaaccg 240

gcgtctgggt catagcccac gagtgcggcc accacgcctt cagcgactac cagtggcttg 300

acgacaccgt cggtctcatc ttccactcct tcctcctcgt ccccttactc tcctggaagt 360

acagtcatcg acgccaccat tccaacactg gctccctcga gagagacgaa gtgtttgtcc 420

ccaagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

cgcttctgat tgtcaatggt ttcctcgtgt tgatcactta cttgcagcac acgcatcctt 780

ccctgcctca ctacgattcg tccgagtggg attggttgag gggagctttg gctatcgttg 840

acagagacta cggaatcttg aacaaggtct tccacaatat taccgacacg cacgtggcgc 900

atcatctgtt ctccacgatg ccgcattatc acgcgatgga agctaccaag gcgataaagc 960

cgatactggg agagtattat cagttcgatg ggacgccggt ggttaagcga tgtggaggga 1020

ggcgaagagt gtatctatgt ggaaccggac aggcaagtga gaagaaagtg tgtctggtac 1080

aacaattagt atgagatatg atgatgtgag accagagatc tgtcacgaac tttctctgct 1140

gtctctcgtg cgcgctctcg tggaa 1165

<210> 36
<211> 1183
<212> DNA
<213> Brassica napus

<400> 36
gcagacggtg cagtcgcgta ttgtgcgaga caccgccctt cactgtcgga gaactcaaga 60

aagcaatccc accgcactgt ttcaaacgct cgatccctcg ctctttctcc tacctcatct 120

gggacatcat catagcctcc tgcttctact acgtcgccac cacttacttc cctctcctcc 180

ctcaccctct ctcctacttc gcctggcctc tctactgggc ctgccaaggg tgcgtcctaa 240

ccggcgtctg ggtcatagcc cacgagtgcg gccactacgc cttcagcgac taccagtggc 300

ttgacgacac cgtcggtctc atcttccact ccttcctcct cgtcccttac ttctcctgga 360

agtacagtca tcgacgccac cattccaaca ctggctccct cgagagagac gaagtgtttg 420

tccccaagaa gaagtcagac atcaagtggt acggcaagta cctcaacaac cctttgggac 480

gcaccgtgat gttaacggtt cagttcactc tcggctggcc gttgtactta gccttcaacg 540

tctcgggaag accttacgac ggcggcttcg cttgccattt ccaccccaac gctcccatct 600

acaacgaccg cgagcgtctc cagatataca tctccgacgc tggcatcctc gccgtctgct 660

acggtctctt ccgttacgcc gccgcgcagg gagtggcctc gatggtctgc ttctacggag 720

tcccgcttct gattgtcaat ggtttcctcg tgttgatcac ttacttgcag cacacgcatc 780

cttccctgcc tcactacgat tcgtccgagt gggattggtt gaggggagct ttggctaccg 840

ttgacagaga ctacggaatc ttgaaaaggg tcttccacat attaccgaca cgcacgtggc 900

gcatcatctg ttctccacga tgccgcatta tcacgcgatg gaagctacca ggcgataaag 960

ccgatactgg ggagagtatt tatcagttcg atgggacgcc ggtggttagg cgatgtggag 1020

ggaggcgagg agtgtattct atggtggacc cggacagcag ggtgagaaga aaggttgtgt 1080

gttctggtac acaataaggt tatgaagata tggatgatgg tgagaccagg agatttgttc 1140

aagactttct cccttgccgt ctcggtgcgc ctctctgttg gaa 1183

<210> 37
<211> 1215
<212> DNA
<213> Brassica napus

<400> 37
cgacacggca gtcgagagct ctgggagaac cgcccttcac ggtcggagag tgaagaaagg 60

atcccaccgg ggtggcgcaa gggggggtcg ctcgctcttt ctcctgcggg aggtggaaca 120

tcatcatagc ctcctgcttc tactacgtcg ccaccactta cttccctctc ctccctcacc 180

ctctctccta cttcgcctgg cctctctact gggcctgcca agggtgcgtc ctaaccggcg 240

tctgggtcat agcccacgag tgcggccacc acgccttcag cgactaccag tggcttgacg 300

acaccgtcgg tctcatcttc cactccttcc tcctcgtccc ttacttctcc tggaagtaca 360

gtcatcgacg ccaccattcc aacactggct ccctcgagag agacgaagtg tttgtcccca 420

agaagaagtc agacatcaag tggtacggca agtacctcaa caaccctttg ggacgcaccg 480

tgatgttaac ggttcagttc actctcggct ggccgttgta cttagccttc aacgtctcgg 540

gaagacctta cgacggcggc ttcgcttgcc atttccaccc caacgctccc atctacaacg 600

accgcgagcg tctccagata tacatctccg acgctggcat cctcgccgtc tgctacggtc 660

tcttccgtta cgccgccgcg caggaagtgg cctcgatggt ctgcttctac ggagtcccgc 720

ttctgatttg tcaatggttt cctcgtgttg atcacttact ttgcagcaca cgcatccttc 780

cctgccctca ctacgattcg tccgagtggg attggttgag gggagctttg gctaccgttt 840

gacagagact acggaatctt tgaacaaggt ctttccacaa tattaccgac acgcacgtgg 900

cgcatcatct gttctccacg atgccgcatt atcacgcgat ggaagctacc aaggcgataa 960

agccgatact ggcagagtat tatcaatttc gatgggacgc cggtgggtta cggcgatgtt 1020

gcatggaggc cgaggagtgt aatcttattg tggaatccgg tacagggcaa gtggagaaga 1080

aaggtgttgt tctggtacac caataagtta ttgaggatat tgatgatatg ggtgtaggaa 1140

ccaaacgaag aaattttgtt caccgatacc ttttctctct tggcctgtct ccctcggtct 1200

cgtcctctgg aacta 1215

<210> 38
<211> 1179
<212> DNA
<213> Brassica napus

<400> 38
tgcccgcgtc agcgcgtacc ctgcgagaac cgcccttcac tgtcggagaa ctcaagaaag 60

caatcccacc gcactgtttc aaacgctcga tccctcgctc tttctcctac ctcatctggg 120

acatcatcat agcctcctgc ttctactacg tcgccaccac ttacttcctt ctcctccctc 180

accctctctc ctacttcgcc tggcctctct actgggcctg ccaagggtgc gtcctaaccg 240

gcgtctgggt catagcccac gagtgcggcc accacgcctt cagcgactac cagtggcttg 300

acgacaccgt cggtctcatc ttccactcct tcctcctcgt cccttacttc tcctggaagt 360

acagtcatcg acgccaccat tccaacactg gctccctcga gagagacgaa gtgtttgtcc 420

ccaagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

cgcttctgat tgtcaatggt ttcctcgtgt tgatcactta cttgcagcac acgcatcctt 780

ccctgcctca ctacgattcg tccgagtggg attggttgag gggagctttg gctaccgttg 840

acagagacta cggaatcttg aacaaggtct tccacaatat taccgacacg cacgtggcgc 900

atcatctgtt ctccacgatg ccgcattatc acgcgatgga agctaccaag gcgataaagc 960

cgatactggg agagtattat cagttcgatg ggacgccggt ggttaaaggc gatgtgtagg 1020

gaggcgaaga gtgtatctat gtgggaaccg gacaggcagg tgagaagaaa ggtggtgtct 1080

ggtacacata gttatgagga tatggatgat ggtgaaagac aagagattct gtcaacgatc 1140

atttctgtct tgactgtcct ggggcccctc ttgtggaaa 1179

<210> 39
<211> 1179
<212> DNA
<213> Brassica napus

<400> 39
ccgcacgtca gtcgcgtacc gtgcgagaca ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgagcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaggtc ttccacaata ttaccgacac gcacgtggcg 900

catcatctgt tctccacgat gccgcattat cacgcgatgg aaagctacca aggcgataaa 960

gccgatactg ggagagtatt atcagttcga tgggacgccc ggtggtttaa ggcgatgtga 1020

gggaggcgaa ggagtgtatc tatgtggcac cggacaggca aggtggagaa gaaaggtgtg 1080

tctggtacaa caataagtta tgaagatatg atgatgttga aagacaagga ggatatgtca 1140

cgatcttctc tgcgttctcg gggggccctc ttctgtgaa 1179

<210> 40
<211> 1215
<212> DNA
<213> Brassica napus

<400> 40
tattcatcct cggctctgat gtgcttgaga gaacgggcct tgcggtcggg aagtcaagaa 60

agcaatccca ccggggtggt caacactcga tccctcgctc tttctcctac ctcatctggg 120

acatcatcat agcctcctgc ttctactacg tcgccaccac ttacttccct ctcctccctc 180

accctctctc ctacttcgcc tggcctctct actgggcctg ccaagggtgc gtcctaaccg 240

gcgtctgggt catagcccac aagtgcggcc accacgcctt cagcgactac cagtggcttg 300

acgacaccgt cggtctcatc ttccactcct tcctcctcgt cccttacttc tcctggaagt 360

acagtcatcg acgccaccat tccaacactg gctccctcga gagagacgaa gtgtttgtcc 420

ccaagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

cgcttctgat tgtcaatggt ttcctcgtgt tgatcactta cttgcagcac acgcatcctt 780

ccctgcctca ctacgatttc gtccgagtgg gattggttga ggggagcttt ggctaccgtt 840

gacagagact acggaatctt tgaacaaggt ctttccacaa tattaccgac acgcacgtgg 900

cgcatcatct gttctccacg atgccgcatt atcacgcgat gggaagctac caagggcgat 960

aaagccgata ctgggagagt attatcagtt tcgatgggac gccgggtggt taaaggcgat 1020

gtggagggag gcggaaggag ttgttattct tattgttgga actcgggaca ggccaaggtg 1080

gagaaagaaa gggtgtgttt ctggttacaa caattaaagt taatggaggg atatgaatga 1140

ttgctgaaag aacaaggaga tatttgttca cgaacctttc tctctttgct gtctctttgg 1200

gtgcgctctc ttgaa 1215

<210> 41
<211> 1136
<212> DNA
<213> Brassica napus

<400> 41
gagttacatt cagtcgcgta ttgagcgaga caccgccctt cactgtcgga gaactcaaga 60

aagcaatccc accgcactgt ttcaaacgct cgatccctcg ctctttctcc tacctcatct 120

gggacatcat catagcctcc tgcttctact acgtcgccac cacttacttc cctctcctcc 180

ctcaccctct ctcctacttc gcctggcctc tctactgggc ctgccaaggg tgcgtcctaa 240

ccggcgtctg ggtcatagcc cacgagtgcg gccaccacgc cttcagcgac taccagtggc 300

ttgacgacac cgtcagtctc atcttccact ccttcctcct cgtcccttac ttctcctgga 360

agtacagtca tcgacgccac cattccaaca ctggctccct cgagagagac gaagtgtttg 420

tccccaagaa gaagtcagac atcaagtggt acggcaagta cctcaacaac cctttgggac 480

gcaccgtgat gttaacggtt cagttcactc tcggctggcc gttgtactta gccttcaacg 540

tctcgggaag accttacgac ggcggcttcg cttgccattt ccaccccaac gctcccatct 600

acaacgaccg cgagcgtctc cagatataca tctccgacgc tggcatcctc gccgtctgct 660

acggtctctt ccgttacgcc gccgcgcagg gagtggcctc gatggtctgc ttctacggag 720

tcccgcttct gattgtcaat ggtttcctcg tgttgatcac ttacttgcag cacacgcatc 780

cttccctgcc tcactacgat tcgtccgagt gggattggtt gaggggagct ttggctaccg 840

ttgacagaga ctacggaatc ttgaacaagg tcttccacat attaccgaca cgcacgtggc 900

gcatcatctg tctccacgat gccgcattat cacgcgatgg aagctaccag gcgataaagc 960

cgatactggg gaggagtatt atcagtcgat ggacgccgtg tagcgatgtg gagggaggcg 1020

acggatgtat ctatgtgacc gacagcaggt gagaagaagg tgtgtctgta cacattaggt 1080

tatgaagaat gatgatggtg aggacaggag gattttgtca cgactttcct tgcgtc 1136

<210> 42
<211> 1102
<212> DNA
<213> Brassica napus

<400> 42
ggcggacaat cagtcgcgta ccctgcgaga caccgccctt cactgtcgga gaactcaaga 60

aagcaatcca gcggctgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatat taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggag tggcctcgat ggtctgctct acggagtccc 720

gcttctgatt gtcaatggtt tcctcgtgtg atcactactg cagcaacacg catctccctg 780

cctcactacg atcgtccgag tggatggtgg agggggagct tggctaccgt tgacaggaga 840

actacggatt cttgaacaag gtcttccacc aataattacc gaacaccgcc aacgtgggcc 900

catcacatcg ttctccccac gaatggcccg cattaatcac gcgattggag ccttccccag 960

gggataaagc gcgatatctc tggggaggat tatatccagt ccaattggga atccggttta 1020

gccaatgtga aggaaggcca agagttttcc tattgggaac ggaacggctg tgagaaaagg 1080

tgttcgtcac acataagctt tg 1102

<210> 43
<211> 1148
<212> DNA
<213> Brassica napus

<400> 43
gacggcagtc agtcgcgtac cctgcgagac accgcccttc actgtcggag aactcaagaa 60

agcaatccag cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagttatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggag tggccttcga tggtctgctt ctacggagtc 720

ccgcttctga ttgtcaatgg tttcctcgtg ttgatcactt acttgcagca cacgcatcct 780

tccctgcctc actacgattc gttcgaggtg gggattggtt gagggggagc tttggcctac 840

cgttgaacag agagacttac gggaattctt gaacaaggtt cttccaacaa ttatttaccg 900

acacgcacgg tgggccgcat tcatcctgtt tctctccacg atggcccgca attattcacg 960

cgcgttggaa agcctaacaa cggcgataaa aggccggata ccttggggag agatatataa 1020

tcagtttcca aatgggaacg ccggtgtgtt taggcaattg ttgagggggg caaagatgtt 1080

attctaatgt gtgaaccgac atggcaaggt gtggaaaaac gtgtgttccg tcaacaataa 1140

gtttagac                                                          1148

<210>  44
<211>  1183
<212>  DNA
<213>  Brassica napus

<400>  44
ccgaacatca gcgcgtaccc tgcgagacac cgcccttcac tgtcggagaa ctcaagaaag      60

caatcccacg cactgtttca aacgctcgat ccctcgctct ttctcctacc tcatctggga     120

catcatcata gcctcctgct tctactacgt cgccaccact tacttccctc tcctccctca     180

ccctctctcc tacttcgcct ggcctctcta ctgggcctgc caagggtacg tcctaaccgg     240

cgtctgggtc atagcccacg agtgcggcca ccacgccttc agcgactacc agtggcttga     300

cgacaccgtc ggtctcatct tccactcctt cctcctcgtc ccttacttct cctggaagta     360

cagtcatcga cgccaccatt ccaacactgg ctccctcgag agagacgaag tgtttgtccc     420

caagaagaag tcagacatca agtggtacgg caagtacctc aacaaccctt tgggacgcac     480

cgtgatgtta acggttcagt tcactctcgg ctggccgttg tacttagcct tcaacgtctc     540

gggaagacct tacgacggcg gcttcgcttg ccatttccac cccaacgctc ccatctacaa     600

cgaccgcgag cgtctccaga tatacatctc cgacgctggc atcctcgccg tctgctacgg     660

tctcttccgt tacgccgccg cgcagggagt ggcctcgatg gtctgcttct acggagtccc     720

gcttctgatt gtcaatggtt tcctcgtgtt gatcacttac ttgcagcaca cgcatccttc     780

cctgcctcac tacgattcgt ccgagtggga ttggttgagg ggagctttgg ctaccgttga     840

cagagactac ggaatctgac aaggtcttcc acaatattac cgacacgcca cgtggcgcat     900

catctgttcc tcacgatgcc ggcattatcc acgcgatgga agcttaccaa ggcgaataaa     960

gccggataac tgggatgagt aattatcagt tcgaatggga accgcccgtt gttaaggcat    1020

tgtgcagggt aggcgaagga atgtatctat gtgtaacctg gacaggctag tggagaaaaa    1080

ggggtgttct ggtacacaaa agatagaggc ttcgagatgg ctcacaacac ctacaatctg    1140

tgactcgatc ctctctatgc agtaccctgg ctgtcctctg gat                      1183

<210>  45
<211>  1166
<212>  DNA
<213>  Brassica napus

<400>  45
attcgggcgt tcaagcgcgt accctgcgag acaccgccct tcactgtcgg agaactcaag      60

aaagcaatcc caccgcactg tttcaaacgc tcgatccctc gctctttctc ctacctcatc     120

tgggacatca tcatagcctc ctgcttctac tacgtcgcca ccacttactt ccctctcctc     180

cctcaccctc tctcctactt cgcctggcct ctctactggg cctgccaagg gtgcgtccta 240

accggcgtct gggtcatagc ccacgagtgc ggccaccacg ccttcagcga ctaccagtgg 300

cttgacgaca ccgtcggtct catcttccac tccttcctcc tcgtccctta cttctcctgg 360

aagtacagtc atcgacgcca ccattccaac actggctccc tcgagagaga cgaagtgttt 420

gtccccaaga agaagtcaga catcaagtgg tacggcaagt acctcaacaa ccctttggga 480

cgcaccgtga tgttaacggt tcagttcact ctcggctgac cgttgtactt agccttcaac 540

gtctcgggaa gaccttacga cggcggcttc gcttgccatt tccaccccaa cgctcccatc 600

tacaacgacc gcgagcgtct ccagatatac atctccgacg ctggcatcct cgccgtctgc 660

tacggtctct tccgttacgc cgccgcgcag ggagtggcct cgatggtctg cttctacgga 720

gtcccgcttc tgattgtcaa tggtttcctc gtgttgatca cttacttgca gcacacgcat 780

ccttccctgc ctcactacga ttcgtccgag tgggattggt tgaggggagc tttggctacc 840

gttgacagag actacggaat cttgaacaag gtcttccaca atattaccga cacgcacgtg 900

gcgcatcatc tgttctccac gatgccgcat tatcacgcga tggaagctac caaggcgata 960

aagccgatac tggggaggag ttattatcag ttcgatggga cgccggtggt agcgatgtgg 1020

agggaggcga tatgtattct attgtgaccg gacagcaggt gagagaaggt ggtgttctgt 1080

acacattagt atgaagattg atgatgtgaa gacaagaagg atttgtcacg acttccttgc 1140

gtcctggtgg tggtgcctct gtggaa 1166

<210> 46
<211> 1152
<212> DNA
<213> Brassica napus

<400> 46
ggcgaacatc agcgcgtacc ctgcgagaca ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgtt tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga gggagctttg gctaccgttg 840

acagagacta cggaatcttg aacaaggtct tccacatatt accgacacgc acggtggcgc 900

atcatcttgt tcttcccgat gccgcattat cacgcgatgg aagctaccaa ggcgataaag 960

ccgatactgg gaagagttat ttattcagtt cgatgggacg ccggtggtta aggcgattgt 1020

ggagggaggc gaggagtgtt attcttatgg tggaccgaca gcaggtgaga agaagggtgt 1080

tgtttctgta cacaatagtt atgaagaatg atgatgggtc agacagagat ttgttcacgg 1140

actttccttg cg 1152

<210> 47
<211> 1149
<212> DNA
<213> Brassica napus

<400> 47
gagaaaaatc agcgcgtacc ctgcgagaca ccgcccttca ctgtcggaga actcaagaaa 60

gcaatcccac cgcatgtttc aaacgctcga tccctcgctc tttctcctac ctcatctggg 120

acatcatcat agcctcctgc ttctactacg tcgccaccac ttacttccct ctcctccctc 180

accctctctc ctacttcgcc tggcctctct actgggcctg ccaagggtgc gtcctaaccg 240

gcgtctgggt catagcccac gagtgcggcc accacgcctt cagcgactac cagtggcttg 300

acgacaccgt cggtctcatc ttccactcct tcctcctcgt cccttacttc tcctggaagt 360

acagtcatcg acgccaccat tccaacactg gctccctcga gagagacgaa gtgtttgtcc 420

ccaagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

cgcttctgat tgtcaatggt ttcctcgtgt tgatcactta cttgcagcac acgcatcctt 780

ccctgcctca ctacgattcg tccgagtggg attggttgag ggagctttgg ctaccgttga 840

cagagactac ggaatcttga acaagtcttc cacaatatta ccgacacgca cgtggcgcat 900

catctgttct ccacgatgcc gcattatcca cgcgatggaa gctaccaagg cgaataaagc 960

cgatactggg agagtattat cagttcgatg ggacgccggt ggttaaggcg atgtggagga 1020

gcgacgatgt atctattgtg accgaatcag gtgagaagaa ggtgtgttct gttacacata 1080

gtatgaagaa ttgatgatgg tgaggaccag agtttgttcc gaacttccct gcgtccgggg 1140

ctccttgga 1149

<210> 48
<211> 1170
<212> DNA
<213> Brassica napus

<400> 48
cccgttcatt cagcgcgtac cctgcgagac accgcccttc actgtcggag aactcaagaa 60

agcaatccca ccgcatgttt caacgctcga tccctcgctc tttctcctac ctcatctggg 120

acatcatcat agcctcctgc ttctactacg tcgccaccac ttacttccct ctcctccctc 180

accctctctc ctacttcgcc tggcctctct actgggcctg ccaagggtgc gtcctaaccg 240

gcgtctgggt catagcccac gagtgcggcc accacgcctt cagcgactac cagtggcttg 300

acgacaccgt cggtctcatc ttccactcct tcctcctcgt cccttacttc tcctggaagt 360

acagtcatcg acaccaccat tccaacactg gctccctcga gagagacgaa gtgtttgtcc 420

ccaagaagaa gtcagacatc aagtggtacg gcaagtacct caacaaccct ttgggacgca 480

ccgtgatgtt aacggttcag ttcactctcg gctggccgtt gtacttagcc ttcaacgtct 540

cgggaagacc ttacgacggc ggcttcgctt gccatttcca ccccaacgct cccatctaca 600

acgaccgcga gcgtctccag atatacatct ccgacgctgg catcctcgcc gtctgctacg 660

gtctcttccg ttacgccgcc gcgcagggag tggcctcgat ggtctgcttc tacggagtcc 720

cgcttctgat tgtcaatggt ttcctcgtgt tgatcactta cttgcagcac acgcatcctt 780

ccctgcctca ctacgattcg tccgagtggg attggttgag ggagctttgg ctaccgttga 840

cagagactac ggaatcttga acaagtcttc cacaatatta ccgacacgca cgtggcgcat 900

catctgttct cacgatgccg cattatcacg cgatggaaag ctaccaaggc gataaagccg 960

atactgggga gagtattatc agttcgatgg gacgccggtg gttaggcgat ggtggaggga 1020

ggcgaaggat gtattctatg gtggacggac aggcaggtga gaagaaggtg tgtgttctgt 1080

tacaaacata gtatgagaaa tgatgattgt gagaccaaga gatttgtcac cgaacctttt 1140

ccttgcgtca tcgggtgggt ccttctgaga 1170

<210> 49
<211> 1168
<212> DNA
<213> Brassica napus

<400> 49
gcgtacagtc agcgcgtacc ctgcgagaca ccgcccttca ctgtcggaga actcaagaaa 60

gcaatcccac cgcaatgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcagc caccacgcct tcagcgacta ccagtggctt 300

gacgacaccg tcggtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaggtc ttccacaata ttaccgacac gcacgtggcg 900

catcatcttg ttctccacga tgccgcatta tcacgcgatg gaagctacca aggcgataaa 960

gccgatactg gggaggagta ttatcagttc gatgggacgc cggtggttaa ggcgatgtga 1020

gggaggcgag atgtatctat gtggaccgga caggcaggtg agaagaaggt gttgttctgt 1080

acaacatagt tatgaagata tgatgatggg tgaagaacag aagatttgtc acgatcgttc 1140

cttgcgcttg ggtggtgccc tggtgaga 1168

<210> 50
<211> 1149
<212> DNA
<213> Brassica napus

<400> 50
cccaaactgg cagtcgcgta ccctgcgaga caccgccttt cactgtcgga gaactcaaga 60

aagcaatccc accgcactgt ttcaaacgct cgatccctcg ctctttctcc tacctcatct 120

gggacatcat catagcctcc tgcttctact acgtcgccac cacttacttc cctctcctcc 180

ctcaccctct ctcctacttc gcctggcctc tctactgggc ctgccaaggg tgcgtcctaa 240

ccggcgtctg ggtcatagcc cacgagtgcg gccaccacgc cttcagcgac taccagtggc 300

ttgacgacac cgtcggtctc atcttccact ccttcctcct cgtcccttac ttctcctgga 360

agtacagtca tcgacgccac cattccaaca ctggctccct cgagagagac gaagtgtttg 420

tccccaagaa gaagtcagac atcaagtggt acggcaagta cctcaacaac cctttgggac 480

gcaccgtgat gttaacggtt cagttcactc tcgactggcc gttgtactta gccttcaacg 540

tctcgggaag accttacgac ggcggcttcg cttgccattt ccaccccaac gctcccatct 600

acaacgaccg cgagcgtctc cagatataca tctccgacgc tggcatcctc gccgtctgct 660

acggtctctt ccgttacgcc gccgcgcagg gagtggcctc gatggtctgc ttctacggag 720

tcccgcttct gattgtcaat ggtttcctcg tgttgatcac ttacttgcag cacacgcatc 780

cttccctgcc tcactacgat tcgtccgagt gggattggtt gaggggagct ttggctaccg 840

ttgacagaga ctacggaatc ttgaacaagg tcttccacaa tattaccgac acgcacgtgg 900

cgcatcatct gttctcacga tgccgcatta tcacgcgatg gaagctacca aggcgataaa 960

gccgatactg ggagagtatt atcagttcga tgggacgcgg tggctatgcg atgtgaggga 1020

ggcgacgatg tatctatgtg aacggacagc cagtgagaag aacgtgtgtt ctgttacaac 1080

atagtatgaa gaaatgatga tgtgaagaca gagattgtca cgacttcctg cgtcttgggt 1140

gctcctgga 1149

<210> 51
<211> 1178
<212> DNA
<213> Brassica napus

<400> 51
gcccgcgttc agtcgcgtac ctgcgagaca ccgcccttca ctgtcggaga actcaagaaa 60

gcaatcccac cgcactgttt caaacgctcg atccctcgct ctttctccta cctcatctgg 120

gacatcatca tagcctcctg cttctactac gtcgccacca cttacttccc tctcctccct 180

caccctctct cctacttcgc ctggcctctc tactgggcct gccaagggtg cgtcctaacc 240

ggcgtctggg tcatagccca cgagtgcggc caccacgcct tcagcgacta ccagtggctt 300

gacgacaccg tcagtctcat cttccactcc ttcctcctcg tcccttactt ctcctggaag 360

tacagtcatc gacgccacca ttccaacact ggctccctcg agagagacga agtgtttgtc 420

cccaagaaga agtcagacat caagtggtac ggcaagtacc tcaacaaccc tttgggacgc 480

accgtgatgt taacggttca gttcactctc ggctggccgt tgtacttagc cttcaacgtc 540

tcgggaagac cttacgacgg cggcttcgct tgccatttcc accccaacgc tcccatctac 600

aacgaccgcg agcgtctcca gatatacatc tccgacgctg gcatcctcgc cgtctgctac 660

ggtctcttcc gttacgccgc cgcgcaggga gtggcctcga tggtctgctt ctacggagtc 720

ccgcttctga ttgtcaatgg tttcctcgtg ttgatcactt acttgcagca cacgcatcct 780

tccctgcctc actacgattc gtccgagtgg gattggttga ggggagcttt ggctaccgtt 840

gacagagact acggaatctt gaacaaaggt cttccacaat attaccgaca cgcacgtggc 900

gcatcatctg ttctccacga tgccgcatta tcacgcgatg gaagctacca aggcgataaa 960

gccgatactg ggagagtatt atcagttcga tgggacgccg gtggttaagc gatgtggagg 1020

gaggcgaagg agtgtatcta tgtggaaacc ggaacagcag tgagaagaac cgtggtgttc 1080

tgtaacaaca atagtatgag gatatgatga tgggtgaaag acaagaagat atgtcacgat 1140

ccctttctct gctgtcgtgc gtgggtggct ctctgtga 1178

<210>  52
<211>  20
<212>  DNA
<213>  Artificial Sequence

<220>
<223>  Nucleic acid sequence fragment

<400>  52
   gtctcctccc tccaaaaagt                                                    20


<210>  53
<211>  18
<212>  DNA
<213>  Artificial Sequence

<220>
<223>  Nucleic acid sequence fragment

<400>  53
gtgtctcctc cctccaaa                                                     18


<210>  54
<211>  20
<212>  DNA
<213>  Artificial Sequence

<220>
<223>  Nucleic acid sequence fragment

<400>  54
ctacagaaac aaacatgggc                                                   20


<210>  55
<211>  18
<212>  DNA
<213>  Artificial Sequence

<220>
<223>  Nucleic acid sequence fragment

<400>  55
gtctctcctc cctccagc                                                     18


<210>  56
<211>  20
<212>  DNA
<213>  Artificial Sequence

<220>
<223>  Nucleic acid sequence fragment

<400>  56
ctctcctccc tccagctccc                                                   20


<210>  57

<211>  20
<212>  DNA
<213>  Artificial Sequence

<220>
<223>  Nucleic acid sequence fragment

<400>  57
ctcttcgaca tcctcctctc                                                    20


<210>  58
<211>  21
<212>  DNA
<213>  Artificial Sequence

<220>
<223>  Nucleic acid sequence fragment

<400>  58
cctcgtccct tacttctcct g                                                  21


<210>  59
<211>  24
<212>  DNA
<213>  Artificial Sequence

<220>
<223>  Nucleic acid sequence fragment

<400>  59
cctcataact tattgttgta ccag                                               24


<210>  60
<211>  19
<212>  DNA
<213>  Artificial Sequence

<220>
<223>  Nucleic acid sequence fragment

<400>  60
caagacgacc agagacagc                                                     19


<210>  61
<211>  20
<212>  DNA
<213>  Artificial Sequence

<220>
<223>  Nucleic acid sequence fragment

<400>  61
gaactcgaca aatttgcctg                                                    20


<210>  62
<211>  19
<212>  DNA
<213>  Artificial Sequence

<220>
<223>  Nucleic acid sequence fragment probe f

<400>  62
gggtgcaggt ggaagaatg                                                    19


<210>  63
<211>  20
<212>  DNA
<213>  Artificial Sequence

<220>
<223>  Nucleic acid sequence fragment probe r

<400>  63
ttgttgtacc agtacacacc                                                   20


<210>  64
<211>  22
<212>  DNA
<213>  Artificial Sequence

<220>
<223>  Nucleic acid sequence fragment conserved f

<400>  64
gagggaggcg aaggagtgta tc                                                22


<210>  65
<211>  21
<212>  DNA
<213>  Artificial Sequence

<220>
<223>  Nucleic acid sequence fragment conserved r

<400>  65
caggagaagt aagggacgag g                                                 21


<210>  66
<211>  22
<212>  DNA
<213>  Artificial Sequence

<220>
<223>  Nucleic acid sequence fragment degenerate f


<220>
<221>  misc_feature
<222>  (11)..(11)
<223>  n is a, c, g, or t

<220>
<221>  misc_feature
<222>  (14)..(14)
<223>  n is a, c, g, or t

<220>
<221>  misc_feature
<222>  (17)..(17)
<223>  n is a, c, g, or t

<220>
<221>  misc_feature
<222>  (20)..(20)
<223>  n is a, c, g, or t

<400>  66
attccttcct nctnctngtn cc                                                22


<210>  67
<211>  20
<212>  DNA
<213>  Artificial Sequence

<220>
<223>  Nucleic acid sequence fragment degenerate r


<220>
<221>  misc_feature
<222>  (12)..(12)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<400>  67
gctaagtaca anggncancc                                                   20


<210>  68
<211>  1162
<212>  DNA
<213>  Brassica napus

<400>  68
ggccgtaggt cggtcgcgta ccctgcgaga caccgccctt cactgtcgga gaactcaaga      60

aagcaatccc accgcactgt ttcaaacgct cgatccctcg ctctttctcc tacctcatct     120

gggacatcat catagcctcc tgcttctact acgtcgccac cacttacttc cctctcctcc     180

ctcaccctct ctcctacttc gcctgacctc tctactgggc ctgccaaggg tgcgtcctaa     240

ccggcgtctg ggtcatagcc cacgagtgcg gccaccacgc cttcagcgac taccagtggc     300

ttgacgacac cgtcggtctc atcttccact ccttcctcct cgtcccttac ttctcctgga     360

agtacagtca tcgacgccac cattccaaca ctggctccct cgagagagac gaagtgtttg     420

tccccaagaa gaagtcagac atcaagtggt acggcaagta cctcaacaac cctttgggac     480

gcaccgtgat gttaacggtt cagttcactc tcggctggcc gttgtactta gccttcaacg 540

tctcgggaag accttacgac ggcggcttcg cttgccattt ccaccccaac gctcccatct 600

acaacgaccg cgagcgtctc cagatataca tctccgacgc tggcatcctc gccgtctgct 660

acggtctctt ccgttacgcc gccgcgcagg gagtggcctc gatggtctgc ttctacggag 720

tcccgcttct gattgtcaat ggtttcctcg tgttgatcac ttacttgcag cacacgcatc 780

cttccctgcc tcactacgat tcgtccgagt gggattggtt gaggggagct ttggctaccg 840

ttgacagaga ctacggaatc ttgaacaagg tcttccacaa tattaccgac acgcacgtgg 900

cgcatcatct gttctccacg atgccgcatt atcacgcgat ggaagctacc aaaggcgata 960

aagccgatac tggggagagt atttatcagt tcgatgggac gcggtggtta ggcgatgtga 1020

gggaggcgag gatgtatcta ttgtggaacg gaacagcagg tgagaagaac gtgtgttctg 1080

tacaacatag tatgagaaat gatgatgtga agaaccaaga gatttgtcac gactttctct 1140

gcttctgggg gccctttgtg ga 1162


<210> 69
<211> 70
<212> DNA
<213> Brassica napus

<400> 69
gcagggagtg gcctcgatgg tctgcttcta cggagtcccg cttctgattg tcaatggttt 60

cctcgtgttg 70


<210> 70
<211> 70
<212> DNA
<213> Brassica napus

<400> 70
gcagggagtg gcctcgatgg tctgcttcta cggagtcccg cttctgattg tcaatggttt 60

cctcgtgttg 70


<210> 71
<211> 70
<212> DNA
<213> Brassica napus

<400> 71
ccaaggagtt gcctcgatgg tctgcttcta cggagttcct cttctgattg tcaacgggtt 60

cttagttttg 70


<210> 72
<211> 70
<212> DNA
<213> Brassica napus

<400>  72
ccaaggagtt gcctcgatgg tctgcttcta cggagttcct cttctgattg tcaacgggtt      60

cttagttttg                                                              70


<210>  73
<211>  70
<212>  DNA
<213>  Brassica napus

<400>  73
ccaaggagtt gcctcgatgg tctgcttcta cggagttcct cttctgattg tcaacgggtt      60

cttagttttg                                                              70


<210>  74
<211>  70
<212>  DNA
<213>  Brassica napus

<400>  74
ccaaggagtt gcctcgatgg tctgcttcta cggagttcct cttctgattg tcaacgggtt      60

cttagttttg                                                              70


<210>  75
<211>  70
<212>  DNA
<213>  Brassica napus

<400>  75
gcgaggagtg gcctcgatgg tctgtgtcta cggagttccg cttatgattg tcaactgttt      60

cctcgtcttg                                                              70


<210>  76
<211>  70
<212>  DNA
<213>  Brassica napus

<400>  76
gcgaggagtg gcctcgatgg tctgtgtcta cggagttccg cttatgattg tcaactgttt      60

cctcgtcttg                                                              70


<210>  77
<211>  70
<212>  DNA
<213>  Brassica napus

<400>  77
gcgaggagta gcctctgtgg tctgtgtcta cggagttccg cttctaattg tcaactgttt      60

cctcgtcttg                                                              70


<210>  78

<211>  70
<212>  DNA
<213>  Brassica napus

<400>  78
gcgaggagta gcctctgtgg tctgtgtcta cggagttccg cttctaattg tcaactgttt      60

cctcgtcttg                                                              70


<210>  79
<211>  70
<212>  DNA
<213>  Brassica napus

<400>  79
gcgaggagta gcctctgtgg tctgtgtcta cggagttccg cttctaattg tcaactgttt      60

cctcgtcttg                                                              70


<210>  80
<211>  70
<212>  DNA
<213>  Brassica napus

<400>  80
gcgaggagta gcctctgtgg tctgtgtcta cggagttccg cttctaattg tcaactgttt      60

cctcgtcttg                                                              70


<210>  81
<211>  70
<212>  DNA
<213>  Brassica napus

<400>  81
gcgaggagta gcctctgtgg tctgtgtcta cggagttccg cttctaattg tcaactgttt      60

cctcgtcttg                                                              70


<210>  82
<211>  70
<212>  DNA
<213>  Brassica napus

<400>  82
gcgaggagta gcctctgtgg tctgtgtcta cggagttccg cttctaattg tcaactgttt      60

cctcgtcttg                                                              70


<210>  83
<211>  67
<212>  DNA
<213>  Brassica napus

<400>  83
catcatagcc tcctgcttct actacgtcgc caccattact ttcctccccc ccctccacct      60

ccccccc                                                                 67